# EUROPEAN PATENT APPLICATION

(11) **EP 1 323 709 A1**
(43) Date of publication of application: **02.07.2003**
(21) Application number: 01965644.6
(22) Date of filing: 14.09.2001
(51) Int. Cl.: C07C 275/26, C07D 239/47, C07D 401/12, C07D 401/14, C07D 403/12, C07D 471/10, C07D 491/107, C07D 495/10, C07D 233/54, A61K 31/506, A61K 31/175, A61K 31/513, A61K 31/55, A61P 43/00, A61P 29/00, A61P 37/08

(54) **UREA DERIVATIVE AND ADHESIVE-MOLECULE INHIBITOR CONTAINING THE SAME AS ACTIVE INGREDIENT**

(30) Priority: 14.09.2000 JP 2000281040
(71) Applicant: TORAY INDUSTRIES, INC., Tokyo 103-8666 (JP)
(72) Inventor: TAKAHASHI, Toshiya, Fujisawa-shi, Kanagawa 251-0042 (JP); ISHIGAKI, Takeshi, Kamakura-shi, Kanagawa 248-0031 (JP); FUNAHASHI, Miyuki, Fujisawa-shi, Kanagawa 251-0037 (JP); TANIGUCHI, Koji, Kamakura-shi, Kanagawa 248-0036 (JP); KANEKO, Masayuki, Kamakura-shi, Kanagawa 248-0034 (JP); KAINOH, Mie, Fujisawa-shi, Kanagawa 251-0052 (JP); MEGURO, Hiroyuki, Kamakura-shi, Kanagawa 248-0036 (JP)
(74) Representative: Kador & Partner
(86) International application number: JP0107990
(87) International publication number: WO02022563

(57) **Abstract**

Disclosed are novel urea derivatives and their medical uses, especially as adhesion molecule inhibitors useful for therapies of inflammatory diseases. The urea derivative according to the present invention has the chemical structure, for example, represented by the following Formula (35):

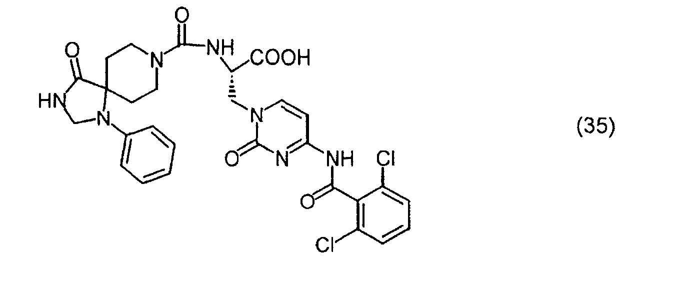

## Description

### Technical Field

The present invention relates to an adhesion molecule inhibitor, especially VLA-4 inhibitor, containing a novel urea derivative or a pharmaceutically acceptable salt thereof, and to a medical use thereof, especially as a therapeutic agent against inflammatory diseases.

### Background Art

Adhesion molecules participate in adhesion between cells and cells, and between cells and cell matrices. Adhesion molecules include a number of families such as integrin family and immunoglobulin super family. The adhesion molecules belonging to integrin family are those expressed on leukocytes such as lymphocytes, monocytes, basophils and eosinophils. These adhesion molecules have heterodimer structure, in which an α chain and a β chain are non-covalently bound, and are classified into some subfamilies depending on the species of the β chain. VLA-4 (very late antigen-4) also called α4β1 or CD49d/CD29, a member of the integrin family, participates in the interactions between leukocytes and vascular endothelial cells or extracellular matrix, and participates in infiltration of leukocytes into inflammatory site. VCAM-1 (vascular cell adhesion molecule-1) and fibronectin are known as the adhesion molecules which interact with VLA-4.

The binding site on fibronectin, which binds to VLA-4 is a fibronectin fragment called CS-1. It has been reported that the minimum unit required for the binding in this fragment consists of 3 amino acid residues, that is, Leucine-Aspartic acid-Valine.

Linear or cyclic peptidic VLA-4 adhesion inhibitor compounds based on the 3 amino acid residues, Leucine-Aspartic acid-Valine have been reported (WO/15973).

On the other hand, it is known that the expression level of VCAM-1 which is another adhesion molecule that also interacts with VLA-4, is increased by stimulation by a cytokine such as IL-1, TNF-α or IL-4, and that VCAM-1 interacts with VLA-4 existing on cells such as lymphocytes, NK cells, monocytes and eosinophils. VLA-4 and VCAM-1 participate in the infiltration of leukocytes into inflammatory sites through blood vessels. From this viewpoint, the interaction between VLA-4 and VCAM-1 is very important in inflammatory reaction.

Among the adhesion molecules, VCAM-1 belongs to the immunoglobulin super family, and 7-Ig-like-domain VCAM-1 and 6-Ig-like-domain VCAM-1 are known. Mutation experiments of VCAM-1 revealed that the binding sites on VCAM-1 for binding to VLA-4 are located in domain 1 and domain 4, and that the amino acid sequence of glutamine-isoleucine-aspartic acid-serine-proline on the CD loop is important for the binding to VLA-4 (e.g., J.Cell Biol., 124 , 601(1994)). J.H.WANG et al. reported a cyclic peptide Cys*GlnIleAspSerProCys* (Cys*Cys* represents disulfide bond) which has an inhibitory activity against adhesion of VLA-4, which cyclic peptide is based on the glutamine-isoleucine-aspartic acid-serine-proline (Proc. Natl. Acad. Sci. USA , 92 , 5714 (1995)). Low molecular compounds having VLA-4-inhibitory activity have also been reported (e.g., US 5770573, US 5821231 and WO99/6436).

The fact that VLA-4 plays an important role in inflammatory reaction has been proved by experiments using anti-VLA-4 antibody in animal models such as contact hypersensitivity, delayed type hypersensitivity models (mouse and rat), experimental autoimmune encephalomyelitis models (mouse and rat), nephrotic nephritis (rat), passive cutaneous anaphylaxis model (guinea pig), immunocomplex-induced pulmonary injury model (rat), spontaneous colitis model (monkey), asthma model (sheep) and adjuvant arthritis model.

It has been proved that the cause of development of chronic inflammatory diseases such as allergic inflammation and chronic rheumatoid arthritis is the repetition of accumulation of leukocytes at the inflammatory site. However, as the drugs for the therapies of these diseases, drugs having activities to inhibit actions of chemical mediators, drugs having activities to inhibit production of chemical mediators, and drugs having activities to inhibit production of active oxygen are conventionally used. Drugs which inhibit activation of leukocytes, such as steroid drugs, are also used. Since these drugs do not have an activity to inhibit accumulation of leukocytes to the inflammatory site as their main actions, they cannot inhibit development of inflammation. In contrast, since adhesion molecules VLA-4 and VCAM-1 mainly participate in the process of accumulation of the leukocytes to the inflammatory site, a novel compound having an activity to inhibit the adhesion of VLA-4 and VCAM-1 is thought to inhibit the accumulation of the leukocytes to the inflammatory site. Thus, the probability that such a compound is an effective therapeutic drug against the above-mentioned diseases is high.

### Disclosure of the Invention

An object of the present invention is to discover a compound which inhibits cell infiltration via adhesion molecules, especially, adhesion molecule VLA-4, thereby making it possible to prevent and cure inflammatory diseases caused by infiltration of leukocytes such as monocytes, lymphocytes and eoshinophils.

The present inventors intensively studied to discover that specific novel urea derivatives and pharmaceutically acceptable salts thereof have activities to inhibit cell adhesion via adhesion molecules, especially adhesion molecule VLA-4, thereby completing the present invention.

That is, the present invention provides a urea derivative of the Formula I: 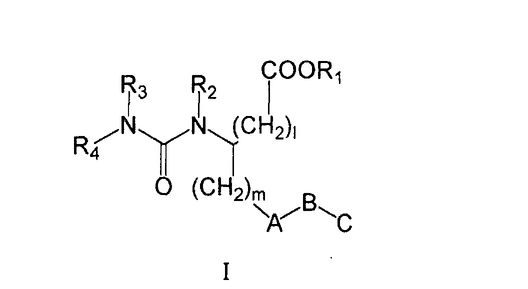 [wherein l represents an integer of 0 to 2; m represents an integer of 1 to 3; R₁ and R₂ independently represent hydrogen or C₁-C₆ linear alkyl; R₃ and R₄ independently represent hydrogen, C₁-C₆ linear alkyl, C₃-C₈ branched alkyl, or phenyl or benzyl, this phenyl or benzyl being substituted with 0 to 2 substituents selected from the group consisting of halogen, methyl, methoxy, cyano, nitro, hydroxyl, amino and tetrazole (excluding cases wherein C is represented by the Formula XIII: 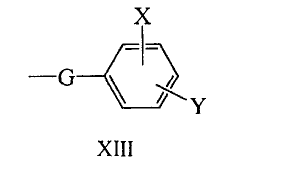 (wherein X and Y independently represent hydrogen, halogen, methyl, methoxy, cyano, nitro, hydroxyl, amino or tetrazole; G may or may not exist, and when G exists, G is a nitrogen atom))
or represent Formula II: 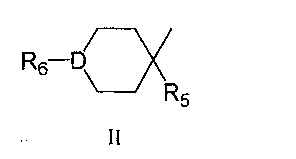 (wherein D represents a carbon atom or nitrogen atom; R₅ represents hydrogen, C₁-C₆ linear alkyl, C₃-C₈ branched alkyl, C₁-C₆ linear N-alkylcarboxamide, C₃-C₈ branched N-alkylcarboxamide, or phenyl or N-phenylcarboxamide, this phenyl or N-phenylcarboxamide being substituted with 0 to 2 substituents selected from the group consisting of halogen, methyl, methoxy, cyano, nitro, hydroxyl, amino and tetrazole (with the proviso that when C is represented by said Formula XIII (wherein symbols therein represent the same meanings as described above), R₅ is C₁-C₆ linear N-alkylcarboxamide, C₃-C₈ branched N-alkylcarboxamide or N-phenylcarboxamide substituted with 0 to 2 substituents selected from the group consisting of halogen, methyl, methoxy, cyano, nitro, hydroxyl, amino and tetrazole); R₆ represents hydrogen, C₁-C₆ linear alkyl, C₃-C₈ branched alkyl, C₁-C₆ linear alkylacyl, C₃-C₈ branched alkylacyl, or phenylsulfone, benzoyl or benzyl, this phenylsulfone, benzoyl or benzyl being substituted with 0 to 2 substituents selected from the group consisting of halogen, methyl, methoxy, cyano, nitro, hydroxyl, amino and tetrazole) (with the proviso that when C is represented by said Formula XIII (wherein symbols therein represent the same meanings as described above), R₆ is phenylsulfone, benzoyl or benzyl, this phenylsulfone, benzoyl or benzyl being substituted with 0 to 2 substituents selected from the group consisting of methyl, cyano, nitro, amino and tetrazole);
R₂ and R₃ may cooperatively represent Formula III: 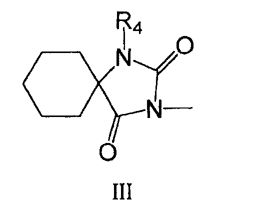 (wherein R₄ represents the same meanings as described above);
R₃ and R₄ may cooperatively represent

### (i) Formula IV:

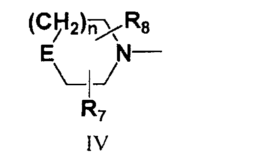

(wherein n represents an integer of 0 to 4; F represents a carbon atom or nitrogen atom; R₇ and R₈ independently represent hydrogen, C₁-C₆ linear alkyl, C₃-C₈ branched alkyl, C₁-C₆ linear alkylacyl, C₃-C₈ branched alkylacyl, pyrrolidine carbonyl, piperidine carbonyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of methyl, methoxy, cyano, nitro, hydroxyl, amino and tetrazole (with the proviso that when C is represented by said Formula XIII (wherein symbols therein represent the same meanings as described above), R₇ and R₈ independently represent pyrrolidine carbonyl, piperidine carbonyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of methyl, cyano, nitro, amino and tetrazole)
or Formula V: 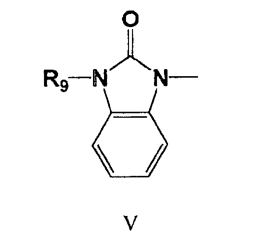 (wherein R₉ represents hydrogen, C₁-C₆ linear alkyl, C₃-C₈ branched alkyl, or phenyl or benzyl, this phenyl or benzyl being substituted with 0 to 2 substituents selected from the group consisting of halogen, methyl, methoxy, cyano, nitro, hydroxyl, amino and tetrazole),

### (ii) Formula VI:

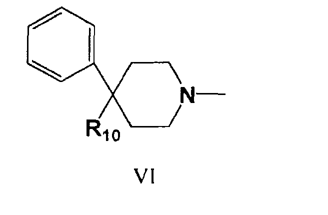

(wherein R₁₀ represents cyano, C₁-C₆ linear alkyl, C₃-C₈ branched alkyl, C₁-C₆ linear alkylamide, C₃-C₈ branched alkylamide, C₅-C₇ cycloalkylamide, C₁-C₆ linear alkylsulfonylamine, C₃-C₈ branched alkylsulfonylamine, or benzamide, phenylsulfonylamine or benzylamino, this benzamide, phenylsulfonylamine or benzylamino being substituted with 0 to 2 substituents selected from the group consisting of halogen, methyl, methoxy, cyano, nitro, hydroxyl, amino and tetrazole) (with the proviso that when C is represented by said Formula XIII (wherein symbols therein represent the same meanings as described above), R₁₀ is C₁-C₆ linear alkylsulfonylamine, C₃-C₈ branched alkylsulfonylamine, or phenylsulfonylamine substituted with 0 to 2 substituents selected from the group consisting of halogen, methyl, methoxy, cyano, nitro, hydroxyl, amino and tetrazole),

### (iii) Formula VII:

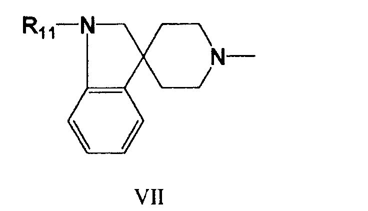

(wherein R₁ represents hydrogen, C₁-C₆ linear alkyl, C₃-C₈ branched alkyl, C₁-C₆ linear alkylacyl, C₃-C₈ branched alkylacyl, C₁-C₆ linear alkylsulfonyl, C₃-C₈ branched alkylsulfonyl, or phenylsulfonyl, benzyl or benzoyl, this phenylsulfonyl, benzyl or benzoyl being substituted with 0 to 2 substituents selected from the group consisting of halogen, methyl, methoxy, cyano, nitro, hydroxyl, amino and tetrazole) (excluding cases where C is represented by said Formula XIII (wherein symbols therein represent the same meanings as described above)),

### (iv) Formula VIII:

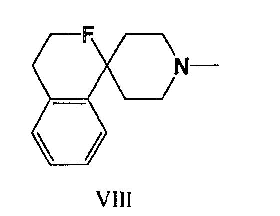

(wherein F represents a carbon atom, oxygen atom, sulfur atom or nitrogen atom; when F is a nitrogen atom, the substituent on said nitrogen atom is hydrogen, C₁-C₆ linear alkyl, C₃-C₈ branched alkyl, C₁-C₆ linear alkylacyl, C₃-C₈ branched alkylacyl, C₁-C₆ linear alkylsulfonyl, C₃-C₈ branched alkylsulfonyl, or phenylsulfonyl, benzyl or benzoyl, this phenylsulfonyl, benzyl or benzoyl being substituted with 0 to 2 substituents selected from the group consisting of halogen, methyl, methoxy, cyano, nitro, hydroxyl, amino and tetrazole) (excluding cases where C is represented by said Formula XIII (wherein symbols therein represent the same meanings as described above)), or

### (v) Formula IX:

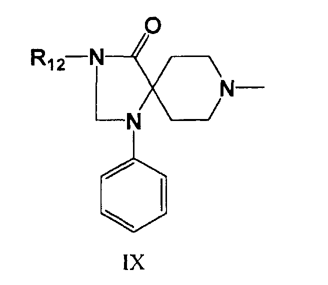

(wherein R₁₂ represents hydrogen, C₁-C₆ linear alkyl, C₃-C₈ branched alkyl, C₆-C₁₀ alkylcycloalkyl, or phenyl or benzyl, this phenyl or benzyl being substituted with 0 to 2 substituents selected from the group consisting of halogen, methyl, methoxy, cyano, nitro, hydroxyl, amino and tetrazole;
A is represented by Formula XI or XII: 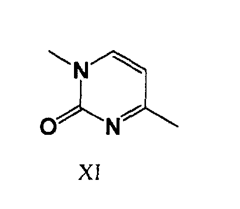 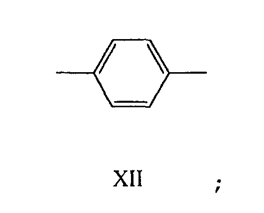 B may or may not exist, when B exists, B represents amide or C₁-C₃ methylene chain;
C is represented by said Formula IV, VI, VII, VIII, IX or XIII (wherein symbols therein represent the same meanings as described above)],
or a pharmaceutically acceptable salt thereof.

The present invention also provides an adhesion molecule inhibitor comprising the urea derivative or a pharmaceutically acceptable salt thereof according to the present invention. The present invention further provides a medical use of the urea derivative or a pharmaceutically acceptable salt thereof according to the present invention, and especially, a therapeutic agent for inflammatory diseases. The present invention still further provides a method for inhibiting an adhesion molecule, comprising administering an effective amount of the urea derivative or a pharmaceutically acceptable salt thereof according to the present invention. The present invention still further provides a use of the urea derivative or a pharmaceutically acceptable salt thereof according to the present invention for the production of a pharmaceutical. The present invention still further provides a use of the urea derivative or a pharmaceutically acceptable salt thereof according to the present invention for the production of an adhesion molecule inhibitor.

By the present invention, novel substances having activities to inhibit cell adhesion via adhesion molecules, especially adhesion molecule VLA-4, were provided. Since the urea derivatives according to the present invention are excellent in inhibiting cell adhesion via adhesion molecules, they are useful as therapeutic drugs against various inflammatory diseases.

### Best Mode for Carrying Out the Invention

As mentioned above, the urea derivatives according to the present invention are represented by Formula I. In Formula I, 1 represents an integer of 0 to 2; m represents an integer of 1 to 3; R₁ and R₂ independently represent hydrogen or C₁-C₆ linear alkyl, that is, methyl, ethyl, n-propyl, n-butyl, n-pentyl or n-hexyl; R₃ and R₄ independently represent hydrogen, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, or phenyl or benzyl, this phenyl or benzyl being substituted with 0 to 2 substituents selected from the group consisting of halogen, methyl, methoxy, cyano, nitro, hydroxyl, amino and tetrazole (excluding cases wherein C is represented by the Formula XIII), such as phenyl, 2-cyanophenyl, 2-hydroxyphenyl, 2-chlorophenyl, 2-nitrophenyl, 2-aminophenyl, 2-bromophenyl, 2-fluorophenyl, 2-tetrazoylphenyl, 2,6-dihydroxyphenyl, 2,6-dimethoxyphenyl, 2,6-dichlorophenyl, 2,6-dinitrophenyl, 2,6-dimethylphenyl, benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl or 2,6-dimethylbenzyl,
Formula II: 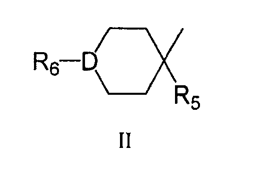 (wherein D represents a carbon atom or nitrogen atom; R₅ represents hydrogen, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₁-C₆ linear N-alkylcarboxamide such as N-methylcarboxamide, N-ethylcarboxamide or N-(n-propyl)carboxamide, C₃-C₈ branched N-alkylcarboxamide such as N-isopropylcarboxamide, N-isobutylcarboxamide, N-isopentylcarboxamide or N-isohexylcarboxamide, or phenyl or N-phenylcarboxamide, this phenyl or N-phenylcarboxamide being substituted with 0 to 2 substituents selected from the group consisting of halogen, methyl, methoxy, cyano, nitro, hydroxyl, amino and tetrazole, such as phenyl, 2-cyanophenyl, 2-hydroxyphenyl, 2-chlorophenyl, 2-nitrophenyl, 2-aminophenyl, 2-bromophenyl, 2-fluorophenyl, 2-tetrazoylphenyl, 2,6-dihydroxyphenyl, 2,6-dimethoxyphenyl, 2,6-dichlorophenyl, 2,6-dinitrophenyl, 2,6-dimethylphenyl, N-phenylcarboxamide, N-(2-cyanophenyl)carboxamide, N-(2-hydroxyphenyl)carboxamide, N-(2-chlorophenyl)carboxamide, N-(2-nitrophenyl)carboxamide, N-(2-aminophenyl)carboxamide, N-(2-brompphenyl)carboxamide, N-(2-fluorophenyl)carboxamide, N-(2-tetrazoylphenyl)carboxamide, N-(2,6-dihydroxyphenyl)carboxamide, N-(2,6-dimethoxyphenyl)carboxamide, N-(2,6-dichlorophenyl)carboxamide, N-(2,6-dinitro)phenylcarboxamide or N-(2,6-dimethylphenyl)carboxamide (with the proviso that when C is represented by said Formula XIII, R₅ is C₁-C₆ linear N-alkylcarboxamide, C₃-C₈ branched N-alkylcarboxamide or N-phenylcarboxamide substituted with 0 to 2 substituents selected from the group consisting of halogen, methyl, methoxy, cyano, nitro, hydroxyl, amino and tetrazole);
R₆ represents hydrogen, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₁-C₆ linear alkylacyl such as acetyl, propionyl, butyryl or valeryl, C₃-C₈ branched alkylacyl such as isopropionyl, isobutyryl, pivaloyl or isopentanoyl, or phenylsulfone, benzoyl or benzyl, this phenylsulfone, benzoyl or benzyl being substituted with 0 to 2 substituents selected from the group consisting of halogen, methyl, methoxy, cyano, nitro, hydroxyl, amino and tetrazole, such as phenylsulfonyl, 2-cyanophenylsulfonyl, 2-hydroxyphenylsulfonyl, 2-chlorophenylsulfonyl, 2-nitrophenylsulfonyl, 2-aminophenylsulfonyl, 2-bromophenylsulfonyl, 2-fluorophenylsulfonyl, 2-tetrazoylphenylsulfonyl, 2,6-dihydroxyphenylsulfonyl, 2,6-dimethoxyphenylsulfonyl, 2,6-dichlorophenylsulfonyl, 2,6-dinitrophenylsulfonyl, 2,6-dimethylphenylsulfonyl, benzoyl, 2-cyanobenzoyl, 2-hydroxybenzoyl, 2-chlorobenzoyl, 2-nitrobenzoyl, 2-aminobenzoyl, 2-bromobenzoyl, 2-fluorobenzoyl, 2-tetrazoylbenzoyl, 2,6-dihydroxybenzoyl, 2,6-dimethoxybenzoyl, 2,6-dichlorobenzoyl, 2,6-dinitrobenzoyl, 2,6-dimethylbenzoyl, benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl or 2,6-dimethylbenzyl (with the proviso that when C is represented by said Formula XIII, R₆ is phenylsulfone, benzyl or benzyl, this phenylsulfone, benzyl or benzyl being substituted with 0 to 2 substituents selected from the group consisting of methyl, cyano, nitro, amino and tetrazole);
R₂ and R₃ may cooperatively represent Formula III: 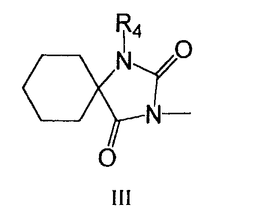 (wherein R₄ represents the same meanings as described above);
R₃ and R₄ may cooperatively represent

### (i) Formula IV:

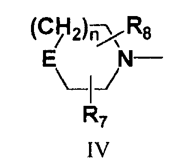

(wherein n represents an integer of 0 to 4; E represents a carbon atom or nitrogen atom; R₇ and R₈ independently represent hydrogen, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₁-C₆ linear alkylacyl such as acetyl, propionyl, butyryl or valeryl, C₃-C₈ branched alkylacyl such as isopropionyl, isobutyryl, pivaloyl or isopentanoyl, pyrrolidine carbonyl, piperidine carbonyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of halogen, methyl, methoxy, cyano, nitro, hydroxyl, amino and tetrazole, such as phenyl, 2-cyanophenyl, 2-hydroxyphenyl, 2-chlorophenyl, 2-nitrophenyl, 2-aminophenyl, 2-bromophcnyl, 2-fluorophenyl, 2-tetrazoylphenyl, 2,6-dihydroxyphenyl, 2,6-dimethoxyphenyl, 2,6-dichlorophenyl, 2,6-dinitrophenyl, 2,6-dimethylphenyl, benzoyl, 2-cyanobenzoyl, 2-hydroxybenzoyl, 2-chlorobenzoyl, 2-nitrobenzoyl, 2-aminobenzoyl, 2-bromobenzoyl, 2-fluorobenzoyl, 2-tetrazoylbenzoyl, 2,6-dihydroxybenzoyl, 2,6-dimethoxybenzoyl, 2,6-dichlorobenzoyl, 2,6-dinitrobenzoyl, 2,6-dimethylbenzoyl, benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl, 2,6-dimethylbenzyl, benzamide, 2-cyanobenzamide, 2-hydroxybenzamide, 2-chlorobenzamide, 2-nitrobenzamide, 2-aminobenzamide, 2-bromobenzamide, 2-fluorobenzamide, 2-tetrazoylbenzamide, 2,6-dihydroxybenzamide, 2,6-dimethoxybenzamide, 2,6-dichlorobenzamide, 2,6-dinitrobenzamide, 2,6-dimethylbenzamide, N-phenylcarboxamide, N-(2-cyanophenyl)carboxamide, N-(2-hydroxyphenyl)carboxamide, N-(2-chlorophenyl)carboxamide, N-(2-nitrophenyl)carboxamide, N-(2-aminophenyl)carboxamide, N-(2-bromophenyl)carboxamide, N-(2-fluorophenyl)carboxamide, N-(2-tetrazoylphenyl)carboxamide, N-(2,6-dihydroxyphenyl)carboxamide, N-(2,6-dimethoxyphenyl)carboxamide, N-(2,6-dichlorophenyl)carboxamide, N-(2,6-dinitro)phenylcarboxamide or N-(2,6-dimethylphenyl)carboxamide (with the proviso that when C is represented by said Formula XIII, R₇ and R₈ independently represent pyrrolidine carbonyl, piperidine carbonyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of methyl, cyano, nitro, amino and tetrazole),
or Formula V: 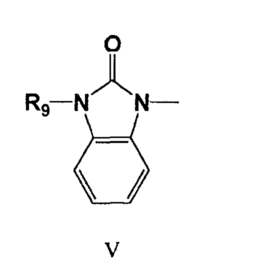 (wherein R₉ represents hydrogen, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl, 4,5-dimethylhexyl, or phenyl or benzyl, this phenyl or benzyl being substituted with 0 to 2 substituents selected from the group consisting of halogen, methyl, methoxy, cyano, nitro, hydroxyl, amino and tetrazole, such as phenyl, 2-cyanophenyl, 2-hydroxyphenyl, 2-chlorophenyl, 2-nitrophenyl, 2-aminophenyl, 2-bromophenyl, 2-fluorophenyl, 2-tetrazoylphenyl, 2,6-dihydroxyphenyl, 2,6-dimethoxyphenyl, 2,6-dichlorophenyl, 2,6-dinitrophenyl, 2,6-dimethylphenyl, benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl or 2,6-dimethylbenzyl,

### (ii) Formula VI:

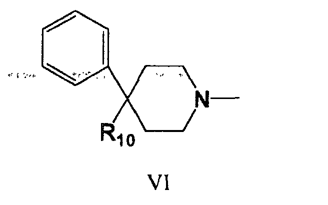

(wherein R₁₀ represents cyano, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₁-C₆ linear alkylacyl, that is, acetyl, propionyl, butyryl, valeryl, pentanoyl or hexanoyl, C₃-C₈ branched alkylacyl such as isopropionyl, isobutyryl, pivaloyl, isopentanoyl, isohexanoyl or isoheptanoyl, C₅-C₇ cycloalkylamide such as cyclopentylamide or cyclohexylamide, C₁-C₆ linear alkylsulfonylamine, that is, methylsulfonylamine, ethylsulfonylamine, n-propylsulfonylamine, n-butylsulfonylamine, n-pentylsulfonylamine or n-hexylsulfonylamine, C₃-C₈ branched alkylsulfonylamine such as isopropylsulfonylamine, isobutylsulfonylamine, t-butylsulfonylamine, isopentylsulfonylamine, isohexylsulfonylamine or isoheptylsulfonylamine, or benzamide, phenylsulfonylamine or benzylamide, this benzamide, phenylsulfonylamine or benzylamide being substituted with 0 to 2 substituents selected from the group consisting of halogen, methyl, methoxy, cyano, nitro, hydroxyl, amino and tetrazole, such as benzamide, 2-cyanobenzamide, 2-hydroxybenzamide, 2-chlorobenzamide, 2-nitrobenzamide, 2-aminobenzamide, 2-bromobenzamide, 2-fluorobenzamide, 2-tetrazoylbenzamide, 2,6-dihydroxybenzamide, 2,6-dimethoxybenzamide, 2,6-dichlorobenzamide, 2,6-dinitrobenzamide, 2,6-dimethylbenzamide, benzylamide, 2-cyanobenzylamide, 2-hydroxybenzylamide, 2-chlorobenzylamide, 2-nitrobenzylamide, 2-aminobenzylamide, 2-bromobenzylamide, 2-fluorobenzylamide, 2-tetrazoylbenzylamide, 2,6-dihydroxybenzylamide, 2,6-dimethoxybenzylamide, 2,6-dichlorobenzylamide, 2,6-dinitrobenzylamide, 2,6-dimethylbenzylamide, phenylsulfonylamine, 2-cyanophenylsulfonylamine, 2-hydroxyphenylsulfonylamine, 2-chlorophenylsulfonylamine, 2-nitrophenylsulfonylamine, 2-aminophenylsulfonylamine, 2-bromophenylsulfonylamine, 2-fluorophenylsulfonylamine, 2-tetrazoylphenylsulfonylaminc, 2,6-dihydroxyphenylsulfonylamine, 2,6-dimethoxyphenylsulfonylamine, 2,6-dichlorophenylsulfonylamine, 2,6-dinitrophenylsulfonylamine or 2,6-dimethylphenylsulfonylamine (with the proviso that when C is represented by said Formula XIII, R₁₀ is C₁-C₆ linear alkylsulfonylamine, C₃-C₈ branched alkylsulfonylamine, or phenylsulfonylamine substituted with 0 to 2 substituents selected from the group consisting of halogen, methyl, methoxy, cyano, nitro, hydroxyl, amino and tetrazole),

### (iii) Formula VII:

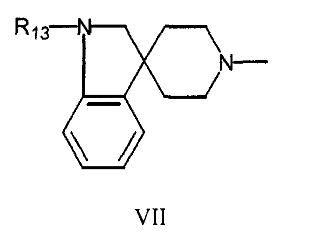

(wherein R₁₁ represents hydrogen, C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₁-C₆ linear alkylacyl, that is, acetyl, propionyl, butyryl, valeryl, pentanoyl or hexanoyl, C₃-C₈ branched alkylacyl such as isopropionyl, isobutyryl, pivaloyl, isopentanoyl, isohexanoyl or isoheptanoyl, C₁-C₆ linear alkylsulfonyl, that is, mesyl, ethanesulfonyl, n-propanesulfonyl, n-butanesulfonyl, n-pentanesulfonyl or n-hexanesulfonyl, C₃-C₈ branched alkylsulfonyl such as isopropanesulfonyl, isobutanesulfonyl, t-butanesulfonyl, isopentanesulfonyl, isohexanesulfonyl or isoheptanesulfonyl, or phenylsulfonyl, benzyl or benzoyl, this phenylsulfonyl, benzyl or benzoyl being substituted with 0 to 2 substituents selected from the group consisting of halogen, methyl, methoxy, cyano, nitro, hydroxyl, amino and tetrazole, such as benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl or 2,6-dimethylbenzyl, phenylsulfonyl, 2-cyanophenylsulfonyl, 2-hydroxyphenylsulfonyl, 2-chlorophenylsulfonyl, 2-nitrophenylsulfonyl, 2-aminophenylsulfonyl, 2-bromophenylsulfonyl, 2-fluorophenylsulfonyl, 2-tetrazoylphenylsulfonyl, 2,6-dihydroxyphenylsulfonyl, 2,6-dimethoxyphenylsulfonyl, 2,6-dichlorophenylsulfonyl, 2,6-dinitrophenylsulfonyl, 2,6-dimethylphenylsulfonyl, benzoyl, 2-cyanobenzoyl, 2-hydroxybenzoyl, 2-chlorobenzoyl, 2-nitrobenzoyl, 2-aminobenzoyl, 2-bromobenzoyl, 2-fluorobenzoyl, 2-tetrazoylbenzoyl, 2,6-dihydroxybenzoyl, 2,6-dimethoxybenzoyl, 2,6-dichlorobenzoyl, 2,6-dinitrobenzoyl or 2,6-dimethylbenzoyl (excluding cases where C is represented by said Formula XIII),

### (iv) Formula VIII:

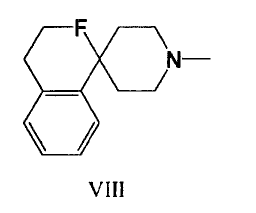

(wherein F represents a carbon atom, oxygen atom, sulfur atom or nitrogen atom; when F is a nitrogen atom, the substituent on said nitrogen atom is C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₁-C₆ linear alkylacyl, that is, acetyl, propionyl, butyryl, valeryl, pentanoyl or hexanoyl, C₃-C₈ branched alkylacyl such as isopropionyl, isobutyryl, pivaloyl, isopentanoyl, isohexanoyl or isoheptanoyl, C₁-C₆ linear alkylsulfonyl, that is, mesyl, ethanesulfonyl, n-propanesulfonyl, n-butanesulfonyl, n-pentanesulfonyl or n-hexanesulfonyl, C₃-C₈ branched alkylsulfonyl such as isopropanesulfonyl, isobutanesulfonyl, t-butanesulfonyl, isopentanesulfonyl, isohexanesulfonyl or isoheptanesulfonyl, or phenylsulfonyl, benzyl or benzoyl, this phenylsulfonyl, benzyl or benzoyl being substituted with 0 to 2 substituents selected from the group consisting of halogen, methyl, methoxy, cyano, nitro, hydroxyl, amino and tetrazole, such as benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl or 2,6-dimethylbenzyl, phenylsulfonyl, 2-cyanophenylsulfonyl, 2-hydroxyphenylsulfonyl, 2-chlorophenylsulfonyl, 2-nitrophenylsulfonyl, 2-aminophenylsulfonyl, 2-bromophenylsulfonyl, 2-fluorophenylsulfonyl, 2-tetrazoylphenylsulfonyl, 2,6-dihydroxyphenylsulfonyl, 2,6-dimethoxyphenylsulfonyl, 2,6-dichlorophenylsulfonyl, 2,6-dinitrophenylsulfonyl, 2,6-dimethylphenylsulfonyl, benzoyl, 2-cyanobenzoyl, 2-hydroxybenzoyl, 2-chlorobenzoyl, 2-nitrobenzoyl, 2-aminobenzoyl, 2-bromobenzoyl, 2-fluorobenzoyl, 2-tetrazoylbenzoyl, 2,6-dihydroxybenzoyl, 2,6-dimethoxybenzoyl, 2,6-dichlorobenzoyl, 2,6-dinitrobenzoyl or 2,6-dimethylbenzoyl (excluding cases where C is represented by said Formula XIII),

### (v) Formula IX:

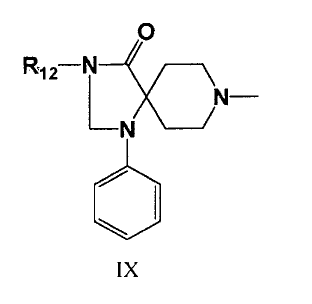

(wherein R₁₂ represents C₁-C₆ linear alkyl, that is, methyl, ethyl, n-propyl, n-butyl, n-pentyl or n-hexyl, C₃-C₈ branched alkyl such as 1-methylethyl, 1-methylpropyl, 2-methylpropyl, 1-methylbutyl, 2-methylbutyl, 3-methylbutyl, 1-methylpentyl, 2-methylpentyl, 3-methylpentyl, 4-methylpentyl, 1-methylhexyl, 2-methylhexyl, 3-methylhexyl, 4-methylhexyl, 5-methylhexyl, 3,5-dimethylhexyl, 3,6-dimethylhexyl or 4,5-dimethylhexyl, C₆-C₁₀ alkylcycloalkyl such as cyclopentylmethyl, cyclohexylmethyl or cycloheptylmethyl, or phenyl or benzyl, this phenyl or benzyl being substituted with 0 to 2 substituents selected from the group consisting of halogen, methyl, methoxy, cyano, nitro, hydroxyl, amino and tetrazole, such as phenyl, 2-cyanophenyl, 2-hydroxyphenyl, 2-chlorophenyl, 2-nitrophenyl, 2-aminophenyl, 2-bromophenyl, 2-fluorophenyl, 2-tetrazoylphenyl, 2,6-dihydroxyphenyl, 2,6-dimethoxyphenyl, 2,6-dichlorophenyl, 2,6-dinitrophenyl, 2,6-dimethylphenyl, benzyl, 2-cyanobenzyl, 2-hydroxybenzyl, 2-chlorobenzyl, 2-nitrobenzyl, 2-aminobenzyl, 2-bromobenzyl, 2-fluorobenzyl, 2-tetrazoylbenzyl, 2,6-dihydroxybenzyl, 2,6-dimethoxybenzyl, 2,6-dichlorobenzyl, 2,6-dinitrobenzyl or 2,6-dimethylbenzyl);
A is represented by Formula XI or XII: 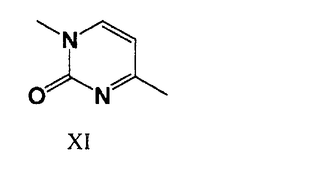 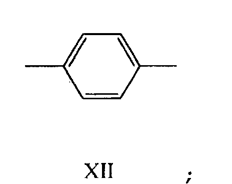 B may or may not exist, when B exists, B represents amide or C₁-C₃ methylene chain;
C is represented by said Formula IV, VI, VII, VIII, IX or XIII 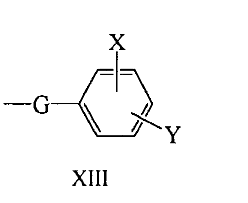 (wherein X and Y represent the same meanings as described above; G may or may not exist, and when G exists, G is a nitrogen atom)]
or a pharmaceutically acceptable salt thereof.

In the definitions of each substituents, the phrase that a certain group does "not exist" or "no group" exists means that the group is "not shown in the structural formula", and includes the both cases where atom(s) actually does(do) not exist and hydrogen atom(s) exist(s). For example, in Formula I, in case where B does not exist, it means that A and C are directly bonded. On the other hand, for example, in Formula XIII, in case where G is a nitrogen atom, it means that one hydrogen atom is bound to G. Those skilled in the art can easily and definitely understand what a specific structural formula mean.

Specific examples of the compounds according to the present invention include 3-(4-((2,6-dichlorophenyl)carbonylamino)-2-oxohydropyrimidinyl)-2-((ethylamino)carbonylamino)propanoic acid, 3-(4-((2,6-dimethylphenyl)carbonylamino)-2-oxohydropyrimidinyl)-2-((ethylamino)carbonylamino)propanoic acid, 3-(4-((2,6-dimethoxyphenyl)carbonylamino)-2-oxohydropyrimidinyl)-2-((ethylamino)carbonylamino)propanoic acid, 3 -(4-((2,6-dimethoxyphenyl)carbonylamino)-2-oxohydropyrimidinyl)-2-((dimethylamino)carbonylamino)propanoic acid, 3-(4-((2,6-dimeththylphenyl)carbonylamino)-2-oxohydropyrimidinyl)-2-((dimethylamino)carbonylamino)propanoic acid, 3-(4-((2,6-dichlorophenyl)carbonylamino)-2-oxohydropyrimidinyl )-2-((dimethylamino)carbonylamino)propanoic acid, 3-(4-((2,6-dichlorophenyl)carbonylamino)-2-oxohydropyrimidinyl)-2-((methyl(methylethyl)amino)carbonylamino)propanoic acid, 3-(4-((2,6-dimethylphenyl)carbonylamino)-2-oxohydropyrimidinyl)-2-((methyl(methylethyl)amino)carbonylamino)propanoic acid, 3-(4-((2,6-dimethoxyphenyl)carbonylamino)-2-oxohydropyrimidinyl)-2-((methyl(methylethyl)amino)carbonylamino)propanoic acid, 3-(4-((2,6-dimethoxyphenyl)carbonylamino)-2-oxohydropyrimidinyl)-2-((methylphenylamino)carbonylamino)propanoic acid, 3-(4-((2,6-dimethylphenyl)carbonylamino)-2-oxohydropyrimidinyl)-2-((methylphenylamino)carbonylamino)propanoic acid, 3-(4-((2,6-dichlorophenyl)carbonylamino)-2-oxohydropyrimidinyl)-2-((methylphenylamino)carbonylamino)propanoic acid, 3-(4-((2,6-dichlorophenyl)carbonylamino)-2-oxohydropyrimidinyl)-2-(((4-phenyl-1-benzyl-4-piperidyl)amino)carbonylamino)propanoic acid, 3-(4-((2,6-dimethylphenyl)carbonylamino)-2-oxohydropyrimidinyl)-2-(((4-phenyl-1-benzyl-4-piperidyl)amino)carbonylamino)propanoic acid, 3-(4-((2,6-dimethoxyphenyl)carbonylamino)-2-oxohydropyrimidinyl)-2-(((4-phenyl-1-benzyl-4-piperidyl)amino)carbonylamino)propanoic acid, 3-(4-((2,6-dimethoxyphenyl)carbonylamino)-2-oxohydropyrimidinyl)-2-(((1-benzyl-4-piperidyl)amino)carbonylamino)propanoic acid, 3-(4-((2,6-dimethylphenyl)carbonylamino)-2-oxohydropyrimidinyl)-2-(((1-benzyl-4-piperidyl)amino)carbonylamino)propanoic acid, 3-(4-((2,6-dichlorophenyl)carbonylamino)-2-oxohydropyrimidinyl)-2-(((1-benzyl-4-piperidyl)amino)carbonylamino)propanoic acid, 3-(4-((2,6-dichlorophenyl)carbonylamino)-2-oxohydropyrimidinyl)-2-(((4-methyl-1-benzyl-4-piperidyl)amino)carbonylamino)propanoic acid, 3-(4-((2,6-dimethylphenyl)carbonylamino)-2-oxohydropyrimidinyl)-2-(((4-methyl-1-benzyl-4-piperidyl)amino)carbonylamino)propanoic acid, 3-(4-((2,6-dimethoxyphenyl)carbonylamino)-2-oxohydropyrimidinyl)-2-(((4-methyl-1-benzyl-4-piperidyl)amino)carbonylamino)propanoic acid, 3-(4-((2,6-dimethoxyphenyl)carbonylamino)-2-oxohydropyrimidinyl)-2-(((4-(2-methylpropyl)-1-benzyl-4-piperidyl)amino)carbonylamino)propanoic acid, 3-(4-((2,6-dimethylphenyl)carbonylamino)-2-oxohydropyrimidinyl)-2-(((4-(2-methylpropyl)-1-benzyl-4-piperidyl)amino)carbonylamino)propanoic acid, 3-(4-((2,6-dichlorophenyl)carbonylamino)-2-oxohydropyrimidinyl)-2-(((4-(2-methylpropyl)-1-benzyl-4-piperidyl)amino)carbonylamino)propanoic acid, 3-(4-((2,6-dichlorophenyl)carbonylamino)-2-oxohydropyrimidinyl)-2-(((4-acetyl-1-benzyl-4-piperidyl)amino)carbonylamino)propanoic acid, 3-(4-((2,6-dimethylphenyl)carbonylamino)-2-oxohydropyrimidinyl)-2-(((4-acetyl-1-benzyl-4-piperidyl)amino)carbonylamino)propanoic acid, 3-(4-((2,6-dimethoxyphenyl)carbonylamino)-2-oxohydropyrimidinyl)-2-(((4-acetyl-1-benzyl-4-piperidyl)amino)carbonylamino)propanoic acid, 3-(4-((2,6-dimethoxyphenyl)carbonylamino)-2-oxohydropyrimidinyl)-2-(((1-(2-methylpropyl)-4-(2-methylpropanoyl)-4-piperidyl)amino)carbonylamino)propanoic acid, 3-(4-((2,6-dimethylphenyl)carbonylamino)-2-oxohydropyrimidinyl)-2-(((1-(2-methylpropyl)-4-(2-methylpropanoyl)-4-piperidyl)amino)carbonylamino)propanoic acid, 3-(4-((2,6-dichlorophenyl)carbonylamino)-2-oxohydropyrimidinyl)-2-(((1-(2-methylpropyl)-4-(2-methylpropanoyl)-4-piperidyl)amino)carbonylamino)propanoic acid, 3-(4-((2,6-dichlorophenyl)carbonylamino)-2-oxohydropyrimidinyl)-2-(((4-acetyl-1-(phenylcarbonyl)-4-piperidyl)amino)carbonylamino)propanoic acid, 3-(4-((2,6-dimethylphenyl)carbonylamino)-2-oxohydropyrimidinyl)-2-(((4-acetyl-1-(phenylcarbonyl)-4-piperidyl)amino)carbonylamino)propanoic acid, 3-(4-((2,6-dimethoxyphenyl)carbonylamino)-2-oxohydropyrimidinyl)-2-(((4-acetyl-1-(phenylcarbonyl)-4-piperidyl)amino)carbonylamino)propanoic acid, 3-(4-((2,6-dichlorophenyl)carbonylamino)-2-oxohydropyrimidinyl)-2-(((4-ethyl-1-(2-methylpropanoyl)-4-piperidyl)amino)carbonylamino)propanoic acid, 3-(4-((2,6-dimethylphenyl)carbonylamino)-2-oxohydropyrimidinyl)-2-(((4-ethyl-1-(2-methylpropanoyl)-4-piperidyl)amino)carbonylamino)propanoic acid, 3-(4-((2,6-dimethoxyphenyl)carbonylamino)-2-oxohydropyrimidinyl)-2-(((4-ethyl-1-(2-methylpropanoyl)-4-piperidyl)amino)carbonylamino)propanoic acid, 3-(4-((2,6-dichlorophenyl)carbonylamino)-2-oxohydropyrimidinyl)-2-(((4-(2-methylpropanoyl)-1-benzyl-4-piperidyl)amino)carbonylamino)propanoic acid, 3-(4-((2,6-dimethylphenyl)carbonylamino)-2-oxohydropyrimidinyl)-2-(((4-(2-methylpropanoyl)-1-benzyl-4-piperidyl)amino)carbonylamino)propanoic acid, 3-(4-((2,6-dimethoxyphenyl)carbonylamino)-2-oxohydropyrimidinyl)-2-(((4-(2-methylpropanoyl)-1-benzyl-4-piperidyl)amino)carbonylamino)propanoic acid, 3-(4-((2,6-dimethylphenyl)carbonylamino)-2-oxohydropyrimidinyl)-2-(((4-(phenylcarbonyl)-1-benzyl-4-piperidyl)amino)carbonylamino)propanoic acid, 3-(4-((2,6-dimethoxyphenyl)carbonylamino)-2-oxohydropyrimidinyl)-2-(((4-(phenylcarbonyl)-1-benzyl-4-piperidyl)amino)carbonylamino)propanoic acid, 3-(4-((2,6-dichlorophenyl)carbonylamino)-2-oxohydropyrimidinyl)-2-(((4-(phenylcarbonyl)-1-benzyl-4-piperidyl)amino)carbonylamino)propanoic acid, 3-(4-((2,6-dichlorophenyl)carbonylamino)-2-oxohydropyrimidinyl)-2-(((1-methyl-4-(phenylcarbonyl)-4-piperidyl)amino)carbonylamino)propanoic acid, 3-(4-((2,6-dimethylphenyl)carbonylamino)-2-oxohydropyrimidinyl)-2-(((1-methyl-4-(phenylcarbonyl)-4-piperidyl)amino)carbonylamino)propanoic acid, 3-(4-((2,6-dimethoxyphenyl)carbonylamino)-2-oxohydropyrimidinyl)-2-(((1-methyl-4-(phenylcarbonyl)-4-piperidyl)amino)carbonylamino)propanoic acid, 3-(4-((2,6-dichlorophenyl)carbonylamino)-2-oxohydropyrimidinyl)-2-(((4-(2-methylpropanoyl)-1-phenyl-4-piperidyl)amino)carbonylamino)propanoic acid, 3-(4-((2,6-dimethylphenyl)carbonylamino)-2-oxohydropyrimidinyl)-2-(((4-(2-methylpropanoyl)-1-phenyl-4-piperidyl)amino)carbonylamino)propanoic acid, 3-(4-((2,6-dimethoxyphenyl)carbonylamino)-2-oxohydropyrimidinyl)-2-(((4-(2-methylpropanoyl)-1-phenyl-4-piperidyl)amino)carbonylamino)propanoic acid, 3-(4-((2,6-dimethylphenyl)carbonylamino)-2-oxohydropyrimidinyl)-2-(((4-acetyl-1-(phenylsulfonyl)-4-piperidyl)amino)carbonylamino)propanoic acid, 3-(4-((2,6-dimethoxyphenyl)carbonylamino)-2-oxohydropyrimidinyl)-2-(((4-acetyl-1-(phenylsulfonyl)-4-piperidyl)amino)carbonylamino)propanoic acid, 3-(4-((2,6-dichlorophenyl)carbonylamino)-2-oxohydropyrimidinyl)-2-(((4-acetyl-1-(phenylsulfonyl)-4-piperidyl)amino)carbonylamino)propanoic acid, 3-(4-((2,6-dichlorophenyl)carbonylamino)-2-oxohydropyrimidinyl)-2-(((4-ethyl-1-(phenylsulfonyl)-4-piperidyl)amino)carbonylamino)propanoic acid, 3-(4-((2,6-dimethylphenyl)carbonylamino)-2-oxohydropyrimidinyl)-2-(((4-ethyl-1-(phenylsulfonyl)-4-piperidyl)amino)carbonylamino)propanoic acid, 3-(4-((2,6-dimethoxyphenyl)carbonylamino)-2-oxohydropyrimidinyl)-2-(((4-ethyl-1-(phenylsulfonyl)-4-piperidyl)amino)carbonylamino)propanoic acid, 3-(4-((2,6-dichlorophenyl)carbonylamino)-2-oxohydropyrimidinyl)-2-(piperidylcarbonylamino)propanoic acid, 3-(4-((2,6-dimethylphenyl)carbonylamino)-2-oxohydropyrimidinyl)-2-(piperidylcarbonylamino)propanoic acid, 3-(4-((2,6-dimethoxyphenyl)carbonylamino)-2-oxohydropyrimidinyl)-2-(piperidylcarbonylamino)propanoic acid, 3-(4-((2,6-dichlorophenyl)carbonylamino)-2-oxohydropyrimidinyl)-2-(pyrrolidinylcarbonylamino)propanoic acid, 3-(4-((2,6-dimethylphenyl)carbonylamino)-2-oxohydropyrimidinyl)-2-(pyrrolidinylcarbonylamino)propanoic acid, 3-(4-((2,6-dimethoxyphenyl)carbonylamino)-2-oxohydropyrimidinyl)-2-(pyrrolidinylcarbonylamino)propanoic acid, 2-(azaperhydroepinylcarbonylamino)-3-(4-((2,6-dimethoxyphenyl)carbonylamino)-2-oxohydropyrimidinyl)propanoic acid, 2-(azaperhydroepinylcarbonylamino)-3-(4-((2,6-dimethylphenyl)carbonylamino)-2-oxohydropyrimidinyl)propanoic acid, 2-(azaperhydroepinylcarbonylamino)-3-(4-((2,6-dichlorophenyl)carbonylamino)-2-oxohydropyrimidinyl)propanoic acid, 3-(4-((2,6-dimethylphenyl)carbonylamino)-2-oxohydropyrimidinyl)-2-((2,6-dimethylpiperidyl)carbonylamino)propanoic acid, 3-(4-((2,6-dimethoxyphenyl)carbonylamino)-2-oxohydropyrimidinyl)-2-((2,6-dimethylpiperidyl)carbonylamino)propanoic acid, 3-(4-((2,6-dichlorophenyl)carbonylamino)-2-oxohydropyrimidinyl)-2-((2,6-dimethylpiperidyl)carbonylamino)propanoic acid, 3-(4-((2,6-dimethylphenyl)carbonylamino)-2-oxohydropyrimidinyl)-2-((2,6-di(methylethyl)piperidyl)carbonylamino)propanoic acid, 3-(4-((2,6-dimethoxyphenyl)carbonylamino)-2-oxohydropyrimidinyl)-2-((2,6-di(methylethyl)piperidyl)carbonylamino)propanoic acid, 3-(4-((2,6-dichlorophenyl)carbonylamino)-2-oxohydropyrimidinyl)-2-((2,6-di(methylethyl)piperidyl)carbonylamino)propanoic acid, 3-(4-((2,6-dichlorophenyl)carbonylamino)-2-oxohydropyrimidinyl)-2-((4-benzylpiperidyl)carbonylamino)propanoic acid, 3-(4-((2,6-dimethylphenyl)carbonylamino)-2-oxohydropyrimidinyl)-2-((4-benzylpiperidyl)carbonylamino)propanoic acid, 3-(4-((2,6-dimethoxyphenyl)carbonylamino)-2-oxohydropyrimidinyl)-2-((4-benzylpiperidyl)carbonylamino)propanoic acid, 3-(4-((2,6-dimethoxyphenyl)carbonylamino)-2-oxohydropyrimidinyl)-2-((4-phenylpiperidyl)carbonylamino)propanoic acid, 3-(4-((2,6-dimethylphenyl)carbonylamino)-2-oxohydropyrimidinyl)-2-((4-phenylpiperidyl)carbonylamino)propanoic acid, 3-(4-((2,6-dichlorophenyl)carbonylamino)-2-oxohydropyrimidinyl)-2-((4-phenylpiperidyl)carbonylamino)propanoic acid, 2-((4-acetylpiperazinyl)carbonylamino)-3-(4-((2,6-dimethoxyphenyl)carbonylamino)-2-oxohydropyrimidinyl)propanoic acid, 2-((4-acetylpiperazinyl)carbonylamino)-3-(4-((2,6-dimethylphenyl)carbonylamino)-2-oxohydropyrimidinyl)propanoic acid, 2-((4-acetylpiperazinyl)carbonylamino)-3-(4-((2,6-dichlorophenyl)carbonylamino)-2-oxohydropyrimidinyl)propanoic acid, 3-(4-((2,6-dichlorophenyl)carbonylamino)-2-oxohydropyrimidinyl)-2-((4-(phenylcarbonyl)piperidyl)carbonylamino)propanoic acid, 3-(4-((2,6-dimethylphenyl)carbonylamino)-2-oxohydropyrimidinyl)-2-((4-(phenylcarbonyl)piperidyl)carbonylamino)propanoic acid, 3-(4-((2,6-dimethoxyphenyl)carbonylamino)-2-oxohydropyrimidinyl)-2-((4-(phenylcarbonyl)piperidyl)carbonylamino)propanoic acid, 3-(4-((2,6-dichlorophenyl)carbonylamino)-2-oxohydropyrimidinyl)-2-((4-cyclohexylcarbonyl)piperidyl)carbonylamino)propanoic acid, 3-(4-((2,6-dimethylphenyl)carbonylamino)-2-oxohydropyrimidinyl)-2-((4-cyclohexylcarbonyl)piperidyl)carbonylamino)propanoic acid, 3-(4-((2,6-dimethoxyphenyl)carbonylamino)-2-oxohydropyrimidinyl)-2-((4-cyclohexylcarbonyl)piperidyl)carbonylamino)propanoic acid, 3-(4-((2,6-dimethoxyphenyl)carbonylamino)-2-oxohydropyrimidinyl)-2-((4-(2,6-dichlorophenyl)carbonyl)piperidyl)carbonylamino)propanoic acid, 3-(4-((2,6-dimethylphenyl)carbonylamino)-2-oxohydropyrimidinyl)-2-((4-(2,6-dichlorophenyl)carbonyl)piperidyl)carbonylamino)propanoic acid, 3-(4-((2,6-dichlorophenyl)carbonylamino)-2-oxohydropyrimidinyl)-2-((4-(2,6-dichlorophenyl)carbonyl)piperidyl)carbonylamino)propanoic acid, 3-(4-((2,6-dichlorophenyl)carbonylamino)-2-oxohydropyrimidinyl)-2-((4-ethylpiperazinyl)carbonylamino)propanoic acid, 3-(4-((2,6-dimethylphenyl)carbonylamino)-2-oxohydropyrimidinyl)-2-((4-ethylpiperazinyl)carbonylamino)propanoic acid, 3-(4-((2,6-dimethoxyphenyl)carbonylamino)-2-oxohydropyrimidinyl)-2-((4-ethylpiperazinyl)carbonylamino)propanoic acid, 3-(4-((2,6-dimethoxyphenyl)carbonylamino)-2-oxohydropyrimidinyl)-2-((2-(phenylcarbonyl)pyrrolidinyl)carbonylamino)propanoic acid, 3-(4-((2,6-dimethylphenyl)carbonylamino)-2-oxohydropyrimidinyl)-2-((2-(phenylcarbonyl)pyrrolidinyl)carbonylamino)propanoic acid, 3-(4-((2,6-dichlorophenyl)carbonylamino)-2-oxohydropyrimidinyl)-2-((2-(phenylcarbonyl)pyrrolidinyl)carbonylamino)propanoic acid, 3-(4-((2,6-dimethoxyphenyl)carbonylamino)-2-oxohydropyrimidinyl)-2-((2-acetylpyrrolidinyl)carbonylamino)propanoic acid, 3-(4-((2,6-dimethylphenyl)carbonylamino)-2-oxohydropyrimidinyl)-2-((2-acetylpyrrolidinyl)carbonylamino)propanoic acid, 3-(4-((2,6-dichlorophenyl)carbonylamino)-2-oxohydropyrimidinyl)-2-((2-acetylpyrrolidinyl)carbonylamino)propanoic acid, 3-(4-((2,6-dichlorophenyl)carbonylamino)-2-oxohydropyrimidinyl)-2-((2-(phenylcarbonyl)piperidyl)carbonylamino)propanoic acid, 3-(4-((2,6-dimcthoxyphenyl)carbonylamino)-2-oxohydropyrimidinyl)-2-((2-(phenylcarbonyl)piperidyl)carbonylamino)propanoic acid, 3-(4-((2,6-dimethylphenyl)carbonylamino)-2-oxohydropyrimidinyl)-2-((2-(phenylcarbonyl)piperidyl)carbonylamino)propanoic acid, 3-(4-((2,6-dimethylphenyl)carbonylamino)-2-oxohydropyrimidinyl)-2-((((acetylamino)cyclohexyl)amino)carbonylamino)propanoic acid, 3-(4-((2,6-dimethoxyphenyl)carbonylamino)-2-oxohydropyrimidinyl)-2-((((acetylamino)cyclohexyl)amino)carbonylamino)propanoic acid, 3-(4-((2,6-dichlorophenyl)carbonylamino)-2-oxohydropyrimidinyl)-2-((((acetylamino)cyclohexyl)amino)carbonylamino)propanoic acid, 3-(4-((2,6-dichlorophenyl)carbonylamino)-2-oxohydropyrimidinyl)-2-((((phenylcarbonylamino)cyclohexyl)amino)carbonylamino)propanoic acid, 3-(4-((2,6-dimethylphenyl)carbonylamino)-2-oxohydropyrimidinyl)-2-((((phenylcarbonylamino)cyclohexyl)amino)carbonylamino)propanoic acid, 3-(4-((2,6-dimethoxyphenyl)carbonylamino)-2-oxohydropyrimidinyl)-2-((((phenylcarbonylamino)cyclohexyl)amino)carbonylamino)propanoic acid, 3-(4-((2,6-dichlorophenyl)carbonylamino)-2-oxohydropyrimidinyl)-2-((((2-methylpropanoylamino)cyclohexyl)amino)carbonylamino)propanoic acid, 3-(4-((2,6-dimethylphenyl)carbonylamino)-2-oxohydropyrimidinyl)-2-((((2-methylpropanoylamino)cyclohexyl)amino)carbonylamino)propanoic acid, 3-(4-((2,6-dimethoxyphenyl)carbonylamino)-2-oxohydropyrimidinyl)-2-((((2-methylpropanoylamino)cyclohexyl)amino)carbonylamino)propanoic acid, 3-(4-((2,6-dichlorophenyl)carbonylamino)-2-oxohydropyrimidinyl)-2-((4-(2-oxo-3-azaindolinyl)piperidyl)carbonylamino)propanoic acid, 3-(4-((2,6-dimethylphenyl)carbonylamino)-2-oxohydropyrimidinyl)-2-((4-(2-oxo-3-azaindolinyl)piperidyl)carbonylamino)propanoic acid, 3-(4-((2,6-dimethoxyphenyl)carbonylamino)-2-oxohydropyrimidinyl)-2-((4-(2-oxo-3-azaindolinyl)piperidyl)carbonylamino)propanoic acid, 3-(4-((2,6-dichlorophenyl)carbonylamino)-2-oxohydropyrimidinyl)-2-((4-(3-methyl-2-oxo-3-azaindolinyl)piperidyl)carbonylamino)propanoic acid, 3-(4-((2,6-dimethylphenyl)carbonylamino)-2-oxohydropyrimidinyl)-2-((4-(3-methyl-2-oxo-3-azaindolinyl)piperidyl)carbonylamino)propanoic acid, 3-(4-((2,6-dimethoxyphenyl)carbonylamino)-2-oxohydropyrimidinyl)-2-((4-(3-methyl-2-oxo-3-azaindolinyl)piperidyl)carbonylamino)propanoic acid, 3-(4-((2,6-dichlorophenyl)carbonylamino)-2-oxohydropyrimidinyl)-2-((4-(2-oxo-3-benzyl-3-azaindolinyl)piperidyl)carbonylamino)propanoic acid, 3-(4-((2,6-dimethylphenyl)carbonylamino)-2-oxohydropyrimidinyl)-2-((4-(2-oxo-3-benzyl-3-azaindolinyl)piperidyl)carbonylamino)propanoic acid, 3-(4-((2,6-dimethoxyphenyl)carbonylamino)-2-oxohydropyrimidinyl)-2-((4-(2-oxo-3-benzyl-3-azaindolinyl)piperidyl)carbonylamino)propanoic acid, 3-(4-((2,6-dichlorophenyl)carbonylamino)-2-oxohydropyrimidinyl)-2-((4-(methylamino)-4-phenylpiperidyl)carbonylamino)propanoic acid, 3-(4-((2,6-dimethylphenyl)carbonylamino)-2-oxohydropyrimidinyl)-2-((4-(methylamino)-4-phenylpiperidyl)carbonylamino)propanoic acid, 3-(4-((2,6-dimethoxyphenyl)carbonylamino)-2-oxohydropyrimidinyl)-2-((4-(methylamino)-4-phenylpiperidyl)carbonylamino)propanoic acid, 3-(4-((2,6-dichlorophenyl)carbonylamino)-2-oxohydropyrimidinyl)-2-((4-(acetylamino)-4-phenylpiperidyl)carbonylamino)propanoic acid, 3-(4-((2,6-dimethylphenyl)carbonylamino)-2-oxohydropyrimidinyl)-2-((4-(acetylamino)-4-phenylpiperidyl)carbonylamino)propanoic acid, 3-(4-((2,6-dimethoxyphenyl)carbonylamino)-2-oxohydropyrimidinyl)-2-((4-(acetylamino)-4-phenylpiperidyl)carbonylamino)propanoic acid, 3-(4-((2,6-dichlorophenyl)carbonylamino)-2-oxohydropyrimidinyl)-2-((4-((methylethyl)amino)-4-phenylpiperidyl)carbonylamino)propanoic acid, 3-(4-((2,6-dimethylphenyl)carbonylamino)-2-oxohydropyrimidinyl)-2-((4-((methylethyl)amino)-4-phenylpiperidyl)carbonylamino)propanoic acid. 3-(4-((2,6-dimethoxyphenyl)carbonylamino)-2-oxohydropyrimidinyl)-2-((4-((methylethyl)amino)-4-phenylpiperidyl)carbonylamino)propanoic acid, 3-(4-((2,6-dimethoxyphenyl)carbonylamino)-2-oxohydropyrimidinyl)-2-((4-(3-methylbutanoylamino)-4-phenylpiperidyl)carbonylamino)propanoic acid, 3-(4-((2,6-dimethylphenyl)carbonylamino)-2-oxohydropyrimidinyl)-2-((4-(3-methylbutanoylamino)-4-phenylpiperidyl)carbonylamino)propanoic acid, 3-(4-((2,6-dichlorophenyl)carbonylamino)-2-oxohydropyrimidinyl)-2-((4-(3-methylbutanoylamino)-4-phenylpiperidyl)carbonylamino)propanoic acid, 3-(4-((2,6-dimethoxyphenyl)carbonylamino)-2-oxohydropyrimidinyl)-2-((4-((methylsulfonyl)amino)-4-phenylpiperidyl)carbonylamino)propanoic acid, 3-(4-((2,6-dimethylphenyl)carbonylamino)-2-oxohydropyrimidinyl)-2-((4-((methylsulfonyl)amino)-4-phenylpiperidyl)carbonylamino)propanoic acid, 3-(4-((2,6-dichlorophenyl)carbonylamino)-2-oxohydropyrimidinyl)-2-((4-((methylsulfonyl)amino)-4-phenylpiperidyl)carbonylamino)propanoic acid, 3-(4-((2,6-dimethoxyphenyl)carbonylamino)-2-oxohydropyrimidinyl)-2-((4-(((methylethyl)sulfonyl)amino)-4-phenylpiperidyl)carbonylamino)propanoic acid, 3-(4-((2,6-dimethylphenyl)carbonylamino)-2-oxohydropyrimidinyl)-2-((4-(((methylethyl)sulfonyl)amino)-4-phenylpiperidyl)carbonylamino)propanoic acid, 3-(4-((2,6-dichlorophenyl)carbonylamino)-2-oxohydropyrimidinyl)-2-((4-(((methylethyl)sulfonyl)amino)-4-phenylpiperidyl)carbonylamino)propanoic acid, 3-(4-((2,6-dichlorophenyl)carbonylamino)-2-oxohydropyrimidinyl)-2-((4-((phenylsulfonyl)amino)-4-phenylpiperidyl)carbonylamino)propanoic acid, 3-(4-((2,6-dimethylphenyl)carbonylamino)-2-oxohydropyrimidinyl)-2-((4-((phenylsulfonyl)amino)-4-phenylpiperidyl)carbonylamino)propanoic acid, 3-(4-((2,6-dimethoxyphenyl)carbonylamino)-2-oxohydropyrimidinyl)-2-((4-((phenylsulfonyl)amino)-4-phenylpiperidyl)carbonylamino)propanoic acid, 2-((2,4,8-triaza-2-methyl-1-oxo-4-phenylspiro[4.5]dec-8-yl)carbonylamino)-3-(4-((2,6-dimethylphenyl)carbonylamino)-2-oxohydropyrimidinyl)propanoic acid, 2-((2,4,8-triaza-2-methyl-1-oxo-4-phenylspiro[4.5]dec-8-yl)carbonylamino)-3-(4-((2,6-dimethoxyphenyl)carbonylamino)-2-oxohydropyrimidinyl)propanoic acid, 2-((2,4,8-triaza-2-methyl-1-oxo-4-phenylspiro[4.5]dec-8-yl)carbonylamino)-3-(4-((2,6-dichlorophenyl)carbonylamino)-2-oxohydropyrimidinyl)propanoic acid, 2-((2,4,8-triaza-1-oxo-4-phenylspiro[4.5]dec-8-yl)carbonylamino)-3-(4-((2,6-dichlorophenyl)carbonylamino)-2-oxohydropyrimidinyl)propanoic acid, 2-((2,4,8-triaza-1-oxo-4-phenylspiro [4.5]dec-8-yl)carbonylamino)-3-(4-((2,6-dimethylphenyl)carbonylamino)-2-oxohydropyrimidinyl)propanoic acid, 2-((2,4,8-triaza-1-oxo-4-phenylspiro[4.5]dec-8-yl)carbonylamino)-3-(4-((2,6-dimethoxyphenyl)carbonylamino)-2-oxohydropyrimidinyl)propanoic acid, 2-((2,4,8-triaza-2-(2-methylpropyl)-1-oxo-4-phenylspiro [4.5]dec-8-yl)carbonylamino)-3-(4-((2,6-dichlorophenyl)carbonylamino)-2-oxohydropyrimidinyl)propanoic acid, 2-((2,4,8-triaza-2-(2-methylpropyl)-1-oxo-4-phenylspiro[4.5]dec-8-yl)carbonylamino)-3 -(4-((2,6-dimethylphenyl)carbonylamino)-2-oxohydropyrimidinyl)propanoic acid, 2-((2,4,8-triaza-2-(2-methylpropyl)-1-oxo-4-phenylspiro [4.5]dec-8-yl)carbonylamino)-3-(4-((2,6-dimethoxyphenyl)carbonylamino)-2-oxohydropyrimidinyl)propanoic acid, 2-((2,4,8-triaza-1-oxo-4-phenyl-2-benzylspiro[4.5]dec-8-yl)carbonylamino)-3 -(4-((2,6-dimethylphenyl)carbonylamino)-2-oxohydropyrimidinyl)propanoic acid, 2-((2,4,8-triaza-1-oxo-4-phenyl-2-benzylspiro[4.5]dec-8-yl)carbonylamino)-3-(4-((2,6-dimethoxyphenyl)carbonylamino)-2-oxohydropyrimidinyl)propanoic acid, 2-((2,4,8-triaza-1-oxo-4-phenyl-2-benzylspiro[4.5]dec-8-yl)carbonylamino)-3-(4-((2,6-dichlorophenyl)carbonylamino)-2-oxohydropyrimidinyl)propanoic acid, 2-((2,4,8-triaza-1-oxo-2,4-diphenylspiro[4.5]dec-8-yl)carbonylamino)-3-(4-((2,6-dichlorophenyl)carbonylamino)-2-oxohydropyrimidinyl)propanoic acid, 2-((2,4,8-triaza-1-oxo-2,4-diphenylspiro[4.5]dec-8-yl)carbonylamino)-3-(4-((2,6-' dimethylphenyl)carbonylamino)-2-oxohydropyrimidinyl)propanoic acid, 2-((2,4,8-triaza-1-oxo-2,4-diphenylspiro[4.5]dec-8-yl)carbonylamino)-3-(4-((2,6-dimethoxyphenyl)carbonylamino)-2-oxohydropyrimidinyl)propanoic acid, 2-((2,4,8-triaza-1-oxo-4-phenylspiro[4.5]dec-8-yl)carbonylamino)-3-(4-((2,4,8-triaza-1-oxo-4-phenylspiro[4.5]dec-8-yl))carbonylamino)-2-oxohydropyrimidinyl)propanoic acid, 2-((2,4,8-triaza-2-methyl-1-oxo-4-phenylspiro[4.5]dec-8-yl)carbonylamino)-3-(4-((2,4,8-triaza-2-methyl-1-oxo-4-phenylspiro[4.5]dec-8-yl))carbonylamino)-2-oxohydropyrimidinyl)propanoic acid, 3-(4-((2,4,8-triaza-1-oxo-4-phenylspiro[4.5]dec-8-yl))carbonylamino)-2-oxohydropyrimidinyl)-2-((2,6-dimethoxyphenyl)carbonylamino)propanoic acid, 3-(4-((2,4,8-triaza-1-oxo-4-phenylspiro[4.5]dec-8-yl))carbonylamino)-2-oxohydropyrimidinyl)-2-((2,6-dimethylphenyl)carbonylamino)propanoic acid, 3-(4-((2,4,8-triaza-1-oxo-4-phenylspiro[4.5]dec-8-yl))carbonyl amino)-2-oxohydropyrimidinyl)-2-((2,6-dichlorophenyl)carbonylamino)propanoic acid, 2-((2,6-dichlorophenyl)carbonylamino)-3-(2-oxo-4-((4-(2-oxo-3-azaindolinyl)piperidyl)carbonylamino)-2-oxohydropyrimidinyl)propanoic acid, 2-((2,6-dimethylphenyl)carbonylamino)-3-(2-oxo-4-((4-(2-oxo-3-azaindolinyl)piperidyl)carbonylamino)-2-oxohydropyrimidinyl)propanoic acid, 2-((2,6-dimethoxyphenyl)carbonylamino)-3-(2-oxo-4-((4-(2-oxo-3-azaindolinyl)piperidyl)carbonylamino)-2-oxohydropyrimidinyl)propanoic acid, 2-((2,6-dichlorophenyl)carbonylamino)-3-(2-oxo-4-((4-(3-methyl-2-oxo-3-azaindolinyl)piperidyl)carbonylamino)-2-oxohydropyrimidinyl)propanoic acid, 2-((2,6-dimethylphenyl)carbonylamino)-3-(2-oxo-4-((4-(3-methyl-2-oxo-3-azaindolinyl)piperidyl)carbonylamino)-2-oxohydropyrimidinyl)propanoic acid, 2-((2,6-dimethoxyphenyl)carbonylamino)-3-(2-oxo-4-((4-(3-methyl-2-oxo-3-azaindolinyl)piperidyl)carbonylamino)-2-oxohydropyrimidinyl)propanoic acid, 2-((2,6-dichlorophenyl)carbonylamino)-3-(4-((4-((acetylamino)-4-phenylpiperidyl)carbonylamino)-2-oxohydropyrimidinyl)propanoic acid, 2-((2,6-dimethylphenyl)carbonylamino)-3-(4-((4-((acetylamino)-4-phenylpiperidyl)carbonylamino)-2-oxohydropyrimidinyl)propanoic acid, 2-((2,6-dimethoxyphenyl)carbonylamino)-3-(4-((4-((acetylamino)-4-phenylpiperidyl)carbonylamino)-2-oxohydropyrimidinyl)propanoic acid, 2-((2,6-dimethoxyphenyl)carbonylamino)-3-(4-((4-((acetylamino)-4-phenylpiperidyl)carbonylamino)phenyl)propanoic acid, 2-((2,6-dimethylphenyl)carbonylamino)-3-(4-((4-((acetylamino)-4-phenylpiperidyl)carbonylamino)phenyl)propanoic acid, 2-((2,6-dichlorophenyl)carbonylamino)-3-(4-((4-((acetylamino)-4-phenylpiperidyl)carbonylamino)phenyl)propanoic acid, 3-(4-((2,4,8-triaza-1-oxo-4-phenylspiro[4.5]dec-8-yl)carbonylamino)phenyl)-2-((2,6-dichlorophenyl)carbonylamino)propanoic acid, 3-(4-((2,4,8-triaza-1-oxo-4-phenylspiro[4.5]dec-8-yl)carbonylamino)phenyl)-2-((2,6-dimethylphenyl)carbonylamino)propanoic acid, 3-(4-((2,4,8-triaza-1-oxo-4-phenylspiro[4.5]dec-8-yl)carbonylamino)phenyl)-2-((2,6-dimethoxyphenyl)carbonylamino)propanoic acid, 2-((2,4,8-triaza-1-oxo-4-phenylspiro[4.5]dec-8-yl)carbonylamino)-3-(4-((2,6-dichlorophenyl)carbonylamino)phenyl)propanoic acid, 2-((2,4,8-triaza-1-oxo-4-phenylspiro[4.5]dec-8-yl)carbonylamino)-3-(4-((2,6-dimethylphenyl)carbonylamino) phenyl)propanoic acid, 2-((2,4,8-triaza-1-oxo-4-phenylspiro[4.5]dec-8-yl)carbonylamino)-3-(4-((2,6-dimethoxyphenyl)carbonylamino) phenyl)propanoic acid, 3-(4-((2,6-dichlorophenyl)carbonylamino)phenyl)-2-((4-(2-oxo-3-azaindolinyl)piperidyl)carbonylamino)propanoic acid, 3-(4-((2,6-dimethylphenyl)carbonylamino)phenyl)-2-((4-(2-oxo-3-azaindolinyl)piperidyl)carbonylamino)propanoic acid, 3-(4-((2,6-dimethoxyphenyl)carbonylamino)phenyl)-2-((4-(2-oxo-3-azaindolinyl)piperidyl)carbonylamino)propanoic acid, 3-(4-((2,6-dichlorophenyl)carbonylamino)phenyl)-2-((4-(acetylamino)-4-phenylpiperidyl)carbonylamino)propanoic acid, 3-(4-((2,6-dimethylphenyl)carbonylamino)phenyl)-2-((4-(acetylamino)-4-phenylpiperidyl)carbonylamino)propanoic acid, 3-(4-((2,6-dimethoxyphenyl)carbonylamino)phenyl)-2-((4-(acetylamino)-4-phenylpiperidyl)carbonylamino)propanoic acid, 2-(2,4-diaza-1,3-dioxospiro[4.5]dec-2-yl)-3-(4-((2,6-dimethoxyphenyl)carbonylamino)phenyl)propanoic acid, 2-(2,4-diaza-1,3-dioxospiro[4.5]dec-2-yl)-3-(4-((2,6-dimethylphenyl)carbonylamino)phenyl)propanoic acid, 2-(2,4-diaza-1,3-dioxospiro[4.5]dec-2-yl)-3-(4-((2,6-dichlorophenyl)carbonylamino)phenyl)propanoic acid, 2-(2,4-diaza-4-methyl-1,3-dioxospiro[4.5]dec-2-yl)-3-(4-((2,6-dimethoxyphenyl)carbonylamino)phenyl)propanoic acid, 2-(2,4-diaza-4-methyl-1,3-dioxospiro[4.5]dec-2-yl)-3-(4-((2,6-dimethylphenyl)carbonylamino)phenyl)propanoic acid, 2-(2,4-diaza-4-methyl-1,3dioxospiro[4.5]dec-2-yl)-3-(4-((2,6-dichlorophenyl)carbonylamino)phenyl)propanoic acid, 2-(2,4-diaza-1,3-dioxo-4-benzylspiro[4.5]dec-2-yl)-3-(4-((2,6-dimethoxyphenyl)carbonylamino)phenyl)propanoic acid, 2-(2,4-diaza-1,3-dioxo-4-benzylspiro[4.5]dec-2-yl)-3-(4-((2,6-dimethylphenyl)carbonylamino)phenyl)propanoic acid, 2-(2,4-diaza-1,3-dioxo-4-benzylspiro[4.5]dec-2-yl)-3-(4-((2,6-dichlorophenyl)carbonylamino)phenyl)propanoic acid, 3 -(4-((2,6-dichlorophenyl)carbonylamino)-2-oxohydropyrimidinyl)-2-(spiro(indoline-3,4'-piperidine)-10-ylcarbonylamino)propanoic acid, 3 -(4-((2,6-dimethylphenyl)carbonylamino)-2-oxohydropyrimidinyl)-2-(spiro(indoline-3,4'-piperidine)-10-ylcarbonylamino)propanoic acid, 3-(4-((2,6-dimethoxyphenyl)carbonylamino)-2-oxohydropyrimidinyl)-2-(spiro(indoline-3,4'-piperidine)-10-ylcarbonylamino)propanoic acid, 3-(4-((2,6-dimethoxyphenyl)carbonylamino)-2-oxohydropyrimidinyl)-2-((1-methylspiro(indoline-3,4'-piperidine)-10-yl)carbonylamino)propanoic acid, 3-(4-((2,6-dichlorophenyl)carbonylamino)-2-oxohydropyrimidinyl)-2-((1-methylspiro(indoline-3,4'-piperidine)-10-yl)carbonylamino)propanoic acid, 3-(4-((2,6-dimethylphenyl)carbonylamino)-2-oxohydropyrimidinyl)-2-((1-methylspiro(indoline-3,4'-piperidine)-10-yl)carbonylamino)propanoic acid, 3-(4-((2,6-dimethoxyphenyl)carbonylamino)-2-oxohydropyrimidinyl)-2-((1-(methylsulfonyl)spiro(indoline-3,4'-piperidine)-10-yl)carbonylamino)propanoic acid, 3-(4-((2,6-dimethylphenyl)carbonylamino)-2-oxohydropyrimidinyl)-2-((1-(methylsulfonyl)spiro(indoline-3,4'-piperidine)-10-yl)carbonylamino)propanoic acid, 3-(4-((2,6-dichlorophenyl)carbonylamino)-2-oxohydropyrimidinyl)-2-((1-(methylsulfonyl)spiro(indoline-3,4'-piperidine)-10-yl)carbonylamino)propanoic acid, 3-(4-((2,6-dichlorophenyl)carbonylamino)-2-oxohydropyrimidinyl)-2-((1-(phenylsulfonyl)spiro(indoline-3,4'-piperidine)-10-yl)carbonylamino)propanoic acid, 3-(4-((2,6-dimethylphenyl)carbonylamino)-2-oxohydropyrimidinyl)-2-((1-(phenylsulfonyl)spiro(indoline-3,4'-piperidine)-10-yl)carbonylamino)propanoic acid, 3-(4-((2,6-dimethoxyphenyl)carbonylamino)-2-oxohydropyrimidinyl)-2-((1-(phenylsulfonyl)spiro(indoline-3,4'-piperidine)-10-yl)carbonylamino)propanoic acid, 3-(4-((2,6-dichlorophenyl)carbonylamino)-2-oxohydropyrimidinyl)-2-((1-(phenylcarbonyl)spiro(indoline-3,4'-piperidine)-10-yl)carbonylamino)propanoic acid, 3-(4-((2,6-dimethylphenyl)carbonylamino)-2-oxohydropyrimidinyl)-2-((1-(phenylcarbonyl)spiro(indoline-3,4'-piperidine)-10-yl)carbonylamino)propanoic acid, 3-(4-((2,6-dimethoxyphenyl)carbonylamino)-2-oxohydropyrimidinyl)-2-((1-(phenylcarbonyl)spiro(indoline-3,4'-piperidine)-10-yl)carbonylamino)propanoic acid, 3-(4-((2,6-dichlorophenyl)carbonylamino)-2-oxohydropyrimidinyl)-2-((1-(2-methylpropyl)spiro(indoline-3,4'-piperidine)-10-yl)carbonylamino)propanoic acid, 3-(4-((2,6-dimethylphenyl)carbonylamino)-2-oxohydropyrimidinyl)-2-((1-(2-methylpropyl)spiro(indoline-3,4'-piperidine)-10-yl)carbonylamino)propanoic acid, 3-(4-((2,6-dimethoxyphenyl)carbonylamino)-2-oxohydropyrimidinyl)-2-((1-(2-methylpropyl)spiro(indoline-3,4'-piperidine)-10-yl)carbonylamino)propanoic acid, 3-(4-((2,6-dichlorophenyl)carbonylamino)-2-oxohydropyrimidinyl)-2-((1-propanoylspiro(indoline-3,4'-piperidine)-10-yl)carbonylamino)propanoic acid, 3-(4-((2,6-dimethylphenyl)carbonylamino)-2-oxohydropyrimidinyl)-2-((1-propanoylspiro(indoline-3,4'-piperidine)-10-yl)carbonylamino)propanoic acid, 3-(4-((2,6-dimethoxyphenyl)carbonylamino)-2-oxohydropyrimidinyl)-2-((1-propanoylspiro(indoline-3,4'-piperidine)-10-yl)carbonylamino)propanoic acid, 3-(4-((2,6-dichlorophenyl)carbonylamino)-2-oxohydropyrimidinyl)-2-((1-(2-methylpropanoyl)spiro(indoline-3,4'-piperidine)-10-yl)carbonylamino)propanoic acid, 3-(4-((2,6-dimethylphenyl)carbonylamino)-2-oxohydropyrimidinyl)-2-((1-(2-methylpropanoyl)spiro(indoline-3,4'-piperidine)-10-yl)carbonylamino)propanoic acid, 3-(4-((2,6-dimethoxyphenyl)carbonylamino)-2-oxohydropyrimidinyl)-2-((1-(2-methylpropanoyl)spiro(indoline-3,4'-piperidine)-10-yl)carbonylamino)propanoic acid, 3-(4-((2,6-dimethoxyphenyl)carbonylamino)-2-oxohydropyrimidinyl)-2-(( 1-phenylspiro(indoline-3,4'-piperidine)-10-yl)carbonylamino)propanoic acid, 3-(4-((2,6-dimethylphenyl)carbonylamino)-2-oxohydropyrimidinyl)-2-((1-phenylspiro(indoline-3,4'-piperidine)-10-yl)carbonylamino)propanoic acid, 3-(4-((2,6-dichlorophenyl)carbonylamino)-2-oxohydropyrimidinyl)-2-((1-phenylspiro(indoline-3,4'-piperidine)-10-yl)carbonylamino)propanoic acid, 3-(4-((2,6-dimethoxyphenyl)carbonylamino)-2-oxohydropyrimidinyl)-2-(spiro(isochromanl,4'-piperidine)-11-ylcarbonylamino)propanoic acid, 3-(4-((2,6-dimethylphenyl)carbonylamino)-2-oxohydropyrimidinyl)-2-(spiro(isochroman1,4'-piperidine)-11-ylcarbonylamino)propanoic acid, 3-(4-((2,6-dichlorophenyl)carbonylamino)-2-oxohydropyrimidinyl)-2-(spiro(isochroman1,4'-piperidine)-11-ylcarbonylamino)propanoic acid, 3-(4-((2,6-dimethoxyphenyl)carbonylamino)-2-oxohydropyrimidinyl)-2-(spiro(3H,4H-benzo[b]thian-1,4'-piperidine)-11-ylcarbonylamino)propanoic acid, 3-(4-((2,6-dimethylphenyl)carbonylamino)-2-oxohydropyrimidinyl)-2-(spiro(3H,4H-benzo[b]thian-1,4'-piperidine)-11-ylcarbonylamino)propanoic acid, 3-(4-((2,6-dichlorophenyl)carbonylamino)-2-oxohydropyrimidinyl)-2-(spiro(3H,4H-benzo[b]thian-1,4'-piperidine)-11-ylcarbonylamino)propanoic acid, 3-(4-((2,6-dimethoxyphenyl)carbonylamino)-2-oxohydropyrimidinyl)-2-(spiro(1,2,3,4-tetrahydroisoquinoline-1,4'-piperidine)-11-ylcarbonylamino)propanoic acid, 3-(4-((2,6-dimethylphenyl)carbonylamino)-2-oxohydropyrimidinyl)-2-(spiro(1,2,3,4-tetrahydroisoquinoline-1,4'-piperidine)-11-ylcarbonylamino)propanoic acid, 3-(4-((2,6-dichlorophenyl)carbonylamino)-2-oxohydropyrimidinyl)-2-(spiro(1,2,3,4-tetrahydroisoquinoline-1,4'-piperidine)-1-ylcarbonylamino)propanoic acid, 3-(4-((2,6-dimethoxyphenyl)carbonylamino)-2-oxohydropyrimidinyl)-2-((2-methylspiro(1,2,3,4-tetrahydroisoquinoline-1,4'-piperidine)-11-yl)carbonylamino)propanoic acid, 3-(4-((2,6-dimethylphenyl)carbonylamino)-2-oxohydropyrimidinyl)-2-((2-methylspiro( 1,2,3,4-tetrahydroisoquinoline-1,4'-piperidine)-11-yl)carbonylamino)propanoic acid, 3-(4-((2,6-dichlorophenyl)carbonylamino)-2-oxohydropyrimidinyl)-2-((2-methylspiro(1,2,3,4-tetrahydroisoquinoline-1,4'-piperidine)-11-yl)carbonylamino)propanoic acid, 2-((acetylspiro(1,2,3,4-tetrahydroisoquinoline-1,4'-piperidine)-11-yl)carbonylamino)-3-(4-((2,6-dimethoxyphenyl)carbonylamino)-2-oxohydropyrimidinyl)propanoic acid, 2-((acetylspiro(1,2,3,4-tetrahydroisoquinoline-1,4'-piperidine)-11-yl)carbonylamino)-3-(4-((2,6-dimethylphenyl)carbonylamino)-2-oxohydropyrimidinyl)propanoic acid, 2-((acetylspiro(1,2,3,4-tetrahydroisoquinoline-1,4'-piperidine)-1-yl)carbonylamino)-3-(4-((2,6-dichlorophenyl)carbonylamino)-2-oxohydropyrimidinyl)propanoic acid, 3-(4-((2,6-dichlorophenyl)carbonylamino)-2-oxohydropyrimidinyl)-2-((2-(phenylcarbonyl)spire(1,2,3,4-tetrahydroisoquinoline-1,4'-piperidine)-11-yl)carbonylamino)propanoic acid, 3-(4-((2,6-dimethylphenyl)carbonylamino)-2-oxohydropyrimidinyl)-2-((2-(phenylcarbonyl)spire(1,2,3,4-tetrahydroisoquinoline-1,4'-piperidine)-11-yl)carbonylamino)propanoic acid, 3-(4-((2,6-dichlorophenyl)carbonylamino)-2-oxohydropyrimidinyl)-2-((2-(phenylcarbonyl)spire(1,2,3,4-tetrahydroisoquinoline-1,4'-piperidine)-11-yl)carbonylamino)propanoic acid, 3-(4-((2,6-dichlorophenyl)carbonylamino)-2-oxohydropyrimidinyl)-2-((2-(methylsulfonyl)spire(1,2,3,4-tetrahydroisoquinoline-1,4'-piperidine)-11-yl)carbonylamino)propanoic acid, 3-(4-((2,6-dimethylphenyl)carbonylamino)-2-oxohydropyrimidinyl)-2-((2-(methylsulfonyl)spiro(1,2,3,4-tetrahydroisoquinoline-1,4'-piperidine)-11-yl)carbonylamino)propanoic acid, 3-(4-((2,6-dimethoxyphenyl)carbonylamino)-2-oxohydropyrimidinyl)-2-((2-(methylsulfonyl)spiro(1,2,3,4-tetrahydroisoquinoline-1,4'-piperidine)-11-yl)carbonylamino)propanoic acid, 3-(4-((2,6-dimethoxyphenyl)carbonylamino)-2-oxohydropyrimidinyl)-2-((2-(phenylsulfonyl)spiro( 1,2,3,4-tetrahydroisoquinoline-1,4'-piperidine)-11-yl)carbonylamino)propanoic acid, 3-(4-((2,6-dimethylphenyl)carbonylamino)-2-oxohydropyrimidinyl)-2-((2-(phenylsulfonyl)spiro(1,2,3,4-tetrahydroisoquinoline-1,4'-piperidine)-11-yl)carbonylamino)propanoic acid, 3-(4-((2,6-dichlorophenyl)carbonylamino)-2-oxohydropyrimidinyl)-2-((2-(phenylsulfonyl)spiro(1,2,3,4-tetrahydroisoquinoline-1,4'-piperidine)-11-yl)carbonylamino)propanoic acid, 3-(4-((2,6-dichlorophenyl)carbonylamino)-2-oxohydropyrimidinyl)-2-((2-(3-methylbutanoyl)spiro(1,2,3,4-tetrahydroisoquinoline-1,4'-piperidine)-11-yl)carbonylamino)propanoic acid, 3-(4-((2,6-dimethylphenyl)carbonylamino)-2-oxohydropyrimidinyl)-2-((2-(3-methylbutanoyl)spiro(1,2,3,4-tetrahydroisoquinoline-1,4'-piperidine)-11-yl)carbonylamino)propanoic acid, 3-(4-((2,6-dimethoxyphenyl)carbonylamino)-2-oxohydropyrimidinyl)-2-((2-(3-methylbutanoyl)spire(1,2,3,4-tetrahydroisoquinoline-1,4'-piperidine)-11-yl)carbonylamino)propanoic acid, 2-((2,6-dimethoxyphenyl)carbonylamino)-3-(4-((2-(methanesulfonyl)spiro(isoindoline-1,4'-piperidine)-10-yl)carbonylamino)-2-oxohydropyrimidinyl)propanoic acid, 2-((2,6-dimethylphenyl)carbonylamino)-3-(4-((2-(methanesulfonyl)spiro(isoindoline-1,4'-piperidine)-10-yl)carbonylamino)-2-oxohydropyrimidinyl)propanoic acid, 2-((2,6-dichlorophenyl)carbonylamino)-3-(4-((2-(methanesulfonyl)spiro(isoindoline-1,4'-piperidine)-10-yl)carbonylamino)-2-oxohydropyrimidinyl)propanoic acid, 2-((2,6-dichlorophenyl)carbonylamino)-3-(2-oxo-4-(spiro(isochroman-1,4'-piperidine-11-ylcarbonylamino)hydropyrimidinyl)propanoic acid, 2-((2,6-dimethylphenyl)carbonylamino)-3-(2-oxo-4-(spiro(isochroman-1,4'-piperidine-11-ylcarbonylamino)hydropyrimidinyl)propanoic acid, 2-((2,6-dimethoxyphenyl)carbonylamino)-3-(2-oxo-4-(spiro(isochroman-1,4'-piperidine-11-ylcarbonylamino)hydropyrimidinyl)propanoic acid, 2-((2,6-dimethoxyphenyl)carbonylamino)-3-(2-oxo-4-(spiro(3H,4H-benzo [d]thian-1,4'-piperidine-11-ylcarbonylamino)hydropyrimidinyl)propanoic acid, 2-((2,6-dimethylphenyl)carbonylamino)-3-(2-oxo-4-(spiro(3H,4H-benzo[d]thian-1,4'-piperidine-11-ylcarbonylamino)hydropyrimidinyl)propanoic acid, 2-((2,6-dichlorophenyl)carbonylamino)-3-(2-oxo-4-(spiro(3H,4H-benzo[d]thian-1,4'-piperidine-11-ylcarbonylamino)hydropyrimidinyl)propanoic acid, 3-(4-((2-acetylspiro( 1,2,3,4-tetrahydroisoquinoline-1,4'-piperidine)-11-yl)carbonylamino)-2-oxohydropyrimidinyl)-2-((2,6-dichlorophenyl)carbonylamino)propanoic acid, 3-(4-((2-acetylspiro(1,2,3,4-tetrahydroisoquinoline-1,4'-piperidine)-11-yl)carbonylamino)-2-oxohydropyrimidinyl)-2-((2,6-dimethylphenyl)carbonylamino)propanoic acid, 3 -(4-((2-acetylspiro(1,2,3,4-tetrahydroisoquinoline-1,4'-piperidine)-11-yl)carbonylamino)-2-oxohydropyrimidinyl)-2-((2,6-dimethoxyphenyl)carbonylamino)propanoic acid, 2-((2,6-dichlorophenyl)carbonylamino)-3-(4-((2-(2-methylpropanoyl)spiro(1,2,3,4-tetrahydroisoquinoline-1,4'-piperidine)-11-yl)carbonylamino)-2-oxohydropyrimidinyl)propanoic acid, 2-((2,6-dimethylphenyl)carbonylamino)-3-(4-((2-(2-methylpropanoyl)spire(1,2,3,4-tetrahydroisoquinoline-1,4'-piperidine)-11-yl)carbonylamino)-2-oxohydropyrimidinyl)propanoic acid, 2-((2,6-dimethoxyphenyl)carbonylamino)-3-(4-((2-(2-methylpropanoyl)spiro(1,2,3,4-tetrahydroisoquinoline-1,4'-piperidine)- 11-yl)carbonylamino)-2-oxohydropyrimidinyl)propanoic acid, 2-((2,6-dichlorophenyl)carbonylamino)-3-(2-oxo-4-((2-(phenylcarbonyl)spiro(1,2,3,4-tetrahydroisoquinoline-1,4'-piperidine)-11-yl)carbonylamino)hydropyrimidinyl)propanoic acid, 2-((2,6-dimethylphenyl)carbonylamino)-3-(2-oxo-4-((2-(phenylcarbonyl)spire(1,2,3,4-tetrahydroisoquinoline-1,4'-piperidine)-11-yl)carbonylamino)hydropyrimidinyl)propanoic acid, 2-((2,6-dimethoxyphenyl)carbonylamino)-3-(2-oxo-4-((2-(phenylcarbonyl)spire(1,2,3,4-tetrahydroisoquinoline-1,4'-piperidine)-11-yl)carbonylamino)hydropyrimidinyl)propanoic acid, 2-((2,6-dimethoxyphenyl)carbonylamino)-3-(4-((2-(methylsulfonyl)spire(1,2,3,4-tetrahydroisoquinoline-1,4'-piperidine)-11-yl)carbonylamino)-2-oxohydropyrimidinyl)propanoic acid, 2-((2,6-dimethylphenyl)carbonylamino)-3-(4-((2-(methylsulfonyl)spire(1,2,3,4-tetrahydroisoquinoline-1,4'-piperidine)-11-yl)carbonylamino)-2-oxohydropyrimidinyl)propanoic acid, 2-((2,6-dichlorophenyl)carbonylamino)-3-(4-((2-(methylsulfonyl)spire(1,2,3,4-tetrahydroisoquinoline-1,4'-piperidine)-11-yl)carbonylamino)-2-oxohydropyrimidinyl)propanoic acid, 2-((2,6-dichlorophenyl)carbonylamino)-3-(2-oxo-4-((2-(phenylsulfonyl)spiro(1,2,3,4-tetrahydroisoquinoline-1,4'-piperidine)-11-yl)carbonylamino)hydropyrimidinyl)propanoic acid, 2-((2,6-dimethylphenyl)carbonylamino)-3-(2-oxo-4-((2-(phenylsulfonyl)spiro(1,2,3,4-tetrahydroisoquinoline-1,4'-piperidine)-11-yl)carbonylamino)hydropyrimidinyl)propanoic acid, and 2-((2,6-dimethoxyphenyl)carbonylamino)-3-(2-oxo-4-((2-(phenylsulfonyl)spiro( 1,2,3,4-tetrahydroisoquinoline-1,4'-piperidine)-11-yl)carbonylamino)hydropyrimidinyl)propanoic acid.

The processes for producing the compounds represented by Formula I (hereinafter, for example, "the compounds represented by Formula I" may also be indicated simply as "Formula I") will now be described. However, the process for producing each of the compounds is not restricted to that described herein In the various production processes, the reaction conditions may be appropriately selected from those described below.

Among the compounds represented by Formula I, those wherein l=0, m=1, R₁ and R₂ are hydrogen atoms, A is Formula XI, B is amide, C is Formula XIII, G does not exist, R₃ and R₄ are independently hydrogen, C₁-C₆ linear alkyl, C₃-C₈ branched alkyl, or phenyl or benzyl, which phenyl or benzyl are substituted with 0 to 2 substituents selected from the group consisting of halogen, methyl, methoxy, cyano, nitro, hydroxyl, amino and tetrazole, that is, those represented by Formula XXI; those wherein R₃ is hydrogen and R₄ is Formula II, that is, those represented by Formula XIV; and those wherein R₃ and R₄ cooperatively form Formula IV, VI, VII, VIII or IX, that is, those represented by Formula XXII: 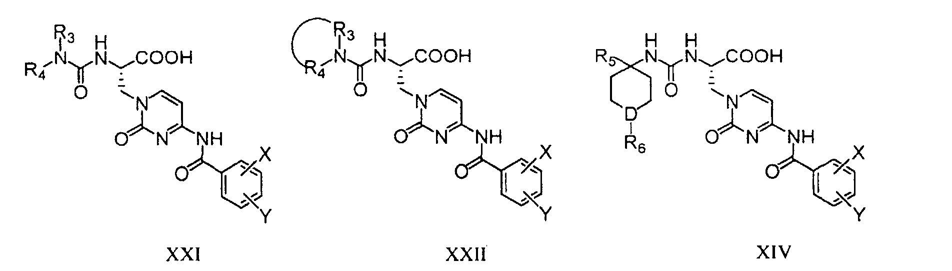 (wherein D, X, Y, R₃, R₄, R₅ and R₆ represent the same meanings as described above)
may be produced by reacting Formula XV: 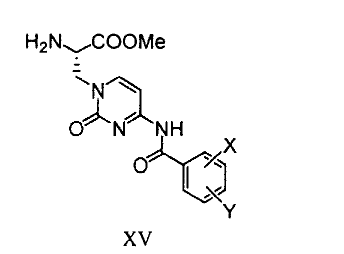 (wherein X and Y represent the same meanings as described above) with p-nitrophenyl chloroformate in a solvent such as dichloromethane, dimethoxyethane or acetonitrile, in the presence of sodium hydrogen carbonate in an amount of about 1 to 4 equivalents with respect to Formula XV; and then reacting the resultant with Formula XXIII, XXIV or XXV in the presence of a tertiary amine such as triethylamine or diisopropylamine in an amount of about 1 to 4 equivalents with respect to Formula XV. Alternatively, they may be produced by reacting Formula XV with Formula XXIII, XXIV or XXV in the presence of a tertiary amine such as triethylamine or diisopropylamine in an amount of about 1 to 4 equivalents with respect to Formula XXIII, XXIV or XXV and treating the mixture with diphosgene, triphosgene or carbozylimidazole in an amount of about 0.5 to 2 equivalents with respect to Formula XXIII, XXIV or XXV: 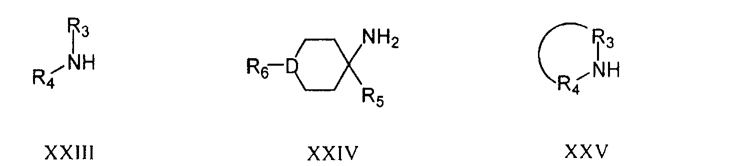 (wherein X, Y, R₃, R₄, R₅ and R₆ represent the same meanings as described above), and then hydrolyzing the ester group by aqueous sodium hydroxide solution or the like in an alcoholic solvent such as methanol. In the reaction using p-nitrophenyl chloroformate, the mixing ratio between Formula XV and Formula XXIII, XXIV or XXV is not restricted, and usually about 1:1 to 1:2, and the reaction may be carried out usually at a temperature from about 0°C to room temperature for about 1 hour to 24 hours. The hydrolysis by the base such as aqueous sodium hydroxide solution may be carried out usually at a temperature from 0°C to room temperature for about 1 hour to 24 hours, and the amount of the base to be added may be usually about 1 to 4 equivalents with respect to Formula XV, although the reaction conditions are not restricted to those mentioned above.

Formula XV may be produced by the steps below.

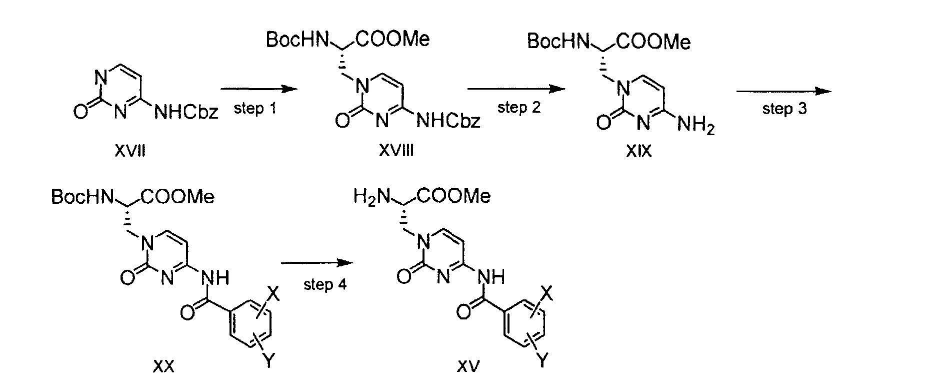

Step 1 may be carried out by reacting N-(2-oxohydropyrimidine-4-yl)(phenylmethoxy)carboxamide Formula XVII and (t-butoxy)-N-(2-oxooxetane-3-yl) carboxamide usually at about 0°C to 50°C for about 1 to 24 hours in a solvent such as tetrahydrofuran or dimethylformamide, and then esterifying the product. As the base, usually, sodium hydride, potassium t-butoxide or the like may be used, but other bases may also be used. The mixing ratio among Formula XVII, (t-butoxy)-N-(2-oxooxetane-3-yl) carboxamide and the base is not restricted, and usually about 1:1:1 to 1:2:2. The esterification may be attained by various methods including those using trimethylsillyldiazometane/methanol, thionyl chloride/methanol or methyl iodide-potassium carbonate/acetone, but the esterification method is not restricted thereto.

Step 2 is the step for removing benzyloxycarbonyl group (referred to as "Cbz" for short) which is a protective group on the nitrogen atom. This step may be attained by hydrogenating the reactant using a catalytic amount of a palladium catalyst such as palladium/carbon or palladium hydroxide, or using a platinum catalyst such as platinum dioxide in an alcoholic solvent such as methanol or ethanol, or in a polar solvent such as ethyl acetate, tetrahydrofuran or dioxane. The reaction temperature is not restricted, and usually a temperature of about 10 to 30°C is appropriate. The reaction time is not restricted and is appropriately selected depending on the reaction temperature. Usually the reaction time may be about 1 to 20 hours.

Step 3 is the step for reacting Formula XIX and Formula XVI to produce Formula XX. In cases where the symbol Z in Formula XVI is chloro or bromo, the step may be carried out by reacting Formula XIX and Formula XVI in a solvent such as tetrahydrofuran, dimethylformamide, chloroform or dichloromethane, in the presence of a tertiary amine such as pyridine, triethylamine or diisopropylamine, usually at about 0°C to 60°C for about 1 hour to 24 hours. 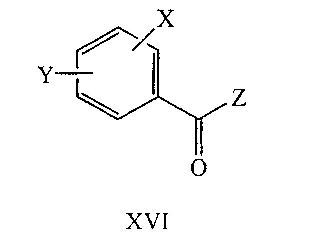 (wherein X and Y represent the same meanings as described above, and Z represents chloro, bromo or hydroxyl)

The mixing ratio of Formula XIX to XVI is not restricted, and is usually about 1:1 to 1:2. The amount of the tertiary amine to be added is not restricted, and usually about 1 to 4 equivalents with respect to Formula XVI. In cases where Z in Formula XVI is hydroxyl, usually, a condensing agent such as dicyclohexylcarbodiimide (DCC), benzotriazole-1-yloxytris(dicyclopentylamino)phosphoniumhexafluoro phosphite salt (PyBOP), benzotriazole-1-yloxytris(dimethylamino)phosphoniumhexafluoro phosphite salt (BOP), diphenylphosphoryl azide (DPPA) or 1-ethyl-3-[3-(dimethylamino))propyl]carbodiimide (WSC) is used in a solvent such as tetrahydrofuran, dimethylformamide, chloroform or dichloromethane in the presence of a tertiary amine such as triethylamine, diisopropylamine, N-methylmorpholine or N-methylpiperidine. The amount of such a condensing agent to be added is not restricted, and usually about 1 to 3 equivalents with respect to Formula XVI. Addition of an additive such as 1-hydroxybenzotriazole (HOBT) may be advantageous in the proceeding of the reaction in some cases.

Step 4 is the step of removing t-butoxycarbonyl group (referred to as "Boc" for short) on the nitrogen atom. This step may be carried out by usually using trifluoroacetic acid, hydrochloric acid, hydrobromic acid or the like in a halogen-containing solvent such as chloroform or dichloromethane. Alternatively, this step may be carried out by using trifluoroacetic acid alone. The reaction temperature is not restricted, and usually a temperature between 0°C and room temperature is selected. The reaction time may be appropriately selected depending on the reaction temperature and the like, and usually, the reaction time may be about 1 to 24 hours.

Among the compounds represented by Formula I wherein l=0, m=1, R₁ is hydrogen, A is a nitrogen atom, R₂ and R₃ cooperatively form Formula III, A is Formula XI, B is amide, C is Formula XIII, and G does not exist, that is, those represented by Formula XXVI: 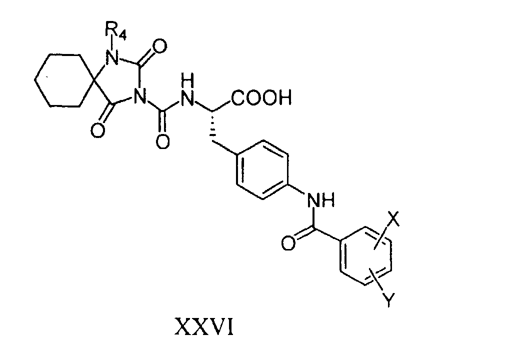 (wherein X, Y and R₄ represent the same meanings as described above) may be produced by hydrolyzing Formula XXVII 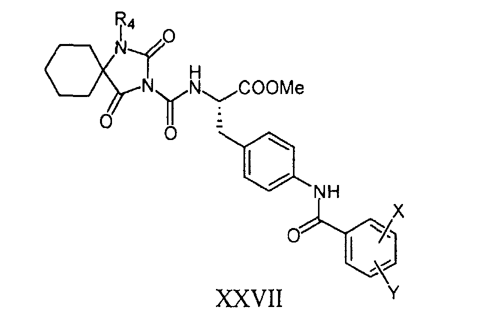 (wherein X, Y and R₄ represent the same meanings as described above) by aqueous sodium hydroxide solution or the like, in an alcoholic solvent such as methanol. The hydrolysis by a base such as aqueous sodium hydroxide solution may be attained, although not restricted, at a temperature of about 0°C to room temperature for about 1 to 24 hours. The amount of the base to be added is usually about 1 to 4 equivalents.

Formula XXVII may be carried out by the following steps.

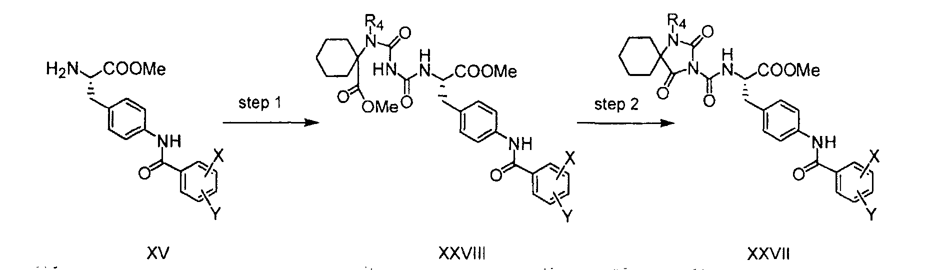

Step 1 is the step of treating methyl 1-aminocyclohexanecarboxylate with di-t-butyl dicarbonate and dimethylaminopyridine in a solvent such as acetonitrile and then reacting the reaction product with Formula XV to obtain Formula XXVIII. The amounts of the di-t-butyldicarbonate and dimethylaminopyridine are not restricted, and usually about 1 to 3 equivalents, and 0.2 to 1 equivalent, respectively. The reaction temperature and the reaction time are not restricted, and may be about 0°C to room temperature for about 1 to 24 hours. This step may also be carried out under the same conditions as employed in the reaction between Formula XV and Formula XIII, XXIV or XXV.

Step 2 is the step of obtaining Formula XXVII using a base such as sodium hydride or potassium t-butoxide in an alcoholic solvent such as methanol, at about 0°C to room temperature. The amount of the base to be added is not restricted, and may be usually about 1 to 3 equivalents. The reaction time is not restricted, and may be usually about 1 to 24 hours.

Among the compounds represented by Formula XXIV, those wherein D is a carbon atom, R₆ is a hydrogen atom, R₅ is -C(O)NHR₁₃, R₁₃ is C₁-C₆ linear alkyl, C₃-C₈ branched alkyl, or phenyl substituted with 0 to 2 substituents selected from the group consisting of halogen, methyl, methoxy, cyano, nitro, hydroxyl, amino and tetrazole, that is, those represented by Formula XXIX 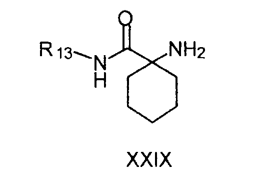 (wherein R₁₃ represents the same meanings as described above) may be produced by the following steps. 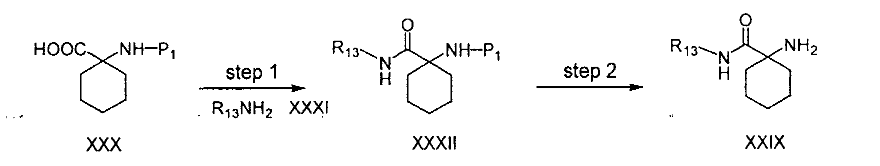 (wherein P₁ represents a protective group and R₁₃ represents the same meanings as described above)

In Formula XXX, P₁ represents a protective group of the nitrogen atom. The protective group and the method for introducing the protective group are described in, for example, Green and Wuts, "Protective Group in Organic Synthesis" (3rd Edition). The protective groups may be appropriately used in accordance with the reaction conditions described therein.

Step 1 may be attained by reacting Formula XXX and Formula XXXI in a solvent such as tetrahydrofuran, dimethylformamide or dichloromethane, in the presence of a tertiary amine such as triethylamine, diisopropylamine, morpholine or N-methylpiperidine. The reaction between Formula XXX and Formula XXXI may be usually carried out, although not restricted, at about 0°C to room temperature for about 1 hour to 24 hours. The mixing ratio of Formula XXX to Formula XXXI is not restricted, and may usually be about 1:1 to 1:2. The amount of the tertiary amine to be added is not restricted, and may usually be about 1 to 4 equivalents with respect to Formula XXX. Usually, as the condensing agent, dicyclohexylcarbodiimide (DCC), benzotriazole-1-yloxytris(dicyclopentylamino)phosphoniumhexafluoro phosphite salt (PyBOP), benzotriazole-1-yloxytris(dimethylamino)phosphoniumhexafluoro phosphite salt (BOP), diphenylphosphoryl azide (DPPA), 1-ethyl-3-[3-(dimethylamino))propyl]carbodiimide (WSC) or the like is used. The amount of the condensing agent is not restricted, and usually about 1 to 3 equivalents with respect to Formula XXX. Addition of an additive such as 1-hydroxybenzotriazole (HOBT) may be advantageous in the proceeding of the reaction in some cases.

Step 2 is the step of removing the protective group on the nitrogen atom. The method is described in the above-mentioned "Protective Group in Organic Synthesis" (3rd Edition). The reaction conditions described therein may appropriately be selected.

Among the compounds represented by Formula XXV, those represented by Formula VI, R₁₀ is represented by R₁₄C(O)NH-, R₁₄ is C₁-C₆ linear alkyl, C₃-C₈ branched alkyl, C₅-C₇ cycloalkyl, or phenyl substituted with 0 to 2 substituents selected from the group consisting of halogen, methyl, methoxy, cyano, nitro, hydroxyl, amino and tetrazole, that is, those represented by Formula XXXIII: 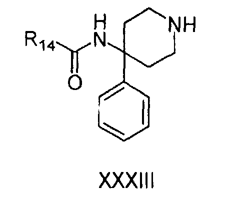 (wherein R₁₄ represents the same meanings as described above) may be produced by the following steps. 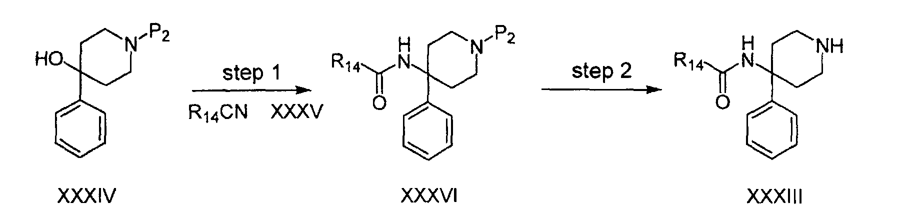 (wherein R₁₄ represents the same meanings as described above, P₂ represents a protective group).

In Formula XXXIV, P₂ represents a protective group of the nitrogen atom. The protective group and the method for introducing the protective group are described in the above-mentioned "Protective Group in Organic Synthesis" (3rd Edition). The protective groups may be appropriately used in accordance with the reaction conditions described therein.

Step 1 may be attained by reacting Formula XXXIV and Formula XXXV in the presence of acetic acid, sulfuric acid or Lewis acid for usually 1 to 24 hours. The reaction temperature is not restricted and usually from ice to about 100°C. The mixing ratio of Formula XXXIV to Formula XXXV is not restricted and usually about 1:1 to 1:10. The equivalent of the acid is not restricted, and usually an excess amount with respect to Formula XXXIV is used.

Step 2 may be appropriately carried out in accordance with the reaction conditions described in the above-mentioned "Protective Group in Organic Synthesis" (3rd Edition).

Among the compounds represented by Formula XXV, those represented by Formula IV, wherein E is a carbon atom, R₇ is a hydrogen atom and R₈ is Formula V, that is, those represented by Formula XXXVII: 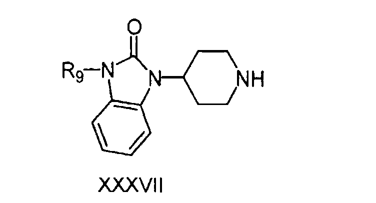 (wherein R₉ represents the same meanings as described above) may be produced by the following steps. 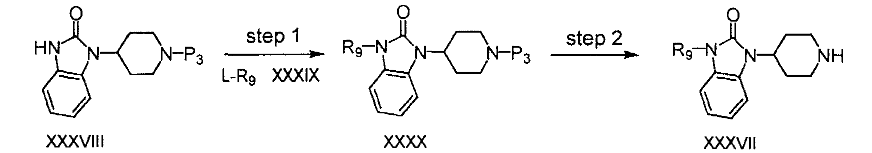 (wherein R₉ represents the same meanings as described above, L represents a leaving group such as halogen, methanesulfonyloxy or p-toluenesulfonyloxy, P₃ represents a protective group).

In Formula XXXVIII, P₃ represents a protective group of the nitrogen atom. The protective group and the method for introducing the protective group are described in the above-mentioned "Protective Group in Organic Synthesis" (3rd Edition). The protective groups may be appropriately used in accordance with the reaction conditions described therein.

Step 1 may be attained by reacting Formula XXXVIII and Formula XXXIX for about 1 to 24 hours in a solvent such as dimethylformamide, dimethylacetylamide or acetonitrile, in the presence of a base such as potassium carbonate, potassium hydroxide, diisopropylamine or triethylamine. The mixing ratio of Formula XXXVIII to Formula XXXIX is not restricted, and is usually about 1:1 to 1:3. The equivalent of the base is not restricted, and is usually 1 to 4 equivalents. The reaction temperature is not restricted, and may be usually about room temperature to reflux.

Step 2 may be appropriately carried out in accordance with the reaction conditions described in the above-mentioned "Protective Group in Organic Synthesis" (3rd Edition).

Among the compounds represented by Formula XXV, Formula XXXXI: 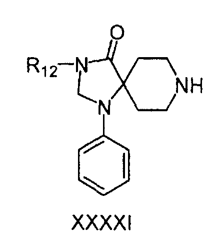 (wherein R₁₂ represents the same meanings as described above) represented by Formula IX may be produced by the following steps. 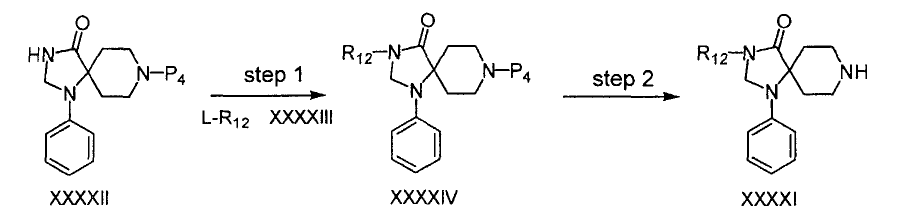 (wherein R₁₂ represents the same meanings as described above, L represents a leaving group such as halogen, methanesulfonyloxy or p-toluenesulfonyloxy, P₄ represents a protective group).

In Formula XXXXII, P₄ represents a protective group of the nitrogen atom. The protective group and the method for introducing the protective group are described in the above-mentioned "Protective Group in Organic Synthesis" (3rd Edition). The protective groups may be appropriately used in accordance with the reaction conditions described therein.

Step 1 may be carried out in the same manner as in step 1 in the production process of Formula XXXVII.

Step 2 may be appropriately carried out in accordance with the reaction conditions described in the above-mentioned "Protective Group in Organic Synthesis" (3rd Edition).

Among the compounds represented by Formula XXV, Formula XXXXV represented by Formula VII, and Formula XXXXVI represented by Formula VIII, may be produced by the methods described in Tetrahedoron, 53 , 10983 , 1997 and Chem.Pharm.Bull. ,46, 242, 1998, Chem.Pharm.Bull. ,46, 1538, 1998, respectively. 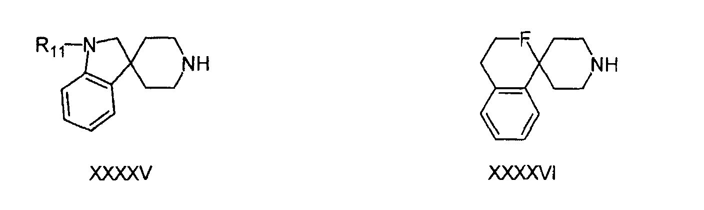 (wherein F and R₁₁ represent the same meanings as described above).

The reaction products obtained by the above-described processes may be isolated and purified in the form of a free compound, a salt or a solvate such as hydrate. The salt may be produced by a usual salt-producing treatment.

Isolation and purification may be carried out by ordinary chemical processes such as extraction, condensation, evaporation, crystallization, filtration, recrystallization and various column chromatography.

Various isomers may be isolated by conventional methods utilizing the differences in the physicochemical properties between the isomers. Optical isomers may be separated by a general optical resolution method such as fractional crystallization or chromatography. Optical isomers may also be produced by an appropriate optically active compound as the starting material.

In cases where the novel urea derivatives used in the present invention have one or more asymmetric carbon atoms, there exist racemic compounds, diasteromers and optical isomers. In the present invention, any of these may be used.

Examples of the pharmaceutically acceptable salts of the compounds represented by Formula I include inorganic salts such as ammonium salt, alkaline metal salts (e.g., sodium salt and potassium salt), alkaline earth metal salts (e.g., calcium salt and magnesium salt); organic salts such as dicyclohexylamine salt, N-methyl-D-glucamine salt, ethanolamine salt, diethanolamine salt, triethanolamine salt, diisopropanolamine salt and tris(hydroxymethyl)aminemethane salt; and lysine- and arginate-addition salts.

The inhibitory activity of the compound according to the present invention against the adhesion of VLA-4 may be determined by using an adhesion-measuring system in which the adhesion between VLA-4-expressing cells such as Ramos cells or Jurkat cells and fibronectin or fibronectin fragment such as a peptide containing CS-1 sequence (Gly Pro Glu Ile Leu Asp Val Pro Ser Thr) (hereinafter referred to as "CS-1 peptide"), immobilized on an immunoplate is measured. Alternatively, a binding-measuring system in which the adhesion between VLA-4 protein and fibronectin or fibronectin fragment such as CS-1 peptide, immobilized on an immunoplate may be used. In the present invention, it is preferred to evaluate the inhibitory activity of a compound using a binding-measuring system in which adhesion between a chimera protein of VLA-4-immunoglobulin (VLA-4-IgG chimera protein) and CS-1 peptide (Japanese Patent Application No. H9-234544), but the method is not restricted thereto. The "VLA-4-IgG chimera protein" herein means the heterodimer complex of the chimera protein between α4 of VLA-4 and immunoglobulin (hereinafter referred to as "VLAα4-IgG chimera protein") and a chimera protein between β1 of VLA-4 and immunoglobulin (hereinafter referred to as "VLAβ1-IgG chimera protein"). As the immunoglobulin, although heavy chain or light chain of IgG, IgM or the like may be used, IgG 1 heavy chain is used in the present invention. When testing the inhibitory effect of a compound, it is preferred to mix VLA-4-IgG chimera protein and the test compound previously.

Since the compounds according to the present invention have inhibitory activities against adhesion of VLA-4, and so inhibit accumulation of leukocytes at the inflammatory site, they may be used as therapeutic drugs against chronic inflammatory diseases. Examples of the chronic inflammatory diseases include allergic inflammatory diseases such as bronchial asthma, atopic dermatitis and allergic rhinitis, hepatitis, nephritis, autoimmune diseases such as chronic rheumatoid arthritis and multiple sclerosis, graft rejections after organ transplantation, type I diabetes, Crohn's disease and ulcerative colitis. In addition to these, they may be used as therapeutic drugs for the prevention of postoperative restenosis, arteriosclerosis and the like.

When using the compound of the present invention as a therapeutic drug against the above-mentioned diseases, the compound represented by Formula I or a base addition salt thereof may be administered as it is in the form of powder, or may be administered as a medical composition in the form of an appropriate formulation, orally or parenterally (e.g., percutaneous administration, intravenous administration, rectal administration and inhalation) to mammals.

Examples of the formulation for administration include tablets, powders, balls, capsules, granules, syrups, liquids, injection solutions, emulsions, suspensions and suppositories. These formulations may be prepared by the methods which *per se* are known, and contain various carriers usually used in the field of formulation. Examples thereof include vehicles, lubricants, binders and disintegrators for solid formulations; and solvents, solubilizers, suspending agents and soothing agents for liquid formulations. Additives such as antiseptics, antioxidants, coloring agents, sweeteners, absorbents, and wetting agents may be used.

Examples of the vehicles include lactose, D-mannitol, starch, sucrose, corn starch, crystalline cellulose and light anhydrous silicic acid. Examples of the lubricants include magnesium stearate, calcium stearate, talc and colloidal silica. Examples of the binders include crystalline cellulose, D-mannitol, dextrin, hydroxypropyl cellulose, hydroxypropylmethyl cellulose, polyvinylpyrrolidone, starch, sucrose, gelatin, methyl cellulose and sodium carboxymethyl cellulose. Examples of the disintegrators include starch, carboxymethyl cellulose, calcium carboxymethyl cellulose, cross carmelose sodium, sodium carboxymethyl starch and L-hydroxypropyl cellulose. Examples of the solvents include water for injection, alcohol, propylene glycol, Macrogol, sesame oil and corn oil. Examples of the solubilizers include polyethylene glycol, propylene glycol, D-mannitol, benzyl benzoate, ethanol, cholesterol, triethanolamine, sodium carbonate and sodium citrate. Examples of the suspending agents include surfactants such as stearyl triethanolamine, sodium lauryl sulfate, laurylamino propionate, lecithin, benzalkonium chloride, benzethonium chloride and glycerin monostearate, and hydrophilic macromolecules such as polyvinyl alcohol, polyvinyl pyrrolidone, methyl cellulose, hydroxymethyl cellulose, hydroxyethyl cellulose and hydroxypropyl cellulose. Examples of the isotonic agents include glucose, sodium chloride, D-sorbitol and D-mannitol. Examples of the buffering agents include buffering solutions containing a phosphoric acid salt, acetic acid salt, carbonic acid salt or citric acid salt. An example of the soothing agents is benzylalcohol. Examples of the antiseptics include p-oxybenzoic acid esters, chlorobutanol, benzylalcohol, phenetyl alcohol, dehydroacetic acid and sorbic acid. Examples of the antioxidants include sulfurous acid salts and ascorbic acid.

The effective dose and the number of-times of administration of the compounds represented by Formula I and pharmaceutically acceptable salts thereof differ depending on the administration form, age and bodyweight of the patient, the type and severity of the disease to be treated, and usually, 1 to 1000 mg, preferably 1 to 300 mg of the compound may be administered once or in several times per day per adult.

The above-mentioned formulations may contain one or more other effective components for therapy of other disease(s). Examples thereof include steroid drugs, nonsteroidal anti-inflammatory drug, lipoxygenase inhibitors, leucotriene inhibitors, bronchodilators, thromboxane synthesis inhibitors, thromboxane antagonists, histamine antagonists, histamine release inhibitors, platelet activating factor (PAF) inhibitors, serotonin antagonist, adenosine receptor antagonists, adrenalin β receptor stimulators, immunosuppressors and immunomodulators.

### Examples

The effect of the present invention will now be described concretely by way of examples thereof. It should be noted that the present invention is not restricted to the examples.

### Example 1

### Methyl 2-((t-butoxy)carbonylamino)-3 -(2-oxo-4-((phenylmethoxy)carbonylamino)hydropyrimidinyl)propionate (1)

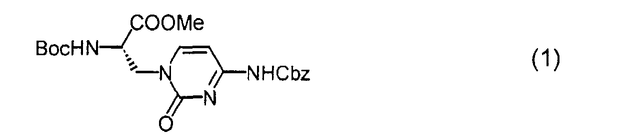

Under argon atmosphere, 4.86 g (19.8 mmol) of N-(2-oxohydropyrimidine-4-yl)(phenylmethoxy)carboxamide was suspended in 50 ml of DMF, and 0.76 g (19 mmol) of sodium hydride was added to the mixture while cooling the mixture in ice, followed by stirring the resulting mixture at room temperature for 40 minutes. To the reaction mixture, 3.37 g (18 mmol) of (t-butoxy)-N-(2-oxooxetane-3-yl)carboxamide in 10 ml of DMF was added while cooling the reaction mixture in ice, followed by stirring the resulting mixture at room temperature overnight. Water was added to the reaction mixture, and precipitated solids were removed by filtration. To the filtrate, 1N hydrochloric acid was added and the resultant was extracted with chloroform. Organic phases were combined, washed with saturated saline, dried over anhydrous sodium sulfate and concentrated. The residue was dissolved in 20 ml of methanol, and 20 ml of trimethylsillyldiazomethane was added while cooling the mixture in ice, followed by stirring the resulting mixture for 30 minutes. The reaction solution was concentrated and the residue was purified by silica gel column chromatography (hexane:ethyl acetate = 1:1) to obtain 4.02 g of methyl 2-((t-butoxy)carbonylamino)-3 -(2-oxo-4-((phenylmethoxy)carbonylamino)hydropyrimidinyl)propionate (yield: 50%). LR-MS(m/z):446(M⁺)
NMR(300MHz,CD₃OD, δ ppm):3.75(3H,s),3.79-3.93(1H,m),4.46-455(1H,m),4.62-4.68(1H,m),5.21(2H,s),7.07-7.28(2H,m),7.32-7.43(4H,m),7.80(1H,m)

### Example 2

### Methyl 3-(4-amino-2-oxohydropyrimidinyl)-2-((t-butoxy)carbonylamino) propionate (2)

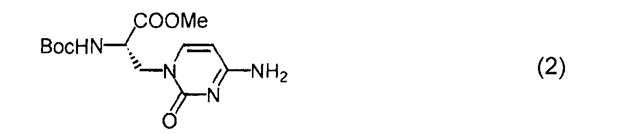

Under argon atmosphere, 4.02 g (9.0 mmol) of methyl 2-((t-butoxy)carbonylamino)-3-(2-oxo-4-((phenylmethoxy)carbonylamino)hydropyrimidinyl)propionate was dissolved in 20 ml of methanol and 400 mg of 10% palladium/carbon was added thereto. The mixture was subjected to hydrogen replacement and stirred at room temperature for 1 hour. The reaction mixture was filtered through Celite and the filtrate was concentrated. The residue was purified by silica gel column chromatography (chloroform:methanol = 4:1) to obtain 2.53 g of methyl 3-(4-amino-2-oxohydropyrimidinyl)-2-((t-butoxy)carbonylamino) propionate (yield: 90%). LR-MS(m/z):312(M⁺)
NMR(300MHz,CD₃OD, δ ppm): 1.39(9H,s),2.31(2H,s),3.73(3H,s),3.75-3.82(1H,m), 4.33-4.40(1H,m), 4.53-4.60(1H,m),5.80(1H,m),7.05-7.21(1H,m),7.4(1H,m)

### Example 3

### Methyl 3-(4-((2,6-dichlorophenyl)carbonylamino)-2-oxohydropyrimidinyl)-2-((t-butoxy)carbonylamino) propionate (3)

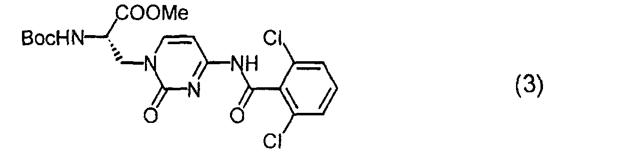

In 15 ml of pyridine and 10 ml of dichloromethane, 2.96 g (9.48 mmol) of methyl 3-(4-amino-2-oxohydropyrimidinyl)-2-((t-butoxy)carbonylamino) propionate was dissolved, and 2.7 ml (19.0 mmol) of dichlorobenzoyl chloride was added, followed by stirring the resulting mixture at 50°C for 2 hours. Methanol was added to the reaction solution and the mixture was concentrated. To the residue, 3N hydrochloric acid was added and the resultant was extracted with chloroform. The organic phase was washed with saturated saline, dried over anhydrous sodium sulfate and concentrated. The residue was purified by silica gel column chromatography (chloroform:methanol = 20:1) to obtain 3.35 g of methyl 3-(4-((2,6-dichlorophenyl)carbonylamino)-2-oxohydropyrimidinyl)-2-((t-butoxy)carbonylamino) propionate (yield: 73%).
LR-MS(m/z):484(M⁺)
IR(KBr):3442,3222,2962,1701,1627,1562,1492,1435,1369,1334,1303,1250,1162, 788cm-1
NMR(300MHz,CD₃OD, δ ppm):1.38(9H,s),3.77(3H,s),3.83-3.94(1H,m), 4.55-4.63(1H,m), 4.68-4.77(1H,m), 7.41-7.55(4H,m),7.93-7.98(1H,m)

### Example 4

### Methyl 2-amino-3-(4-((2,6-dichlorophenyl)carbonylamino)-2-oxohydropyrimidinyl) propionate (4)

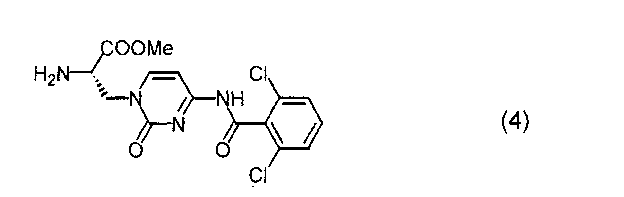

Under argon atmosphere, 202 mg of methyl 3-(4-((2,6-dichlorophenyl)carbonylamino)-2-oxohydropyrimidinyl)-2-((t-butoxy)carbonylamino) propionate was suspended in 9 ml of dichloromethane, and I ml of trifluoroacetic acid was added thereto while cooling the mixture in ice. The reaction solution was stirred at room temperature for 3 hours and concentrated. The residue was dissolved in ethyl acetate and saturated aqueous sodium hydrogen carbonate solution was added to the mixture while cooling the mixture in ice, followed by extraction of the resulting mixture with ethyl acetate. The organic phases were combined, washed with saturated saline, dried over anhydrous sodium sulfate and concentrated to obtain 156 mg of methyl 2-amino-3-(4-((2,6-dichlorophenyl)carbonylamino)-2-oxohydropyrimidinyl) propionate (97%).
LR-MS(m/z):385(M⁺+H)
NMR(300MHz,CD₃OD,δ ppm):3.74(3H,S),3.91-4.02(2H,m),4.23-4.30(1H,m),7.38-7.57(3H,m),8.03-8.05(1H,m)

### Example 7

### Methyl 3-(4-((2,6-dichlorophenyl)carbonylamino)-2-oxohydropyrimidinyl)-2-((((N-phenylcarbamoyl)cyclohexyl)amino)carbonylamino) propionate (7)

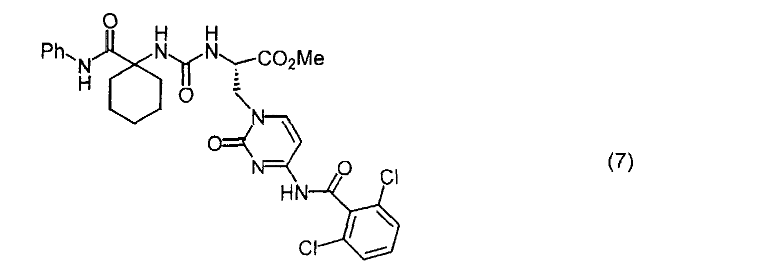

Under argon atmosphere, to a solution of 78 mg of methyl 2-amino-3-(4-((2,6-dichlorophenyl)carbonylamino)-2-oxohydropyrimidinyl) propionate in 5 ml of acetonitrile and 5 ml of dichloromethane, 90 mg of sodium hydrogen carbonate and 47 mg of chloroformic acid p-nitrophenyl ester were added, and the resulting mixture was stirred at room temperature for 2 hours. To the reaction mixture, a solution of 68 mg of (aminocyclohexyl)-N-benzamide and 0.1 ml of triethylamine in 3 ml of acetonitrile was added and the resulting mixture was stirred for 44 hours. To the reaction mixture, IN hydrochloric acid was added and the mixture was extracted with chloroform. Organic phases were combined, washed with saturated aqueous sodium hydrogen carbonate solution and with saturated saline, dried over anhydrous magnesium sulfate and concentrated. The residue was recrystallized from ethyl acetate to obtain 56 mg of methyl 3-(4-((2,6-dichlorophenyl)carbonylamino)-2-oxohydropyrimidinyl)-2-((((N-phenylcarbamoyl)cyclohexyl)amino)carbonylamino) propionate (yield: 45%).
mp.248°C
LR-MS(m/z):629(M⁺) IR(KBr):3393,2936,1713,1654,1628,1558,1493,1437,1369,1342,1304,1246,792,
757cm⁻¹
NMR(300MHz,DMSO-d₆, δ ppm):1.20-1.70(8H,m), 1.90-2.06(2H,m), 3.64(3H,s), 4.04(1H,m), 4.28(1H,m), 4.54(1H,m), 6.39(1H,s), 6.80(1H,d,J=8.2Hz), 6.99(1H,t,J=7.4Hz), 7.25(3H,t,J=8.0Hz), 7.45-7.61(5H,m), 7.89(1H,bd,J=7.5Hz), 9.34(1H,bs), 11.64(1H,bs)

### Example 8

### 3-(4-((2,6-dichlorophenyl)carbonylamino)-2-oxohydropyrimidinyl)-2-((((N-phenylcarbamoyl)cyclohexyl)amino)carbonylamino)propanoic acid (8)

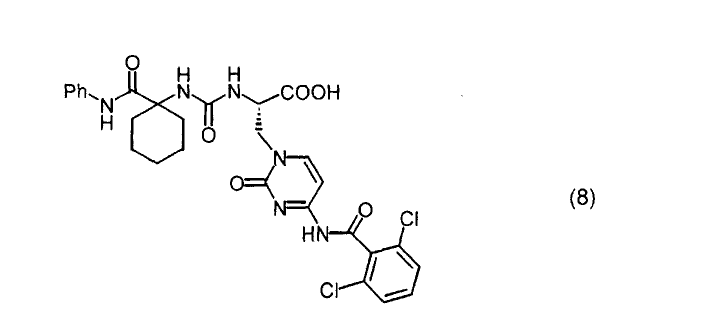

In 10 ml of methanol and 10 ml of tetrahydrofuran, 52 mg of methyl 3-(4-((2,6-dichlorophenyl)carbonylamino)-2-oxohydropyrimidinyl)-2-((((N-phenylcarbamoyl)cyclohexyl)amino)carbonylamino) propionate was dissolved, and 1 ml of 1N aqueous sodium hydroxide solution was added, followed by stirring the mixture at room temperature for 2 hours. The reaction solution was concentrated and 1N hydrochloric acid was added to the residue, followed by recovery of the precipitated crystals to obtain 29 mg of 3-(4-((2,6-dichlorophenyl)carbonylamino)-2-oxohydropyrimidinyl)-2-((((N-phenylcarbamoyl)cyclohexyl)amino)carbonylamino)propanoic acid (yield: 55%). mp.193-195°C
LR-MS(m/z):615(M⁺)
IR(KBr):3368,2938.2860,1719,1668,1656,1629,1599,1561,1492,1433,1367,1312,13 03,1244,1196,1158,1133,788,756,694cm⁻¹
NMR(300MHz,DMSO-d₆, δ ppm): 1.15-1.70(8H,m), 1.89-1.96(2H,m), 3.96(1H,dd,J=8.2, 13.0Hz), 4.34(1H,dd,J=4.4, 13.0Hz), 4.49(1H,m), 6.37(1H,s), 6.71(1H,d,J=8.2Hz), 6.98(1H,t,J=7.4Hz), 7.19-7.29(3H,m), 7.44-7.59(5H,m), 7.90(1H,d,J=7.4Hz), 9.34(1H,bs), 11.62(1H,bs)

### Example 9

### 3-(4-((2,6-dichlorophenyl)carbonylamino)-2-oxohydropyrimidinyl)-2-(pyrrolidinylcarbonylamino)propanoic acid (9)

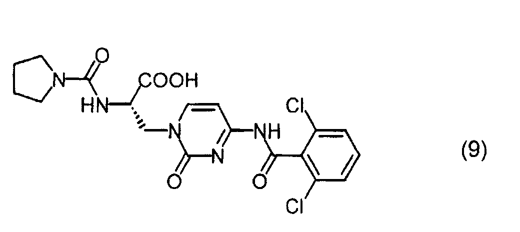

Under argon atmosphere, to a solution of 60 mg of methyl 2-amino-3-(4-((2,6-dichlorophenyl)carbonylamino)-2-oxohydropyrimidinyl) propionate in 4 ml of acetonitrile, 51.4 mg of saturated sodium hydrogen carbonate and 41.4 mg of chloroformic acid p-nitrophenyl ester were added while cooling the mixture in ice, and the resulting mixture was stirred at room temperature for 18 hours. To the reaction mixture, 16.3 µl of pyrrolidine and 27 µl of triethylamine were added and the resulting mixture was stirred at room temperature for 2 hours. After concentrating the reaction mixture, 10 ml of saturated aqueous sodium hydrogen carbonate solution was added and the resulting mixture was extracted with chloroform. Organic phases were combined, washed with 1N hydrochloric acid and with saturated saline, dried over anhydrous sodium sulfate and concentrated. The residue was dissolved in 3 ml of methanol and 0.3 ml of 1N aqueous sodium hydroxide solution was added, followed by stirring the resulting mixture at room temperature for 20 hours. To the reaction solution, 1N hydrochloric acid was added and the mixture was extracted with ethyl acetate. Organic phases were combined, washed with saturated saline, dried over anhydrous sodium sulfate and concentrated. The residue was purified by column chromatography (chloroform:methanol = 20:1) to obtain 14 mg of 3-(4-((2,6-dichlorophenyl)carbonylamino)-2-oxohydropyrimidinyl)-2-(pyrrolidinylcarbonylamino)propanoic acid (yield: 23%) LR-MS(m/z):466(M⁺-H)
IR(KBr):3423, 2925, 1719, 1627, 1561, 1493, 1432, 1365, 1306, 1246cm⁻¹
NMR(300MHz,CDCl₃, δ ppm): 1.80-2.00 (m, 4H), 3.22-3.40 (m, 4H), 4.39 (dd, J=14.0, 8.8 Hz, 1H), 4.48 (dd, J=14.0, 4.1 Hz, 1H), 4.62 (dd, J=8.8, 4.1 Hz, 1H),6.96 (br s, 1H), 7.30-7.40 (m, 3H), 7.63 (d, J=7.4 Hz, 1H), 8.11 (d, J=7.4 Hz, 1H). HR-MS:C₁₉H₁₉Cl₂N₅O₅ Calcd.: 466.0685, Found: 466.0639
[α]²⁰_{D}:-101.6° (c=0.70,MeOH)

### Example 10

### Methyl 3-(4-((2,6-dichlorophenyl)carbonylamino)-2-oxohydropyrimidinyl)-2-(piperidylcarbonylamino) propionate (10)

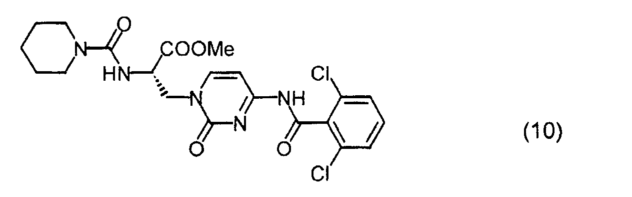

Under argon atmosphere, 61 mg of methyl 2-amino-3-(4-((2,6-dichlorophenyl)carbonylamino)-2-oxohydropyrimidinyl) propionate was dissolved in 2 ml of acetonitrile and 3 ml of dichloromethane, and 22 mg of saturated sodium hydrogen carbonate and 35 mg of chloroformic acid p-nitrophenyl ester were added while cooling the mixture in ice, followed by stirring the resulting mixture at room temperature for 2 hours. To the reaction mixture, 11 µl of piperidine and 56 µl of triethylamine were added and the resulting mixture was stirred at room temperature for 15 hours. After concentrating the reaction mixture, 10 ml of saturated aqueous sodium hydrogen carbonate solution was added and the resulting mixture was extracted with ethyl acetate. Organic phases were combined, washed with 1N hydrochloric acid and with saturated saline, dried over anhydrous sodium sulfate and concentrated. The residue was purified by column chromatography (dichloromethane/methanol = 20:1) to obtain 60 mg of methyl 3-(4-((2,6-dichlorophenyl)carbonylamino)-2-oxohydropyrimidinyl)-2-(piperidylcarbonylamino) propionate (yield: 76%).
LR-MS(m/z):495(M⁺)
IR(KBr):3372,2937,2855,1716,1658,1627,1562,1494,1433,1368,1305,1247,1131, 790cm⁻¹
NMR(300MHz,CDCl₃,δ ppm): 1.42-1.66(6H,m),3.23-3.38(4H,m),3.78(3H,s),4.28-4.42(2H,m),4.70-4.78(1H,m),6.10-6.18(1H,m),7.27-7.58(4H,m),7.82-7.91(1H,m), 9.38(1H,brs)
[α]_{D}²⁰: -115.5° (C=0.10, MeOH)

### Example 11

### 3-(4-((2,6-dichlorophenyl)carbonylamino)-2-oxohydropyrimidinyl)-2-(piperidylcarbonylamino) propanoic acid (11)

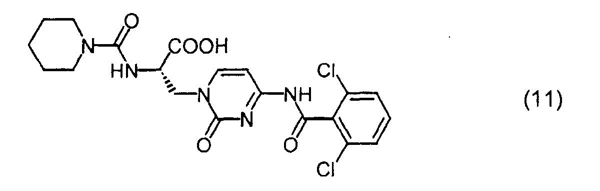

In 2 ml of methanol, 55 mg of methyl 3-(4-((2,6-dichlorophenyl)carbonylamino)-2-oxohydropyrimidinyl)-2-(piperidylcarbonylamino) propionate was added and 0.5 ml of 1N aqueous sodium hydroxide solution was added, followed by stirring the mixture at room temperature for 12 hours. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/n-hexane to obtain 46.4 mg of 3-(4-((2,6-dichlorophenyl)carbonylamino)-2-oxohydropyrimidinyl)-2-(piperidylcarbonylamino) propanoic acid (yield: 87%)
LR-MS(m/z):481(M⁺-H)
IR(KBr):3402,2938,2856,1715,1627,1494,1431,1367,1306,1250,1133,902,791cm⁻¹ NMR(300MHz,CDCl₃, δ ppm):1.50-1.70(6H,m),3.22-3.46(4H,m),4.31-4.53(2H,m),4.60-4.70(1 H,m),7.01-7.42(5H,m),7.60-7.71(1H,m),8.08(1H,brs) HR-MS:C₂₀H₂₀Cl₂N₅O₅ Calcd.: 480.0841, Found: 480.0863
[α]_{D}²⁰: -30.4° (C=0.04, MeOH)

### Example 12

### 2-(azaperhydroepinylcarbonylamino)-3-(4-((2,6-dichlorophenyl)carbonylamino)-2-oxohydropyrimidinyl)propanoic acid (12)

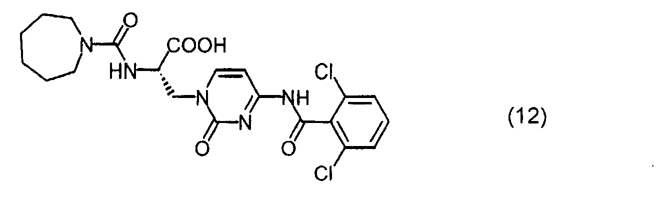

Under argon atmosphere, to a solution of 50 mg of methyl 2-amino-3-(4-((2,6-dichlorophenyl)carbonylamino)-2-oxohydropyrimidinyl) propionate in 4 ml of acetonitrile, 17.4 mg of saturated sodium hydrogen carbonate and 32 mg of chloroformic acid p-nitrophenyl ester were added while cooling the mixture in ice, and the resulting mixture was stirred at room temperature for 1 hour. To the reaction mixture, 28 µl of hexamethyleneimine and 100 µl of triethylamine were added, and the mixture was stirred at room temperature for 15 hours. After concentrating the reaction mixture, 10 ml of saturated aqueous sodium hydrogen carbonate solution was added and the resulting mixture was extracted with chloroform. Organic phases were combined, washed with saturated saline, dried over anhydrous sodium sulfate and concentrated. The residue was dissolved in 3 ml of methanol, and 0.3 ml of 1N aqueous sodium hydroxide solution was added, followed by stirring the resulting mixture at room temperature for 20 hours. To the reaction solution, 1N hydrochloric acid was added and the resulting mixture was extracted with ethyl acetate. Organic phases were combined, washed with saturated saline, dried over anhydrous sodium sulfate and concentrated. The residue was purified by column chromatography (dichloromethane/methanol = 20:1) to obtain 26.2 mg of 2-(azaperhydroepinylcarbonylamino)-3-(4-((2,6-dichlorophenyl)carbonylamino)-2-oxohydropyrimidinyl)propanoic acid (yield: 39%). LR-MS(m/z):494(M⁺-H)
IR(KBr):3346, 2926, 1727, 1703, 1647, 1562, 1529, 1488, 1434, 1371, 1314, 1253cm⁻¹
NMR(300MHz,CD₃OD, δ ppm):1.50-1.80 (m, 8H), 3.35-3.45 (m, 4H), 4.13 (dd, J=13.5, 9.9 Hz, 1H), 4.62-4.80 (m, 2H), 7.50-7.60 (m, 4H), 8.03 (d, J=7.2 Hz). HR-MS:C₂₁H₂₂Cl₂N₅O₅ Calcd.: 494.0998, Found: 494.1004
[α]²⁰_{D}:-125.0° (c=0.50,MeOH)

### Example 13

### Methyl 3-(4-((2,6-dichlorophenyl)carbonylamino)-2-oxohydropyrimidinyl)-2-((4-benzylpiperidyl)carbonylamino) propionate (13)

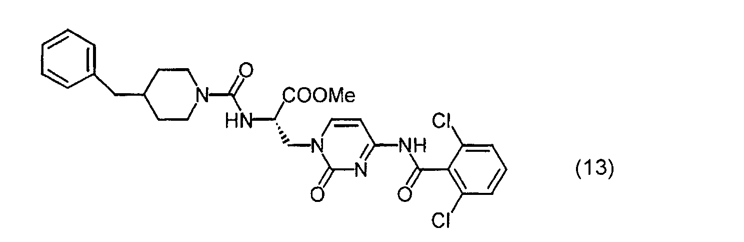

Under argon atmosphere, 72.0 mg of methyl 2-amino-3-(4-((2,6-dichlorophenyl)carbonylamino)-2-oxohydropyridiminyl) propionate was dissolved in 4 ml of acetonitrile and 4 ml of dichloromethane, and 29 mg of saturated sodium hydrogen carbonate and 30 mg of chloroformic acid p-nitrophenyl ester were added while cooling the mixture in ice, followed by stirring the resulting mixture at room temperature for 3 hours. To the reaction mixture, 30 µl of 4-benzylpiperidine and 49 µl of triethylamine were added and the resulting mixture was stirred at room temperature for 19 hours. After concentrating the reaction mixture, 10 ml of saturated aqueous sodium hydrogen carbonate solution was added and the resulting mixture was extracted with ethyl acetate. Organic phases were combined, washed with 1N hydrochloric acid and with saturated saline, dried over anhydrous sodium sulfate and concentrated. The residue was purified by column chromatography (dichloromethane/methanol = 20:1) to obtain 30.5 mg of methyl 3-(4-((2,6-dichlorophenyl)carbonylamino)-2-oxohydropyrimidinyl)-2-((4-benzylpiperidyl)carbonylamino) propionate (yield: 37%).
LR-MS(m/z):585(M⁺) IR(KBr):3341,2924,2850,1716,1658,1628,1562,1493,1433,1368,1304,1246,1055,
966,901,790,701 cm⁻¹
NMR(300MHz,CDCl₃, δ ppm):1.07-1.30(2H,m),1.02-1.10(2H,m),1.60-1.82(3H,m),2.50-2.60(2H,m),2.61-2.68(2H,m),3.78(3H,m),3.83-3.96(2H,m),4.31-4.40(2H,m),4.65-4.76(1H,m),6.05-6.16(1H,m),7.04-7.40(8H,m),7.51(1H,brs),7.78-7.82(1H,m),9.07(1H,brs)
[α]_{D}²⁰: -117.1° (C=0.08, MeOH)

### Example 14

### 3-(4-((2,6-dichlorophenyl)carbonylamino)-2-oxohydropyrimidinyl)-2-((4-benzylpiperidyl)carbonylamino)propanoic acid (14)

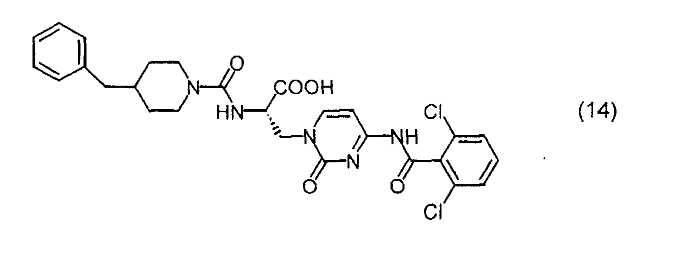

In 2 ml of methanol, 26 mg of methyl 3-(4-((2,6-dichlorophenyl)carbonylamino)-2-oxohydropyrimidinyl)-2-((4-benzylpiperidyl)carbonylamino) propionate was dissolved, and 0.2 ml of 1N aqueous sodium hydroxide solution was added, followed by stirring the mixture at room temperature for 3 hours. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/n-hexane to obtain 16.6 mg of 3-(4-((2,6-dichlorophenyl)carbonylamino)-2-oxohydropyrimidinyl)-2-((4-benzylpiperidyl)carbonylamino)propanoic acid (yield: 72%).
LR-MS(m/z):570(M⁺-H) IR(KBr):3407,2924,1717,1628,1494,1431,1367,1306,1246,1196,1132,964,901,791,7
47,701 cm⁻¹
NMR(300MHz,CD₃OD, δ ppm):1.07-1.30(2H,m),1.60-1.80(3H,m),2.51-2.79(4H,m),3.86-3.98(2H,m),4.32-4.50(2H,m),4.54-4.60(1H,m),7.11-7.40(10H,m)7.61(1H,brs),7.87(1H,brs)
HR-MS:C₂₇H₂₆Cl₂N₅O₅ Calcd.: 570.1311, Found: 570.1323
[α]_{D}²⁰: -113.5° (C=0.05, MeOH)

### Example 15

### Methyl 3-(4-((2,6-dichlorophenyl)carbonylamino)-2-oxohydropyrimidinyl)-2-((4-phenylpiperidyl)carbonylamino) propionate (15)

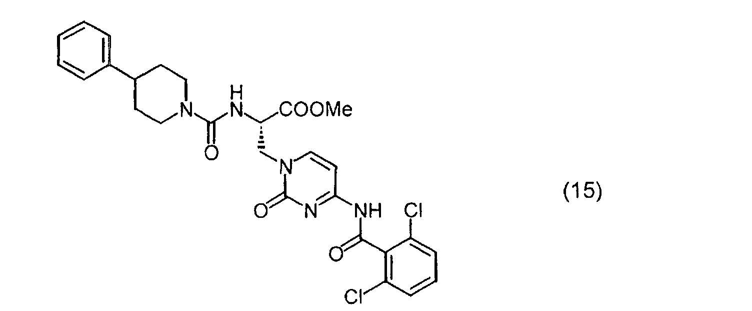

Under argon atmosphere, to a solution of 7 mg of triphosgene in 1 ml of dichloromethane, 24.4 mg of methyl 2-amino-3-(4-((2,6-dichlorophenyl)carbonylamino)-2-oxohydropyrimidinyl) propionate and 1 ml of a solution of 0.012 ml of diisopropylethylamine in dichloromethane were dropped, and the resulting mixture was stirred at room temperature for 10 minutes. To the reaction mixture, 1 ml of a solution of 10.8 mg of 4-phenylpiperidine and 0.013 ml of diisopropylethylamine in dichloromethane were added, and the resulting reaction mixture was stirred at room temperature for 45 minutes. After concentrating the reaction mixture, 6 ml of 10% aqueous citric acid solution was added and the resulting mixture was extracted with ethyl acetate. Organic phases were combined, washed with saturated saline, dried over anhydrous sodium sulfate and concentrated. The residue was purified by silica gel column chromatography (chloroform/methanol = 30:1) to obtain 22.1 mg of methyl 3-(4-((2,6-dichlorophenyl)carbonylamino)-2-oxohydropyrimidinyl)-2-((4-phenylpiperidyl)carbonylamino) propionate (yield: 61%).
LR-MS(m/z):571(M⁺)
IR(KBr):2937,2850,1741,1716,1657,1626,1561,1493,1432,1369,1305,1243,1131,10 11,985,902,797cm⁻¹
NMR(300MHz,CDCl₃, δ ppm):1.52-1.76(2H,m),1.80-1.89(2H,m),2.60-2.73(1 H,m),2.81-2.96(2H,m),3.77(3H,s),4.01-4.12(2H,m),4.36-4.42(2H,m),4.72-4.80(1H,m),6.21-6.25(1H,m),7.15-7.25(3H,m),7.26-7.39(5H,m),7.56(1H,brs),7.81-7.87(1 H.m),8.88(1H,brs)
[α]²⁰_{D}:+11.7° (c=0.02,MeOH)

### Example 16

### 3-(4-((2,6-dichlorophenyl)carbonylamino)-2-oxohydropyrimidinyl)-2-((4-phenylpiperidyl)carbonylamino)propanoic acid (16)

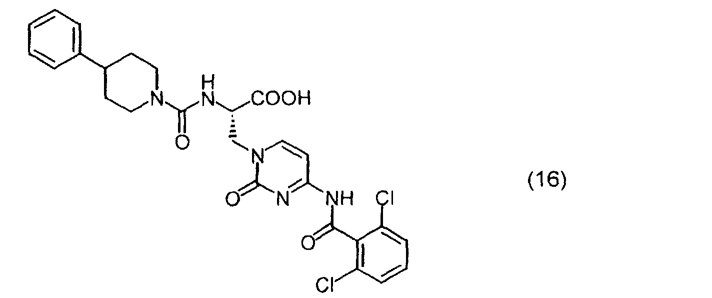

In 1.2 ml of methanol, 32.7 mg of methyl 3-(4-((2,6-dichlorophenyl)carbonylamino)-2-oxohydropyrimidinyl)-2-((4-phenylpiperidyl)carbonylamino) propionate was dissolved, and 0.3 ml of 1N aqueous sodium hydroxide solution was added, followed by stirring the resulting mixture overnight at room temperature. To the reaction solution, 4 ml of 0. IN hydrochloric acid was added and the resulting mixture was extracted with ethyl acetate. Organic phases were combined, washed with saturated saline, dried over anhydrous sodium sulfate and concentrated. The residue was reprecipitated from methanol/ether to obtain 18.5 mg of 3-(4-((2,6-dichlorophenyl)carbonylamino)-2-oxohydropyrimidinyl)-2-((4-phenylpiperidyl)carbonylamino)propanoic acid (yield: 58%).
LR-MS(m/z):558(M+H⁺)
IR(KBr):3402,2934,2852,1723,1651,1634,1562,1491,1431,1368,1305,1241,1192, 1131,984,901,792cm⁻¹
NMR(300MHz,CD₃OD, δ ppm): 1.54-1.70(2H,m),1.73-1.85(2H,m),2.68-2.70(1H,m),2.84-3.00(2H,m),4.05-4.17(3H,m),4.64-4.42(2H,m),4.72-4.82(3H,m),7.12-7.32(5H,m),7.41-7.60(4H,m),7.98-8.02(1H.m)
HR-MS:C₂₆H₂₆Cl₂N₅O₅
Calcd.: 558.1311, Found: 558.1271

### Example 17

### Methyl 3-(4-((2,6-dichlorophenyl)carbonylamino)-2-oxohydropyrimidinyl)-2-((4-indol-3-ylpiperidyl)carbonylamino) propionate (17)

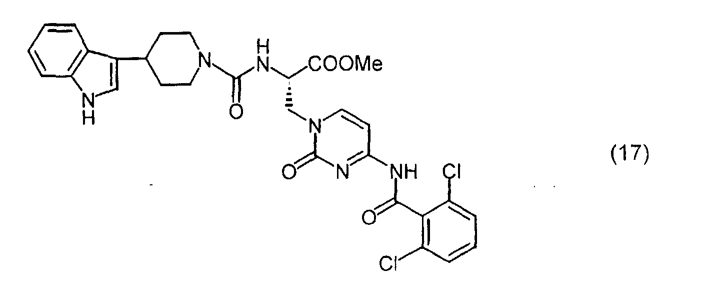

Under argon atmosphere, to a solution of 51.8 mg of methyl 2-amino-3-(4-((2,6-dichlorophenyl)carbonylamino)-2-oxohydropyrimidinyl) propionate in 2 ml of acetonitrile and 3 ml of dichloromethane, 18 mg of saturated sodium hydrogen carbonate and 29 mg of chloroformic acid p-nitrophenyl ester were added while cooling the mixture in ice, and the resulting mixture was stirred at room temperature for 7 hours. To the reaction mixture, 4 ml of a solution of 32 mg of 3-(4-piperidyl)indole and 0.46 ml of triethylamine in acetonitrile were added and the resulting mixture was stirred overnight at room temperature. After concentrating the reaction mixture, 10 ml of saturated aqueous sodium hydrogen carbonate solution was added and the resulting mixture was extracted with chloroform. Organic phases were combined, washed with saturated saline, dried over anhydrous sodium sulfate and concentrated. The residue was purified by silica gel column chromatography (dichloromethane:methanol = 30:1) to obtain 72 mg of methyl 3-(4-((2,6-dichlorophenyl)carbonylamino)-2-oxohydropyrimidinyl)-2-((4-indol-3-ylpiperidyl)carbonylamino) propionate (yield: 91%).
LR-MS(m/z):610(M⁺)
IR(KBr):3299,2932,2851,1716,1658,1562,1491,1432,1368,1305,1244,1130,1103,10 03,983,901,788,743cm⁻¹
NMR(300MHz,CDCl₃, δ ppm):1.33-1.60(2H,m),1.79-2.02(2H,m),2.77-2.99(3H,m),3.76(3H,s),3.81-4.04(2H,m),4.24-4.39(2H,m),4.79-4.90(1H,m),6.05-6,18(1H,m),6.93(1H,s),7.05-7.39(6H,m),7.42-7.63(2H,m),7.84-7.96(1H,m),8.60(1H,brs),9.82(1H,brs)
[α]_{D}²⁰: -160.2° (C=0.13, MeOH)

### Example 18

### 3-(4-((2,6-dichlorophenyl)carbonylamino)-2-oxohydropyrimidinyl)-2-((4-indol-3-ylpiperidyl)carbonylamino)propanoic acid (18)

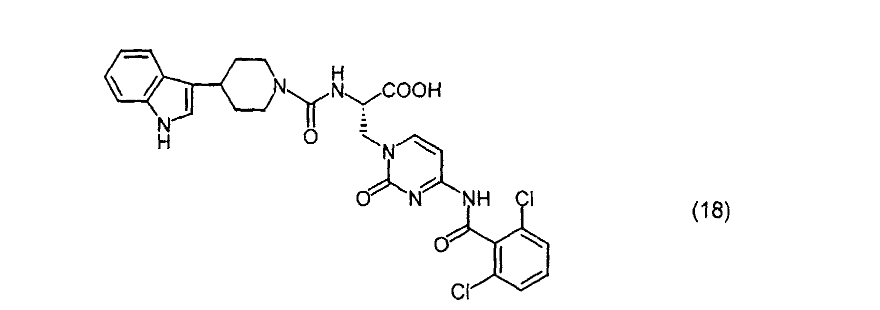

In 2 ml of methanol, 52 mg of methyl 3-(4-((2,6-dichlorophenyl)carbonylamino)-2-oxohydropyrimidinyl)-2-((4-indol-3-ylpiperidyl)carbonylamino) propionate was dissolved, and 0.5 ml of 1N aqueous sodium hydroxide solution was added, followed by stirring the resulting mixture at room temperature for 1 hour. To the reaction solution, 1N hydrochloric acid and water were added, and the resulting mixture was extracted with ethyl acetate. Organic phases were combined, washed with saturated saline, dried over anhydrous sodium sulfate and concentrated. The residue was reprecipitated from chloroform/ether to obtain 14.5 mg of 3-(4-((2,6-dichlorophenyl)carbonylamino)-2-oxohydropyrimidinyl)-2-((4-indol-3-ylpiperidyl)carbonylamino)propanoic acid (yield: 82%).
LR-MS(m/z):595(M⁺-H)
IR(KBr):3405,2934,2853,1717,1627,1563,1492,1431,1366,1305,1245,1195,1131,11 03,984,901,787,744scm⁻¹
NMR(300MHz,CD₃OD δ ppm): 1.57-1.74(2H,m),2.02-2.15(2H,m),2.81-3.10(3H,m),4.02-4.16(2H,m),4.32-4.53(2H,m),4.56-4.65( 1H,m), 6.99(1H,s),7.05-7.21(2H,m),7.32-7.50(4H,m),7.58-7.65(2H,m),7.82-7.90(1H,m)
HR-MS:C₂₈H₂₅Cl₂N₆O₅ Calcd.: 595.1263, Found: 595.1249
[α]_{D}²⁰: -56.5° (C=0.04, MeOH)

### Example 19

### Methyl 3-(4-((2,6-dichlorophenyl)carbonylamino)-2-oxohydropyrimidinyl)-2-((4-(cyclohexylcarbonyl)piperidyl)carbonylamino) propionate (19)

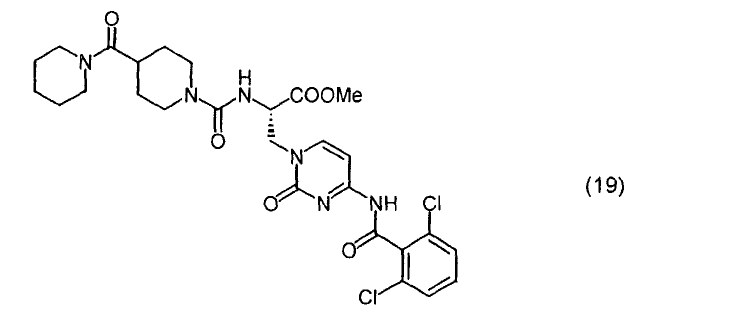

Under argon atmosphere, to a solution of 60 mg of methyl 2-amino-3-(4-((2,6-dichlorophenyl)carbonylamino)-2-oxohydropyrimidinyl) propionate in 4 ml of acetonitrile, 19.7 mg of saturated sodium hydrogen carbonate and 37.7 mg of chloroformic acid p-nitrophenyl ester were added while cooling the mixture in ice, followed by stirring the resulting mixture at room temperature for 3 hours. To the reaction mixture, 4 ml of a solution of 42.9 mg of 4-piperidylpiperidyl ketone and 0.11 ml of triethylamine in acetonitrile were added, and the resulting mixture was stirred overnight at room temperature. After concentrating the reaction mixture, 10 ml of saturated aqueous sodium hydrogen carbonate solution was added and the resulting mixture was extracted with chloroform. Organic phases were combined, washed with saturated saline, dried over anhydrous sodium sulfate and concentrated. The residue was purified by silica gel column chromatography (chloroform/methanol = 30:1) to obtain 71.0 mg of methyl 3-(4-((2,6-dichlorophenyl)carbonylamino)-2-oxohydropyrimidinyl)-2-((4-(cyclohexylcarbonyl)piperidyl)carbonylamino) propionate (yield: 75%).
LR-MS(m/z):606(M⁺)
IR(KBr):3005,2941,2856,1741,1718,1659,1626,1493,1433,1369,1305,1216cm⁻¹
NMR(300MHz,CDCl₃, δ ppm): 1.45-1.92(10H,m),2.60-2.75(1H,m),2.77-2.94(2H,m),3.38-3.51(4H,m),3.78(3H,s),3.90-4.06(2H,m),4.31(1H,dd,J=13.8,4.8),4.45(1H,dd,J=13.8,6.7),4.72-4.79(1H,m),6.13(1H,brs),7.29-7.55(4H,m),7.72-7.77(1H,m),8.80(1H,brs)
[α]²⁰_{D}:-120.5° (c=0.10, MeOH)

### Example 20

### 3-(4-((2,6-dichlorophenyl)carbonylamino)-2-oxohydropyrimidinyl)-2-((4-(cyclohexylcarbonyl)piperidyl)carbonylamino)propanoic acid (20)

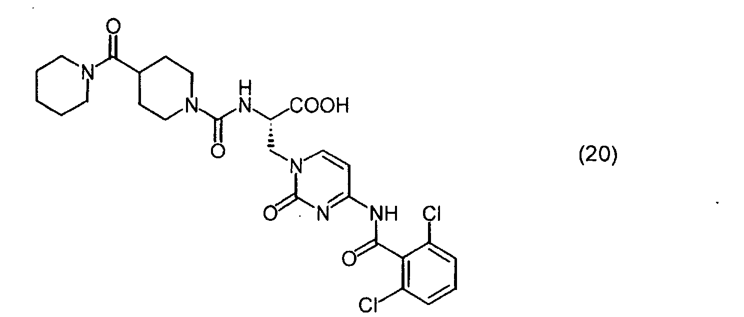

In 1.5 ml of methanol and 1.5 ml of tetrahydrofuran, 66.5 mg of methyl 3-(4-((2,6-dichlorophenyl)carbonylamino)-2-oxohydropyrimidinyl)-2-((4-(cyclohexylcarbonyl)piperidyl)carbonylamino) propionate was dissolved, and 1.5 ml of 1N aqueous sodium hydroxide solution was added, followed by stirring the resulting mixture overnight at room temperature. To the reaction solution, 2 ml of 1N hydrochloric acid and 6 ml of water were added and the resulting mixture was extracted with ethyl acetate. Organic phases were combined, washed with saturated saline, dried over anhydrous sodium sulfate and concentrated. The residue was reprecipitated from chloroform/ether to obtain 58.8 mg of 3-(4-((2,6-dichlorophenyl)carbonylamino)-2-oxohydropyrimidinyl)-2-((4-(cyclohexylcarbonyl)piperidyl)carbonylamino)propanoic acid (yield: 90%). LR-MS(m/z):591(M⁺-H)
IR(KBr):3418,2935,2857,1722,1631,1491,1433,1366,1304,1245,790cm⁻¹
NMR(300MHz,CD₃OD, δ ppm):1.48-1.74(10H,m),2.83-2.97(3H,m),3.50-3.59(4H,m),3.92-4.05(2H,m),4.10(1H,dd,J=13.2,9.0),4.64(1H,dd,J=13.2,4.4), 4.71(1H,dd,J=9.0,4.4),7.41-7.56(4H,m),7.95-7.78(1H,m)
HR-MS:C₂₆H₂₉Cl₂N₆O₆ Calcd.: Found:591.1525
[α]²⁰_{D}:-138.0° (c=0.10, MeOH)

### Example 21

### Methyl 3-(4-((2,6-dichlorophenyl)carbonylamino)-2-oxohydropyrimidinyl)-2-((4-(phenylcarbonylamino)piperidyl)carbonylamino) propionate (21)

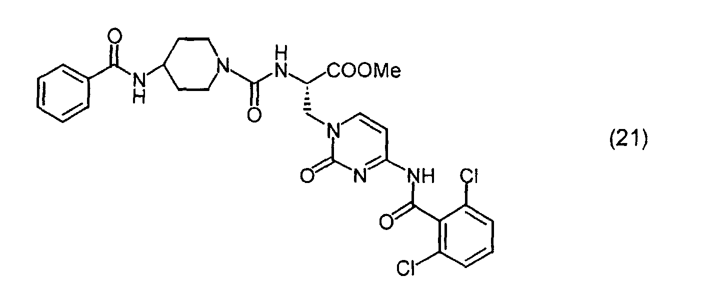

Under argon atmosphere, to a solution of 53.5 mg of methyl 2-amino-3-(4-((2,6-dichlorophenyl)carbonylamino)-2-oxohydropyrimidinyl) propionate in 2 ml of acetonitrile and 3 ml of dichloromethane, 18 mg of saturated sodium hydrogen carbonate and 30 mg of chloroformic acid p-nitrophenyl ester were added while cooling the mixture in ice, and the resulting mixture was stirred at room temperature for 4.5 hours. To the reaction mixture, 2 ml of a solution of 35 mg of phenyl-N-(4-piperidyl)carboxamide and 0.35 ml of triethylamine in acetonitrile were added, and the resulting mixture was stirred overnight at room temperature. After concentrating the reaction mixture, 10 ml of saturated aqueous sodium hydrogen carbonate solution was added and the resulting mixture was extracted with chloroform. Organic phases were combined, washed with 1N hydrochloric acid and with saturated saline, dried over anhydrous sodium sulfate and concentrated. The residue was purified by silica gel column chromatography (dichloromethane:methanol = 30:1) to obtain 68 mg of methyl 3-(4-((2,6-dichlorophenyl)carbonylamino)-2-oxohydropyrimidinyl)-2-((4-(phenylcarbonylamino)piperidyl)carbonylamino) propionate (yield: 79%).
LR-MS(m/z):614(M⁺)
IR(KBr):3369,2952,1739,1635,1562,1531,1492,1432,1367,1330,1302,1243,1196,11 67,1133,1075,972,790cm⁻¹
NMR(300MHz,CDCl₃, δ ppm):1.32-1.56(2H,m),1.93-2.05(2H,m),2.85-3.02(2H,m),3.80(3H,s),3.93-4.04(2H,m),4.05-4.22(1H,m),4.28-4.51(2H,m),4.64-4.73(1H,m),6.49(1H,brs),7.27-7.48(4H,m),7.54-7.61(1H,m),7.76-7.85(2H,m)
[α]_{D}²⁰: -137.1° (C=0.07, MeOH)

### Example 22

### 3-(4-((2,6-dichlorophenyl)carbonylamino)-2-oxohydropyrimidinyl)-2-((4-(phenylcarbonylamino)piperidyl)carbonylamino)propanoic acid (22)

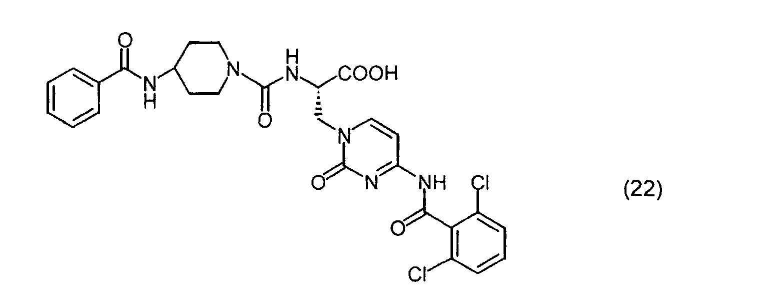

In 2 ml of methanol, 62 mg of methyl 3-(4-((2,6-dichlorophenyl)carbonylamino)-2-oxohydropyrimidinyl)-2-((4-(phenylcarbonylamino)piperidyl)carbonylamino) propionate was dissolved, and 0.5 ml of 1N aqueous sodium hydroxide solution was added, and the mixture was stirred at room temperature for 2 hours. To the reaction solution, 1N hydrochloric acid and water were added and the resulting mixture was extracted with ethyl acetate. Organic phases were combined, washed with saturated saline, dried over anhydrous sodium sulfate and concentrated. The residue was reprecipitated from chloroform/ether to obtain 53.3 mg of 3-(4-((2,6-dichlorophenyl)carbonylamino)-2-oxohydropyrimidinyl)-2-((4-(phenylcarbonylamino)piperidyl)carbonylamino)propanoic acid (yield: 89%). LR-MS(m/z):599(M⁺-H)
IR(KBr):3374,2948,1717,1636,1563,1532,1492,1431,1367,1331,1304,1244,1196, 1167,1134,1075,972,901,791cm⁻¹
NMR(300MHz,CD₃OD, δ ppm):1.30-1.54(2H,m),1.91-2.05(2H,m),2.80-3.02(2H,m),3.86-4.08(2H,m),4.10-4.22(1 H,m),4.28-4.52(2H,m),4.64-4.76(1 H,m),7.27-7.48(6H,m),7.54-7.61(1H,m),7.76-7.90(2H,m)
HR-MS:C₂₇H₂₅Cl₂N₆O₆ Calcd.: 599.1213, Found: 599.1214
[α]_{D}²⁰: -108.0° (C=0.04, MeOH)

### Example 23

### Methyl 3-(4-((2,6-dichlorophenyl)carbonylamino)-2-oxohydropyrimidinyl)-2-((4-((2,6-dichlorophenyl)carbonyl)piperazinyl)carbonylamino) propionate (23)

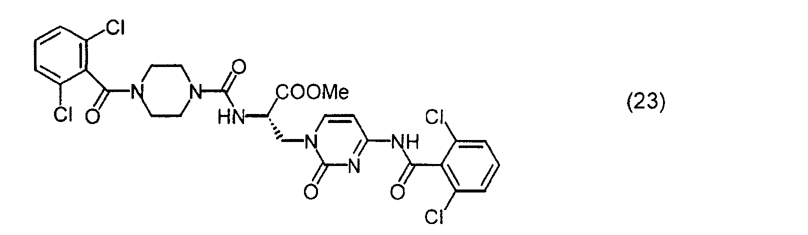

Under argon atmosphere, 48 mg of methyl 2-amino-3-(4-((2,6-dichlorophenyl)carbonylamino)-2-oxohydropyrimidinyl) propionate was dissolved in 2 ml of acetonitrile and 3 ml of dichloromethane, and 16 mg of saturated sodium hydrogen carbonate and 26 mg of chloroformic acid p-nitrophenyl ester were added while cooling the mixture in ice, followed by stirring the resulting mixture at room temperature for 4 hours. To the reaction mixture, 30 mg of 2,6-dichlorophenylcarbonylpiperazine and 42 µl of triethylamine were added, and the resulting mixture was stirred at room temperature for 38 hours. After concentrating the reaction mixture, saturated aqueous sodium hydrogen carbonate solution was added and the resulting mixture was extracted with ethyl acetate. Organic phases were combined, washed with 1N hydrochloric acid and with saturated saline, dried over anhydrous sodium sulfate and concentrated. The residue was purified by column chromatography (dichloromethane/methanol = 30:1) to obtain 40.9 mg of methyl 3-(4-((2,6-dichlorophenyl)carbonylamino)-2-oxohydropyrimidinyl)-2-((4-((2,6-dichlorophenyl)carbonyl)piperazinyl)carbonylamino) propionate (yield: 51%). LR-MS(m/z):668(M⁺)
IR(KBr):3404,3081,2953,1716,1649,1562,1492,1431,1367,1303,1256,1132,1005, 795cm⁻¹
NMR(300MHz,CDCl₃, δ ppm):3.18-3.23(2H,m),341-3.53(4H,m),3.78(3H,s),3.76-3.82(2H,m),4.21-4.44(2H,m),4.71-4.80(1H,m),6.70-6.79(1H,m),7.22-7.41(6H,m),7.57(1H,brs),7.80-7.84(1H,m),9.16(1H,brs)
[α]_{D}²⁰: -118.6° (C=0.11, MeOH)

### Example 24

### 3-(4-((2,6-dichlorophenyl)carbonylamino)-2-oxohydropyrimidinyl)-2-((4-((2,6-dichlorophenyl)carbonyl)piperazinyl)carbonylamino) propanoic acid (24)

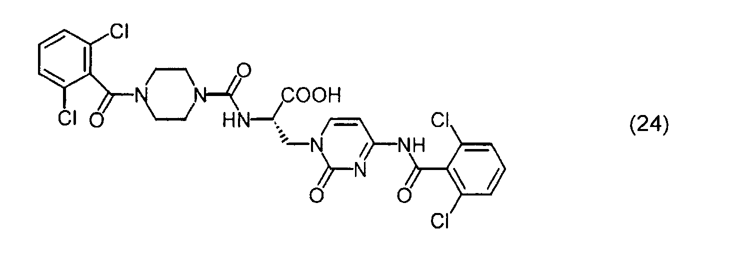

In 2 ml of methanol, 35 mg of methyl 3-(4-((2,6-dichlorophenyl)carbonylamino)-2-oxohydropyrimidinyl)-2-((4-benzylpiperidyl)carbonylamino) propionate was dissolved, and 0.25 ml of 1N aqueous sodium hydroxide solution was added, followed by stirring the resulting mixture at room temperature for 2 hours. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/n-hexane to obtain 26 mg of 3-(4-((2,6-dichlorophenyl)carbonylamino)-2-oxohydropyrimidinyl)-2-((4-((2,6-dichlorophenyl)carbonyl)piperazinyl)carbonylamino) propanoic acid (yield: 79%).
LR-MS(m/z):653(M⁺-H)
IR(KBr):3389,3081,2925,1718,1636,1564,1492,1430,1366,1257,1196,1132,1003,90 1,795cm⁻¹
NMR(300MHz,CD₃OD, δ ppm):3.22-3.28(2H,m),3.42-3.58(4H,m),3.80-3.87(2H,m),4.25-4.37(1H,m),4.42-4.51(1H,m),4.60-4.64(1H,m),7.28-7.40(8H,m),7.60(1H,brs),7.82(1H,brs)
HR-MS:C₂₆H₂₁C₁₄N₆O₆ Calcd.: 653.0277, Found: 653.0273
[α]_{D}²⁰: -99.0° (C=0.05, MeOH)

### Example 25

### Methyl 3-(4-((2,6-dichlorophenyl)carbonylamino)-2-oxohydropyrimidinyl)-2-((2-(phenylcarbonyl)pyrrolidinyl)carbonylamino) propionate (25)

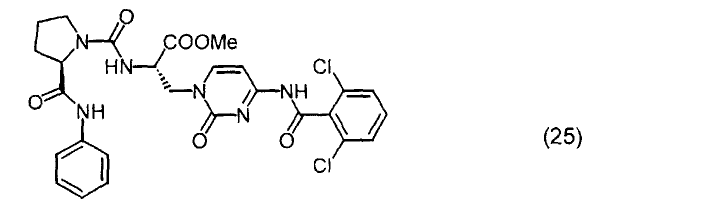

Under argon atmosphere, 59 mg of methyl 2-amino-3-(4-((2,6-dichlorophenyl)carbonylamino)-2-oxohydropyrimidinyl) propionate was dissolved in 2 ml of acetonitrile and 3 ml of dichloromethane, and 21 mg of saturated sodium hydrogen carbonate and 34 mg of chloroformic acid p-nitrophenyl ester were added while cooling the mixture in ice, followed by stirring the resulting mixture at room temperature for 4 hours. To the reaction mixture, 34 mg of N-phenylpyrrolidin-2-ylcarboxamide and 53 µl of triethylamine were added, and the resulting mixture was stirred at room temperature for 12 hours. After concentrating the reaction mixture, saturated aqueous sodium hydrogen carbonate solution was added and the resulting mixture was extracted with ethyl acetate. Organic phases were combined, washed with 1N hydrochloric acid and with saturated saline, dried over anhydrous sodium sulfate and concentrated. The residue was purified by column chromatography (dichloromethane/methanol = 20:1) to obtain 63.8 mg of methyl 3-(4-((2,6-dichlorophenyl)carbonylamino)-2-oxohydropyrimidinyl)-2-((2-(phenylcarbonyl)pyrrolidinyl)carbonylamino) propionate (yield: 62%).
LR-MS(m/z):600(M⁺)
IR(KBr):3395,2958,1652,1559,1495,1433,1367,1307,1248,1194,902,792,759cm⁻¹ NMR(300MHz,CDCl₃, δ ppm):1.78-2.18(3H,m),2.42-2.53(1H,m),3.23-3.36(1 H,m),3.41-3.50(1H,m),3.78(3H,s),4.25-4.43(2H,m),4.57-4.76(2H,m),6.42-6.50(1H,m),7.01-7.06(1H,m),7.22-7.40(5H,m),7.41-7.58(2H,m),7.78-7.81(1H,m),8.99(1H,brs),9.42(1H,brs)
[α]_{D}²⁰: -91.4° (C=0.08, MeOH)

### Example 26

### 3-(4-((2,6-dichlorophenyl)carbonylamino)-2-oxohydropyrimidinyl)-2-((2-(phenylcarbonyl)pyrrolidinyl)carbonylamino)propanoic acid (26)

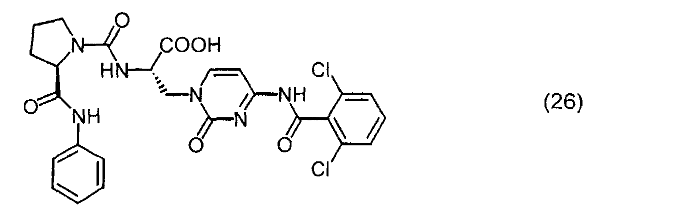

In 2 ml of methanol, 59 mg of methyl 3-(4-((2,6-dichlorophenyl)carbonylamino)-2-oxohydropyrimidinyl)-2-((2-(phenylcarbonyl)pyrrolidinyl)carbonylamino) propionate was dissolved, and 0.5 ml of 1N aqueous sodium hydroxide solution was added, followed by stirring the resulting mixture at room temperature for 3.5 hours. To the reaction solution, 1N hydrochloric acid was added and the mixture was extracted with ethyl acetate. Organic phases were combined, washed with saturated saline, dried over anhydrous sodium sulfate and concentrated. The residue was reprecipitated from chloroform/n-hexane to obtain 49.6 mg of 3-(4-((2,6-dichlorophenyl)carbonylamino)-2-oxohydropyrimidinyl)-2-((2-(phenylcarbonyl)pyrrolidinyl)carbonylamino)propanoic acid (yield: 84%). LR-MS(m/z):585(M⁺-H)
IR(KBr):3395,2958,1652,1559,1495,1433,1367,1307,1248,1194,902,792,759cm⁻¹
NMR(300MHz,CD₃OD, δ ppm):1.60-2.42(4H,m),3.21-3.35(1H,m),3.40-3.58(1H,m),4.21-4.38(1H,m),4.40-4.55(2H,m),4.62-4.74(1H,m),7.01-7.10(1H,m),7.22-7.65(8H,m),7.87(1H,brs),9.20(1H,brs)
HR-MS:C₂₆H₂₃Cl₂N₆O₆ Calcd.: 585.1056, Found: 585.1034
[α]_{D}²⁰: -68.2° (C=0.04, MeOH)

### Example 27

### Methyl 3-(4-((2,6-dichlorophenyl)carbonylamino)-2-oxohydrpyrimidinyl)-2-((1-(methylsulfonyl)spiro[indoline-3,4'-piperidin]-10-yl)carbonylamino) propionate (27)

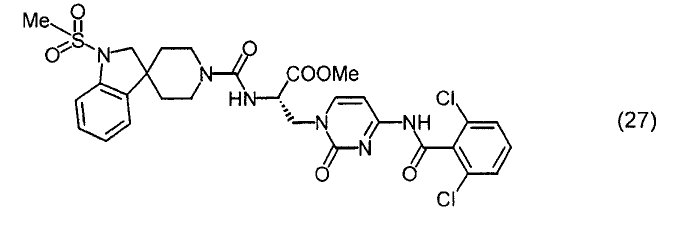

Under argon atmosphere, 60 mg of methyl 2-amino-3-(4-((2,6-dichlorophenyl)carbonylamino)-2-oxohydropyrimidinyl) propionate was dissolved in 2 ml of acetonitrile and 4 ml of dichloromethane, and 22 mg of saturated sodium hydrogen carbonate and 36 mg of chloroformic acid p-nitrophenyl ester were added to the mixture while cooling the mixture in ice, followed by stirring the resulting mixture at room temperature for 4 hours. To the reaction mixture, 51 mg of 1-(methylsulfonyl)spiro[indoline-3,4'-piperidine] and 56 µl of triethylamine were added, and the resulting mixture was stirred at room temperature for 12 hours. After concentrating the reaction mixture, saturated aqueous sodium hydrogen carbonate solution was added and the resulting mixture was extracted with ethyl acetate. Organic phases were combined, washed with saturated saline, dried over anhydrous sodium sulfate and concentrated. The residue was purified by column chromatography (dichloromethane/methanol = 30:1) to obtain 65 mg of methyl 3-(4-((2,6-dichlorophenyl)carbonylamino)-2-oxohydrpyrimidinyl)-2-((1-(methylsulfonyl)spiro[indoline-3,4'-piperidin]-10-yl)carbonylamino) propionate (yield: 60%).
LR-MS(m/z):676(M⁺)
IR(KBr):3410,2930,1738,1716,1657,1562,1492,1433,1345,1304,1247,1159,1049,
984, 775cm⁻¹
NMR(300MHz,CDCl₃,ppm):1.64-1.87(4H,m),2.82-2.95(2H,m),2.92(3H,s), 3.78(3H,s),3.84(2H,s),3.90-4.02(2H,m),4.28-4.46(2H,m),4.75-4.82(1H,m),6.47-6.56(1H,m),7.02-7.43(7H,m),7.57(1H,brs),7.83-7.92(1H,m),9.15(1H,brs)
[α]_{D}²⁰: -120.7° (C=0.14, MeOH)

### Example 28

3-(4-((2,6-dichlorophenyl)carbonylamino)-2-oxohydropyrimidinyl)-2-((1-(methylsulfonyl)spiro[indoline-3,4'-piperidin]-10-yl)carbonylamino)propanoic acid (28)

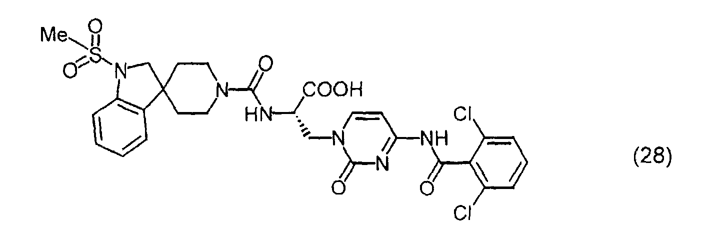

In 3 ml of methanol, 58 mg of methyl 3-(4-((2,6-dichlorophenyl)carbonylamino)-2-oxohydropyrimidinyl)-2-((1-(methylsulfonyl)spiro[indoline-3,4'-piperidin]-10-yl)carbonylamino) propionate was dissolved, and 0.5 ml of 1N aqueous sodium hydroxide solution was added, followed by stirring the resulting mixture at room temperature for 3.5 hours. To the reaction mixture, 1N hydrochloric acid was added, and the resulting mixture was extracted with ethyl acetate. Organic phases were combined, washed with saturated saline, dried over anhydrous sodium sulfate and concentrated. The residue was reprecipitated from chloroform/n-hexane to obtain 48.4 mg of 3-(4-((2,6-dichlorophenyl)carbonylamino)-2-oxohydropyrimidinyl)-2-((1-(methylsulfonyl)spiro[indoline-3,4'-piperidin]-10-yl)carbonylamino)propanoic acid (yield: 85%).
LR-MS(m/z):661(M⁺-H) IR(KBr):3413,2931,1716,1629,1564,1493,1431,1343,1248,1158,1050,961,901,777c m⁻¹
NMR(300MHz,CD₃OD, δ ppm):1.63-1.98(4H,m),2.93-3.10(2H,m),2.95(3H,s),3.84(2H,s),3.94-4.18(2H,m),4.32-4.68(3H,m),7.01-7.50(9H,m),7.65(1H,brs),8.16(1H,brs)
HR-MS:C₂₈H₂₇Cl₂N₆O₇S Calcd.:661.1039, Found: 661.1069
[α]_{D}²⁰: -136.4° (C=0.04, MeOH)

### Example 29

### Methyl 3-(4-((2,6-dichlorophenyl)carbonylamino)-2-oxohydropyrimidinyl)-2-((1-(phenylsulfonyl)spiro[indoline-3,4'-piperidin]-10-yl)carbonylamino) propionate (29)

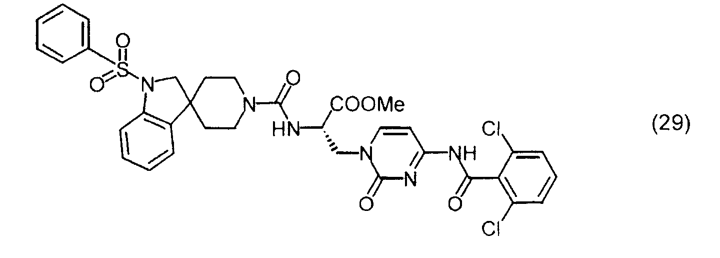

Under argon atmosphere, 51.5 mg of methyl 2-amino-3-(4-((2,6-dichlorophenyl)carbonylamino)-2-oxohydropyrimidinyl) propionate was dissolved in 2 ml of acetonitrile and 3 ml of dichloromethane, and 18 mg of saturated sodium hydrogen carbonate and 28 mg of chloroformic acid p-nitrophenyl ester were added to the mixture, followed by stirring the resulting mixture at room temperature for 6.5 hours. To the reaction mixture, 53 mg of 1-(phenylsulfonyl)spiro[indoline-3,4'-piperidine] and 46 µl of triethylamine were added, and the resulting mixture was stirred at room temperature for 12 hours. After concentrating the reaction mixture, saturated aqueous sodium hydrogen carbonate solution was added and the resulting mixture was extracted with ethyl acetate. Organic phases were combined, washed with 1N hydrochloric acid and with saturated saline, dried over anhydrous sodium sulfate and concentrated. The residue was purified by column chromatography (dichloromethane/methanol = 50:1) to obtain 83.9 mg of methyl 3-(4-((2,6-dichlorophenyl)carbonylamino)-2-oxohydropyrimidinyl)-2-((1-(phenylsulfonyl)spiro[indoline-3,4'-piperidin]-10-yl)carbonylamino) propionate (yield: 87%).
LR-MS(m/z):738(M⁺)
IR(KBr):3291,3069,2949,1715,1657,1561,1491,1433,1363,1308,1244,1169,1048, 986,926,790,757,739cm⁻¹
NMR(300MHz,CDCl₃, δ ppm):1.15-1.24(2H,m),1.52-1.67(2H,m),2.71-2.84(2H,m),3.74(3H,s),3.79(2H,m),3.76-3.88(2H,m),4.26-4.42(2H,m),4.71-4.80(1H,m),6.38-6.41(1H,m),6.98-7.06(2H,m),7.20-7.60(8H,m),7.65(1H,brs),7.77-7.83(1 H.m),9.04(1H,brs)
[α]_{D}²⁰: -130.0° (C=0.11, MeOH)

### Example 30

### 3-(4-((2,6-dichlorophenyl)carbonylamino)-2-oxohydropyrimidinyl)-2-((1-(phenylsulfonyl)spiro[indoline-3,4'-piperidin]-10-yl)carbonylamino)propanoic acid

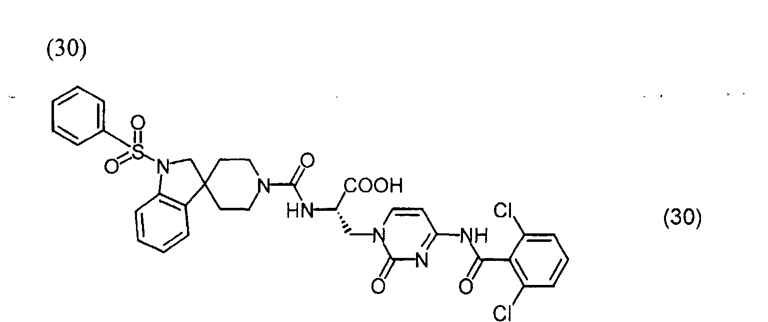

In 1 ml of methanol, 76 mg of methyl 3-(4-((2,6-dichlorophenyl)carbonylamino)-2-oxohydropyrimidinyl)-2-((1-(methylsulfonyl)spiro[indoline-3,4'-piperidin]-10-yl)carbonylamino) propionate was dissolved, and 0.5 ml of 1N sodium hydroxide solution was added, followed by stirring the resulting mixture at room temperature for 30 hours. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/ether to obtain 62.5 mg of 3-(4-((2,6-dichlorophenyl)carbonylamino)-2-oxohydropyrimidinyl)-2-((1-(phenylsulfonyl)spiro[indoline-3,4'-piperidin]-10-yl)carbonylamino)propanoic acid (yield: 86%).
LR-MS(m/z):723(M⁺-H)
IR(KBr):3409,2937,1717,1629,1563,1492,1432,1361,1307,1253,1167,1092,984,927, 791,758cm⁻¹
NMR(300MHz,CD₃OD, δ ppm):1.17-1.31(2H,m),1.52-1.70(2H,m),2.71-2.90(2H,m),3.70-3.90(4H,m),4.24-4.52(2H,m),4.63-4.82(1H,m),6.65-7.08(2H,m),7.20-7.70(11H,m),7.75-7.88(2H,m),7.99(1H,brs)
[α]_{D}²⁰: -54.2° (C=0.05, MeOH)

### Example 31

### Methyl 3-(4-((2,6-dichlorophenyl)carbonylamino)-2-oxohydropyrimidinyl)-2-((4-(2-oxo-3-azaindolinyl)piperidyl)carbonylamino) propionate (31)

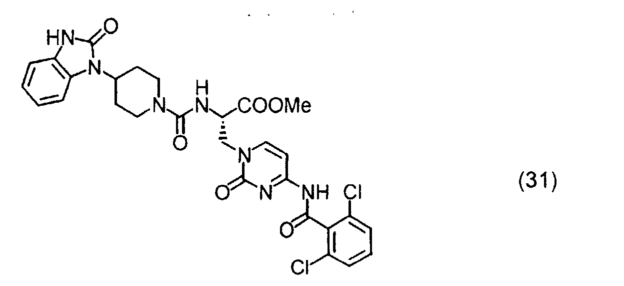

Under argon atmosphere, 156 mg of methyl 2-amino-3-(4-((2,6-dichlorophenyl)carbonylamino)-2-oxohydropyrimidinyl) propionate was dissolved in 10 ml of acetonitrile and 10 ml of dichloromethane, and 52.4 mg of sodium hydrogen carbonate and 92.2 mg of chloroformic p-nitrophenyl ester were added thereto while cooling the mixture in ice, followed by stirring the resulting mixture at room temperature for 3 hours. To the reaction mixture, 12 ml of a solution of 176 mg of 4-(2-keto-1-benzimidazolinyl)piperidine and 0.35 ml of triethylamine in dichloromethane were added, and the resulting mixture was stirred overnight at room temperature. After concentrating the reaction mixture, 10 ml of saturated aqueous sodium hydrogen carbonate solution was added and the resulting mixture was extracted with chloroform. Organic phases were combined, washed with 1N hydrochloric acid and with saturated saline, dried over anhydrous sodium sulfate and concentrated. The residue was purified by silica gel column chromatography (chloroform/methanol to obtain 128 mg of methyl 3-(4-((2,6-dichlorophenyl)carbonylamino)-2-oxohydropyrimidinyl)-2-((4-(2-oxo-3-azaindolinyl)piperidyl)carbonylamino) propionate (yield: 50%).
LR-MS(m/z):627(M⁺)
IR(KBr):3057,2953,2847,1686,1628,1487,1433,1369,1308,1266,1246,1196,1133, 1013,897,797,736cm⁻¹
NMR(300MHz,CD₃OD, δ ppm):1.74-1.84(2H,m),2.31-2.48(2H,m),2.93-3.08(2H,m),3.81(3H,s),4.11-4.23(3H,m),4.42-4.57(1H,m),4.61-4.74(2H,m),7.03-7.12(3H,m),7.25-7.30(1H,m),7.40-7.51(3H.m),7.55-7.62(1H,m),8.03-8.05(1H,m)
[α]²⁰_{D}:-144.6° (c=0.03,MeOH)

### Example 32

### 3-(4-((2,6-dichlorophenyl)carbonylamino)-2-oxohydropyrimidinyl)-2-((4-(2-oxo-3-azaindolinyl)piperidyl)carbonylamino)propanoic acid (32)

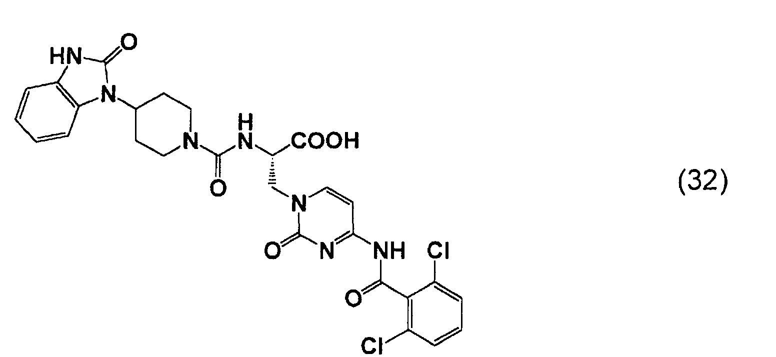

In 3.0 ml of methanol, 112.6 mg of methyl 3-(4-((2,6-dichlorophenyl)carbonylamino)-2-oxohydropyrimidinyl)-2-((4-(2-oxo-3-azaindolinyl)piperidyl)carbonylamino) propionate was dissolved, and 1 ml of 1N aqueous sodium hydroxide solution was added, followed by stirring the resulting mixture at room temperature for 5 hours. To the reaction solution, 1.2 ml of 1N hydrochloric acid and 8 ml of water were added, and the resulting mixture was extracted with ethyl acetate. Organic phases were combined, washed with saturated saline, dried over anhydrous sodium sulfate and concentrated. The residue was reprecipitated from methanol/ether to obtain 103.4 mg of 3-(4-((2,6-dichlorophenyl)carbonylamino)-2-oxohydropyrimidinyl)-2-((4-(2-oxo-3-azaindolinyl)piperidyl)carbonylamino)propanoic acid (yield: 94%).
LR-MS(m/z):612(M⁺-H)
IR(KBr):3398,2931,2862,1691,1659,1487,1430,1367,1303,1245,1193,1011,899,792, 756cm⁻¹
NMR(300MHz,CD₃OD, δ ppm):1.72-1.84(2H,m),2.30-2.48(2H,m),2.93-3.07(2H,m),4.11-4.22(3H,m),4.42-4.57(1H,m),4.65-4.76(2H,m),7.00-7.12(3H,m),7.24-7.30(1H,m),7.40-7.51(3H,m),7.55-7.62(1H,m),8.03-8.05(1H,m) HR-MS:C₂₇H₂₄Cl₂N₇O₆ Calcd.: 612.1165, Found: 612.1146
[α]²⁰_{D}:-135.1° (c=0.07,MeOH)

### Example 33

### Methyl 3-(4-((2,6-dichlorophenyl)carbonylamino)-2-oxohydropyrimidinyl)-2-((4-(3-methyl-2-oxo-3-azaindolinyl)piperidyl)carbonylamino) propionate (33)

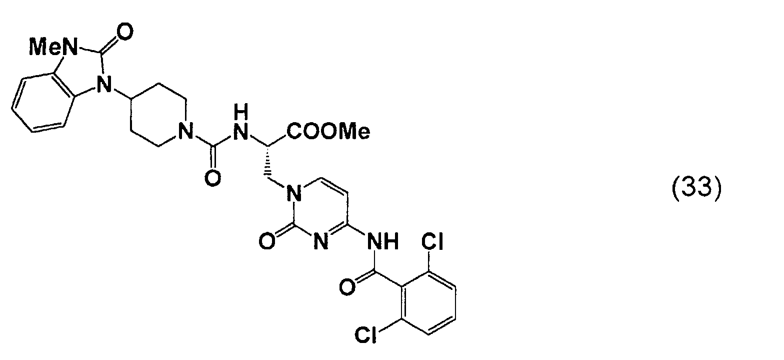

Under argon atmosphere, 55 mg of methyl 2-amino-3-(4-((2,6-dichlorophenyl)carbonylamino)-2-oxohydropyrimidinyl) propionate was dissolved in 2 ml of acetonitrile and 3 ml of dichloromethane, and 18.5 mg of saturated sodium hydrogen carbonate and 30.2 mg of chloroformic acid p-nitrophenyl ester were added while cooling the mixture in ice, followed by stirring the resulting mixture at room temperature for 4 hours. To the reaction mixture, 39.3 mg of 1-methyl-3-(4-piperidyl)-3-hydrobenzimidazol-2-one and 49 µl of triethylamine were added, and the resulting mixture was stirred at room temperature for 14 hours. After concentrating the reaction mixture, saturated aqueous sodium hydrogen carbonate solution was added and the resulting mixture was extracted with ethyl acetate. Organic phases were combined, washed with 1N hydrochloric acid and with saturated saline, dried over anhydrous sodium sulfate and concentrated. The residue was purified by column chromatography (dichloromethane/methanol = 30:1) to obtain 65 mg of methyl 3-(4-((2,6-dichlorophenyl)carbonylamino)-2-oxohydropyrimidinyl)-2-((4-(3 -methyl-2-oxo-3-azaindolinyl)piperidyl)carbonylamino) propionate (yield: 68%).
LR-MS(m/z):641(M⁺)
IR(KBr):3423,3071,2952,1660,1562,1495,1434,1368,1305,1247,1197,1132,1053, 986,903,791,753,743,700cm⁻¹
NMR(300MHz,CDCl₃, δ ppm): 1.72-1.84(2H,m),2.26-2.48(2H,m),2.80-2.97(2H,m),3.31(3H,s),3.76(3H,m),4.06-4.20(2H,m),4.31-4.49(3H,m),4.80-4.88(1H,m),6.40-6.55(1H,m),6.93-7.20(5H,m),7.22-7.40(4H,m),7.58(1H,brs),7.81-7.88(1H.m),9.56(1H,brs)
[α]_{D}²⁰: -139.4° (C=0.11, MeOH)

### Example 34

### 3-(4-((2,6-dichlorophenyl)carbonylamino)-2-oxohydropyrimidinyl)-2-((4-(3-methyl-2-oxo-3-azaindolinyl)piperidyl)carbonylamino)propanoic acid (34)

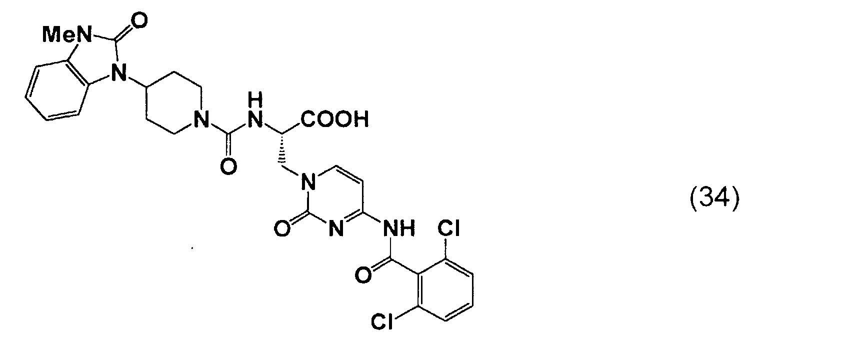

In 2 ml of methanol, 59 mg of methyl 3-(4-((2,6-dichlorophenyl)carbonylamino)-2-oxohydropyrimidinyl)-2-((4-(3-methyl-2-oxo-3-azaindolinyl)piperidyl)carbonylamino) propionate was dissolved, and 0.5 ml of 1N aqueous sodium hydroxide solution was added, followed by stirring the resulting mixture at room temperature for 4 hours. To the reaction mixture, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/ether to obtain 47.3 mg of 3-(4-((2,6-dichlorophenyl)carbonylamino)-2-oxohydropyrimidinyl)-2-((4-(3-methyl-2-oxo-3-azaindolinyl)piperidyl)carbonylamino)propanoic acid (yield: 84%).
LR-MS(m/z):627(M⁺)
IR(KBr):3406,2937,1655,1563,1494,1434,1397,1367,1306,1247,1196,1163,1 132,10 52,986,900,791,753cm⁻¹
NMR(300MHz,CD₃OD, δ ppm):1.75-1.96(2H,m),2.22-2.48(2H,m),2.83-3.05(2H,m),3.40(3H,s),4.03-4.22(2H,m),4.32-4.58(3H,m),4.70-4.84(1H,m),6.94-7.40(9H,m),7.65(1H,brs),8.04(1H,brs)
HR-MS:C₂₈H₂₈Cl₂N₇O₆ Calcd.: 628.1478, Found: 628.1437
[α]_{D}²⁰: -118.3° (C=0.05, MeOH)

### Example 35

### 2-((2,4,8-triaza-1-oxo-4-phenylspiro[4.5]dec-8-yl)carbonylamino)-3-(4-((2,6-dichlorophenylcarbonylamino)-2-oxohydropyrimidinyl)propanoic acid (35)

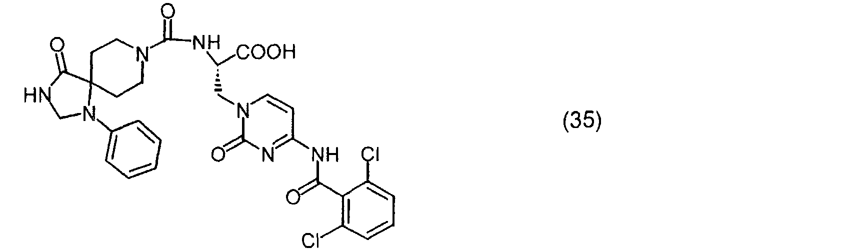

Under argon atmosphere, methyl 2-amino-3-(4-((2,6-dichlorophenyl)carbonylamino)-2-oxohydropyrimidinyl) propionate (40mg, 0.104mmol) was dissolved in 0.5 ml of THF, and 2,4,8-triaza-1-oxo-4-phenylspiro[4.5]decane-8-carbonyl chloride (61.0mg, 0.208mmol) and triethylamine (29 µl, 0.208mmol) were added thereto, followed by stirring the resulting mixture at room temperature for 12 hours. To the reaction mixture, IN hydrochloric acid was added and the resulting mixture was extracted with ethyl acetate. Organic phases were combined, washed with saturated saline, dried over anhydrous sodium sulfate and concentrated. The residue was dissolved in 4 ml of methanol, and 0.5 ml of 1N aqueous sodium hydroxide solution was added thereto, followed by stirring the resulting mixture at room temperature for 12 hours. To the reaction mixture, 1N hydrochloric acid was added and the resulting mixture was extracted with ethyl acetate. Organic phases were combined, washed with saturated saline, dried over anhydrous sodium sulfate and concentrated. The residue was purified by column chromatography (ethyl acetate/methanol = 15/1) to obtain 2-((2,4,8-triaza-1-oxo-4-phenylspiro[4.5]dec-8-yl)carbonylamino)-3-(4-((2,6-dichlorophenylcarbonylamino)-2-oxohydropyrimidinyl)propanoic acid (30.4 mg) (yield: 54%).
LR-MS(m/z):628(M⁺+H)
IR(KBr):3423, 2923, 1709,1627, 1494, 1432, 1367, 1305, 1252cm⁻¹ NMR(300MHz,CD3OD δ ppm):1.63-1.72 (m, 2H), 2.45-2.64 (m, 2H), 3.50-3.70 (m, 2H), 3.87-3.95 (m, 2H), 4.10-4.20 (m, 1H), 4.60-4.80 (m, 2H), 4.70 (s, 2H), 6.75-6.82 (m, 3H), 7.23 (d, J=8.1 Hz, 1H), 7.26 (d, J=7.2 Hz, 1H), 7.42-7.55 (m, 4H), 7.99 (d, J=7.2 Hz, 1H).
HR-MS:C₂₈H₂₇Cl₂N₇O₆ Calcd.: 628.1478, Found: 628.1440
[α]²⁰_{D}:-87.7° (c=0.42,MeOH)

### Example 36

### Methyl 3-(4-((2,6-dichlorophenyl)carbonylamino)-2-oxohydropyrimidinyl)-2-((4-(methylamino)-4-phenylpiperidyl)carbonylamino) propionate (36)

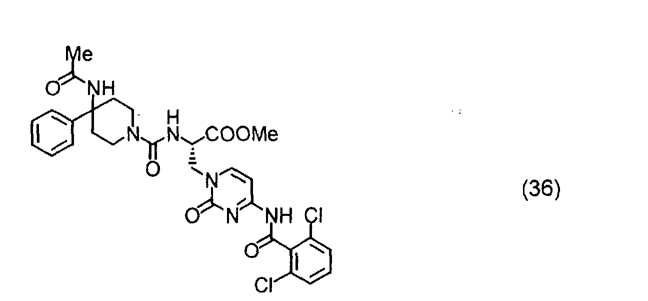

Under argon atmosphere, to a solution of 87.2 mg of methyl 2-amino-3-(4-((2,6-dichlorophenyl)carbonylamino)-2-oxohydropyrimidinyl) propionate trifluoroacetic acid salt in 5 ml of acetonitrile and 5 ml of dichloromethane, 36.7 mg of sodium hydrogen carbonate and 47.6 mg of chloroformic acid p-nitrophenyl ester were added while cooling the mixture in ice, and the resulting mixture was stirred at room temperature for 3 hours. To the reaction mixture, 3.5 ml of a solution of 59.2 mg of 4-acetoamide-4-phenylpiperidine and 0.15 ml of triethylamine in dichloromethane was added, and the resulting mixture was stirred overnight at room temperature. After concentrating the reaction mixture, 10 ml of saturated aqueous sodium hydrogen carbonate solution was added and the resulting mixture was extracted with chloroform. Organic phases were combined, washed with 1N hydrochloric acid and with saturated saline, dried over anhydrous sodium sulfate and concentrated. The residue was purified by silica gel column chromatography (chloroform/methanol = 20:1) to obtain 36.6 mg of methyl 3-(4-((2,6-dichlorophenyl)carbonylamino)-2-oxohydropyrimidinyl)-2-((4-(methylamino)-4-phenylpiperidyl)carbonylamino) propionate (yield: 33%).
LR-MS(m/z):628(M⁺)
IR(KBr):3353,3075,2982,2856,1732,1658,1560,1493,1432,1367,1301,1259,1131, 985,789cm⁻¹
NMR(300MHz,CD₃OD, δ ppm):1.85-1.96(2H,m),2.01(3H,s),2.36-2.46(2H,m),3.10-3.21(2H,m),3.77(3H,s),3.80-3.90(2H,m),4.13(1H,dd,J=13.2,9.1), 4.62(1H,dd,J=13.2,4.7),4.72(1H,dd,J=9.1,4.7),7.15-7.23(1H,m),7.28-7.58(8H,m).
7.94-7.99(1H.m),8.19(1H,brs)
[α]²⁰_{D}:-111.2° (c=0.03,MeOH)

### Example 37

### 3-(4-((2,6-dichlorophenyl)carbonylamino)-2-oxohydropyrimidinyl)-2-((4-(methylamino)-4-phenylpiperidyl)carbonylamino)propanoic acid (37)

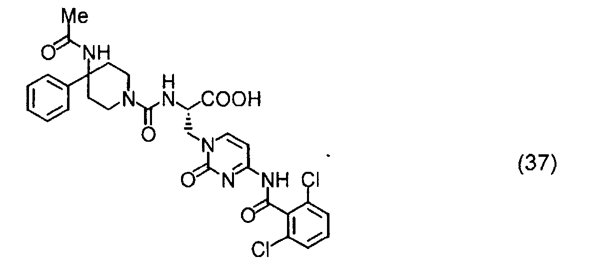

In 1.5 ml of methanol, 33.7 mg of methyl 3-(4-((2,6-dichlorophenyl)carbonylamino)-2-oxohydropyrimidinyl)-2-((4-(methylamino)-4-phenylpiperidyl)carbonylamino) propionate was dissolved, and 0.35 ml of 1N aqueous sodium hydroxide solution was added, followed by stirring the mixture at room temperature for 5.5 hours. To the reaction solution, 4 ml of 0.1N hydrochloric acid was added, and the resulting mixture was extracted with ethyl acetate. Organic phases were combined, washed with saturated saline, dried over anhydrous sodium sulfate and concentrated. The residue was reprecipitated from methanol/ether to obtain 30.2 mg of 3-(4-((2,6-dichlorophenyl)carbonylamino)-2-oxohydropyrimidinyl)-2-((4-(methylamino)-4-phenylpiperidyl)carbonylamino)propanoic acid (yield: 91%).
LR-MS(m/z):613(M⁺-H)
IR(KBr):3372,3075,2935,1722,1657,1560,1493,1431,1367,1303,1261,1195,1132, 986,901,790cm⁻¹
NMR(300MHz,CD₃OD, δ ppm):1.86-1.99(2H,m),2.01(3H,s),2.36-2.45(2H,m),3.09-3.23(2H,m),3.82-3.93(2H,m),4.12(1H,dd,J=13.2,9.5),4.66(1H,dd,J=13.2,4.2), 4.74(1H,dd,J=9.5,4.2),7.15-7.22(1H,m),7.27-7.58(8H,m),7.93-7.98(1H,m), 8.19(1H,brs)

### Example 38

### Methyl 3-(4-((2,6-dichlorophenyl)carbonylamino)-2-oxohydropyrimidinyl)-2-((4-(2-methylpropanoylamino)-4-phenylpiperidyl)carbonylamino)propanoic acid (38)

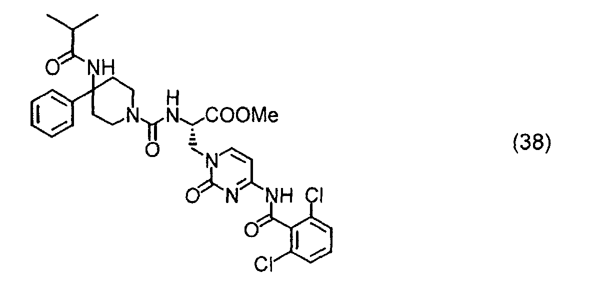

Under argon atmosphere, to a solution of 58.9 mg of methyl 2-amino-3-(4-((2,6-dichlorophenyl)carbonylamino)-2-oxohydropyrmidinyl) propionate in 4 ml of acetonitrile, 19.3 mg of sodium hydrogen carbonate and 37.0 mg of chloroformic acid p-nitrophenyl ester were added while cooling the mixture in ice, and the resulting mixture was stirred at room temperature for 2.5 hours. To the reaction mixture, 3 ml of a solution of 52.8 mg of 2-methyl-N-(4-phenyl(4-piperidyl))propanamide and 0.11 ml of triethylamine in acetonitrile was added, and the resulting mixture was stirred at room temperature for 7 hours. After concentrating the reaction mixture, 10 ml of saturated aqueous sodium hydrogen carbonate solution was added and the resulting mixture was extracted with chloroform. Organic phases were combined, washed with 1N hydrochloric acid and with saturated saline, dried over anhydrous sodium sulfate and concentrated. The residue was purified by silica gel column chromatography (chloroform:methanol = 30:1) to obtain 83.2 mg of methyl 3-(4-((2,6-dichlorophenyl)carbonylamino)-2-oxohydropyrimidinyl)-2-((4-(2-methylpropanoylamino)-4-phenylpiperidyl)carbonylamino)propanoic acid (yield: 82%).
LR-MS(m/z):656(M⁺)
IR(KBr):3319,3013,2968,1741,1657,1564,1532,1494,1433,1368,1306,1242,1210, 1132,991,928,902,755cm⁻¹
NMR(300MHz,CDCl₃, δ ppm):1.07(6H,d,J=6.9),1.86-2.00(2H,m),230-2.46(3H,m),3.03-3.18(2H,m),3.74(3H,s),3.72-3.84(2H,m),4.25-4.44(2H,m),4.72-4.79(1H,m),5.83-5,90(1H,m),6.58(1H,brs),7.16-7.35(8H,m),7.52(1H,brs),7.77-7.81(1H,m),9.22(1H,brs)
[α]²⁰_{D}:-144.3° (c=0.20,MeOH)

### Example 39

### 3-(4-((2,6-dichlorophenyl)carbonylamino)-2-oxohydropyrimidinyl)-2-((4-(2-methylpropanoylamino)-4-phenylpiperidyl)carbonylamino)propanoic acid (39)

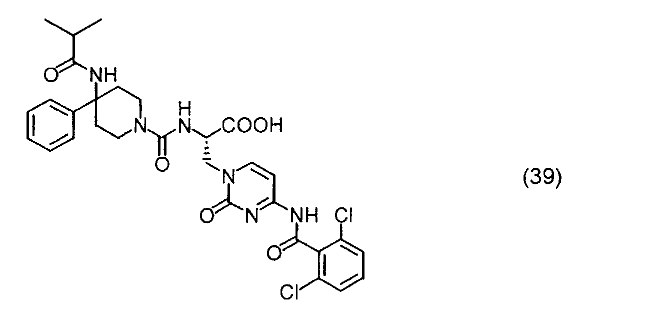

In 2 ml of methanol, 78.5 mg of methyl 3-(4-((2,6-dichlorophenyl)carbonylamino)-2-oxohydropyrimidinyl)-2-((4-(2-methylpropanoylamino)-4-phenylpiperidyl)carbonylamino) propionate was dissolved, and 1 ml of aqueous sodium hydroxide solution was added thereto, followed by stirring the resulting mixture overnight at room temperature. To the reaction solution, 1 ml of 1N hydrochloric acid and 4 ml of water were added, and the resulting mixture was extracted with ethyl acetate. Organic phases were combined, washed with saturated saline, dried over anhydrous sodium sulfate and concentrated. The residue was reprecipitated from methanol/ether to obtain 71.6 mg of 3-(4-((2,6-dichlorophenyl)carbonylamino)-2-oxohydropyrimidinyl)-2-((4-(2-mcthylpropanoylamino)-4-phenylpiperidyl)carbonylamino)propanoic acid (yield: 93%).
LR-MS(m/z):641(M⁺-H)
IR(KBr): 3380,2968,2935,2869,1721,1657,1493,1429,1366,1303,1248,1193,
1131,989, 901 cm⁻¹
NMR(300MHz,CD₃OD, δ ppm):1.09(6H,d,J=6.9),1.83-1.99(2H,m),237-2.51(2H,m),2.59-2.68(1H,m),,3.05-3.20(23H,m),3.82-3.94(2H,m),4.06-4.17(1 H,m),4.62-4.78(2H,m),7.17-7.22(1H,m),7.28-7.59(7H,m),7.97-8.05(2H,m)

### Example 40

### Methyl 3-(4-((2,6-dichlorophenyl)carbonylamino)-2-oxohydropyrimidinyl)-2-(((4-phenyl-1-benzyl-4-piperidyl)carbonylamino) propionate (40)

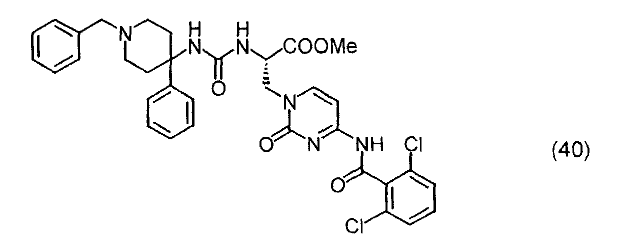

Under argon atmosphere, to a solution of 58.7 mg of methyl 2-amino-3-(4-((2,6-dichlorophenyl)carbonylamino)-2-oxohydropyrimidinyl) propionate in 4 ml of acetonitrile and 4 ml of dichloromethane, 19.2 mg of saturated sodium hydrogen carbonate and 38.3 mg of chloroformic acid p-nitrophenyl ester were added while cooling the mixture in ice, and the resulting mixture was stirred at room temperature for 2.5 hours. To the reaction mixture, 6 ml of a solution of 90.7 mg of 1-benzyl-4-amino-4-phenylpiperidine and 0.11 ml of triethylamine in acetonitrile was added, and the resulting mixture was stirred overnight at room temperature. After concentrating the reaction mixture, 10 ml of saturated aqueous sodium hydrogen carbonate solution was added and the resulting mixture was extracted with chloroform. Organic phases were combined, washed with 1N hydrochloric acid and with saturated saline. dried over anhydrous sodium sulfate and concentrated. The residue was purified by silica gel column chromatography (chloroform/methanol) to obtain 90.7 mg of methyl 3-(4-((2,6-dichlorophenyl)carbonylamino)-2-oxohydropyrimidinyl)-2-(((4-phenyl-1-benzyl-4-piperidyl)carbonylamino) propionate (yield: 88%).
LR-MS(m/z):676(M⁺)
IR(KBr):3305,3061,3027,2947,2811,2773,1717,1654,1558,1489,1431,1367,1299, 1244,1131,996,900,795,751cm⁻¹
NMR(300MHz,CDCl₃, δ ppm):2.04-2.43(6H,m),2.73-2.81(2H,m),3.55(2H,s), 3.74(3H,s),4.21(1H,dd,J=13.3,5.9),4.35(1H,dd,J=13.3,5.9),4.44-4.52(1H,m), 6.02( H,brs),7.20-7.54(16H,m)

### Example 41

### 3-(4-((2,6-dichlorophenyl)carbonylamino)-2-oxohydropyrimidinyl)-2-(((4-phenyl-1-benzyl-4-piperidyl)carbonylamino)propanoic acid (41)

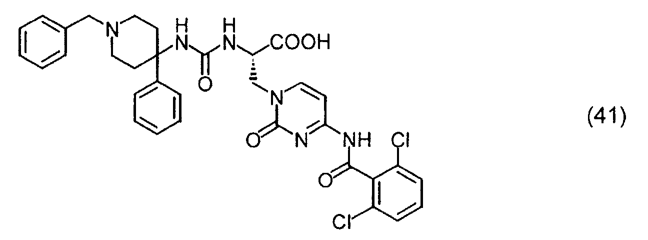

In 1.5 ml of methanol, 66.1 mg of methyl 3-(4-((2,6-dichlorophenyl)carbonylamino)-2-oxohydropyrimidinyl)-2-((4-(methylamino)-4-phenylpiperidyl)carbonylamino) propionate was dissolved, and 1.5 ml of 1N aqueous sodium hydroxide solution was added thereto, followed by stirring the resulting mixture overnight at room temperature. To the reaction solution, 1.5 ml of 1N hydrochloric acid and 10 ml of water were added, and the resulting mixture was extracted with chloroform. Organic phases were combined, washed with saturated saline, dried over anhydrous sodium sulfate and concentrated. The residue was reprecipitated from chloroform/ether to obtain 28.7 mg of 3-(4-((2,6-dichlorophenyl)carbonylamino)-2-oxohydropyrimidinyl)-2-(((4-phenyl-1-benzyl-4-piperidyl)carbonylamino)propanoic acid (yield: 44%).
LR-MS(m/z):661(M⁺-H)
IR(KBr):3391,2944,2810,1657,1623,1564,1495,1430,1364,1302,1246,1133,794cm⁻¹
NMR(300MHz,CD₃OD, δ ppm):1.98-2.17(2H,m),2.23-2.44(2H,m),2.70-3.12(4H,m),3.78-4.00(3H,m),4.45-4.65(2H,m),7.10-7.18(1H,m),7.22-7.53(13H,m),7.90-7.98(1H,m)

### Example 42

### Methyl 3-(4-((2,6-dichlorophenyl)carbonylamino)phenyl-2-((((methoxycarbonyl)cyclohexyl)amino)carbonylamino) propionate (42)

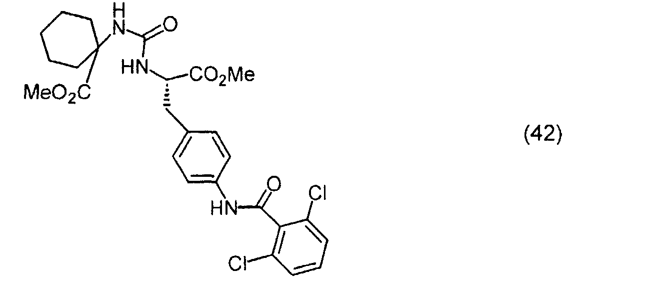

Under argon atmosphere, to a solution of 208 mg of di-t-butyl dicarbonate and 137 mg of 4-(N,N-dimethylamino)pyridine in 1 ml of acetonitrile, a solution of 170 mg of methyl 1-aminocyclohexane carboxylate in 2 ml of acetonitrile was added, and the resulting mixture was stirred at room temperature for 40 minutes. To the reaction mixture, a solution of 261 mg of methyl 2-amino-3-(4-((2,6-dichlorophenyl)carbonylamino)-2-oxohydropyrimidinyl) propionate in 10 ml of acetonitrile was added, and the resulting mixture was stirred at room temperature for 19 hours. To the reaction mixture, 1N hydrochloric acid was added and the resulting mixture was extracted with ethyl acetate. Organic phases were combined, washed with saturated saline, dried over anhydrous magnesium sulfate and concentrated. The residue was purified by silica gel column chromatography (ethyl acetate/n-hexane = 2:1) to obtain 317 mg of methyl 3-(4-((2,6-dichlorophenyl)carbonylamino)phenyl-2-((((methoxycarbonyl)cyclohexyl)amino)carbonylamino) propionate (yield: 86%). m.p.: 255°C
LR-MS(m/z):550(M⁺)
IR(KBr):3435,3307,3125,2938,2858,1772,1747,1712,1664,1604,1538,1520,1432, 1322,1270,1255,1196,799,779cm⁻¹
NMR(300MHz,DMSO-d₆, δ ppm):1.09-1.65(8H,m), 1.82(2H,m), 2.87(1H,dd,J=6.9, 13.7Hz), 2.96(1H,dd,J=5.5, 13.7Hz), 3.51(3H,s), 3.63(3H,s), 4.39(1H,m), 6.16(1H,d,J=8.5Hz), 6.49(1H,s), 7.11(1H,d,J=8.5Hz), 7.45-7.62(5H,m), 10.69(1H,s)

### Example 43

### Methyl 3-(4-((2,6-dichlorophenyl)carbonylamino)phenyl-2-((((methoxycarbonyl)cyclohexyl)amino)carbonylamino) propionate (43)

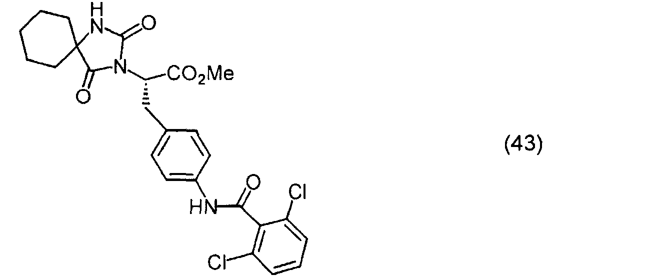

Under argon atmosphere, 278 mg of methyl 3-(4-((2,6-dichlorophenyl)carbonylamino)phenyl-2-((((methoxycarbonyl)cyclohexyl)amino)carbonylamino) propionate was dissolved in 10 ml of methanol and 10 ml of tetrahydrofuran, and 2 mg of potassium t-butoxide was added thereto, followed by stirring the resulting mixture at room temperature for 16 hours. After concentrating the reaction mixture, 1N hydrochloric acid was added to the residue and the resulting mixture was extracted with chloroform. Organic phases were combined, washed with water and with saturated saline, dried over anhydrous magnesium sulfate and concentrated. The residue was crystallized from ethyl acetate to obtain 177 mg of methyl 3-(4-((2,6-dichlorophenyl)carbonylamino)phenyl-2-((((methoxycarbonyl)cyclohexyl)amino)carbonylamino) propionate (yield: 68%). m.p.: 191°C
LR-MS(m/z):518(M⁺)
IR(KBr): 3414,3333,3273,324,3186,3121,3073,2947,2938,2857,1738,1676, 1653, 1608,1550,1510,1433,1414,1367,1332,1279,1238,1219,1195,1170,1140,1062, 785 cm⁻¹
NMR(300MHz,DMSO-d₆, δ ppm):1.05-1.70(10H,m), 3.15-3.35(2H,m), 3.68(3H,s), 4.88(1H,dd,J=5.1, 11.7Hz), 7.10(2H,d,J=8.5Hz), 7.45-7.58(5H,m), 8.68(1H,bs), 10.65(1H,s)

### Example 44

### 3-(4-((2,6-dichlorophenyl)carbonylamino)phenyl-2-((((methoxycarbonyl)cyclohexyl)amino)carbonylamino)propanoic acid (44)

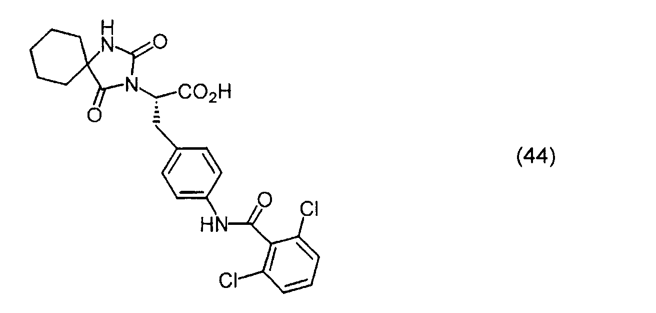

In 5 ml of methanol and 5 ml of tetrahydrofuran, 70 mg of methyl 3-(4-((2,6-dichlorophenyl)carbonylamino)phenyl-2-((((methoxycarbonyl)cyclohexyl)amino)carbonylamino) propionate was dissolved, and 0.5 ml of 1N aqueous sodium hydroxide solution was added, followed by stirring the resulting mixture at room temperature for I hour. After concentrating the reaction mixture, 1N hydrochloric acid was added to the residue and the resulting mixture was extracted with ethyl acetate. Organic phases were combined, washed with water and with saturated saline, dried over anhydrous magnesium sulfate and concentrated. The residue was recrystallized from ethyl acetate/n-hexane to obtain 48 mg of 3-(4-((2,6-dichlorophenyl)carbonylamino)phenyl-2-((((methoxycarbonyl)cyclohexyl)amino)carbonylamino)propanoic acid (yield: 70%). m.p.: 250°C
LR-MS(m/z):504(M⁺)
IR(KBr):3293,3125,3068,2938,2863,1761,1710,1670,1604,1537,1520,1434,1415, 1368,1322,1304,1270,1195,776cm⁻¹
NMR(300MHz,DMSO-d₆, δ ppm):1.05-1.65(10H,m), 3.18-3.32(2H,m), 4.72(1H,m), 7.10(2H,d,J=8.2Hz), 7.45-7.58(5H,m), 8.61(1H,bs), 10.64(1H,s), 13.12(1H,bs)

### Example 45

### Methyl 3-(4-((2,6-dichlorophenyl)carbonylamino)-2-oxohydropyrimidinyl)-2-((methylbenzylamino)carbonylamino) propionate (45)

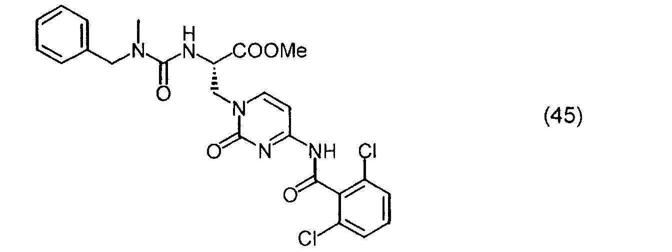

Under argon atmosphere, 38.5 mg of methyl 2-amino-3-(4-((2,6-dichlorophenyl)carbonylamino)-2-oxohydropyrimidinyl) propionate was dissolved in 2 ml of THF, and 59.0 mg of chloro-N-methyl-N-benzylamide and 125 ml of triethylamine were added thereto, followed by stirring the resulting mixture at room temperature for 3 days. To the reaction solution, 1N hydrochloric acid was added and the resulting mixture was extracted with ethyl acetate. Organic phases were combined, washed with saturated aqueous sodium hydrogen carbonate solution and with saturated saline, dried over anhydrous sodium sulfate and concentrated. The residue was purified by silica gel column chromatography (ethyl acetate/methanol = 20:1) to obtain 36.2 mg of methyl 3-(4-((2,6-dichlorophenyl)carbonylamino)-2-oxohydropyrimidinyl)-2-((methylbenzylamino)carbonylamino) propionate (yield: 68%).
LR-MS(m/z):531(M⁺)
NMR(300MHz,CD₃OD, δ ppm):2.85(3H,s),3.78(3H,s),4.06-4.19(1H,m),4.38-4.60(2H,m),4.57-4.66(1H,m),4.72-4.78(1H,m),7.18-7.28(3H,m),7.30-7.38(2H,m),7.41-7.55(4H,m),7.88-7.92(1H,m)

### Example 46

### 3-(4-((2,6-dichlorophenyl)carbonylamino)-2-oxohydropyrimidinyl)-2-((methylbenzylamino)carbonylamino)propanoic acid (46)

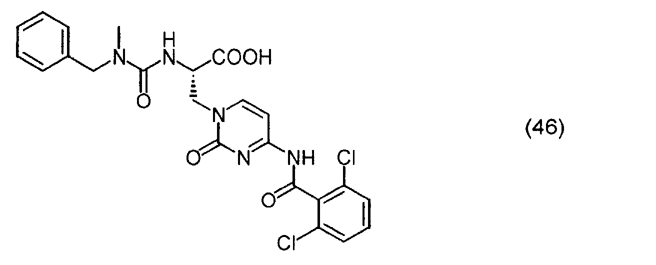

In 1 ml of methanol, 59.6 mg of methyl 3-(4-((2,6-dichlorophenyl)carbonylamino)-2-oxohydropyrimidinyl)-2-((methylbenzylamino)carbonylamino) propionate was dissolved, and 0.3 ml of 1N aqueous sodium hydroxide solution was added thereto, followed by stirring the resulting mixture overnight. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/ether to obtain 35.4 mg of 3-(4-((2,6-dichlorophenyl)carbonylamino)-2-oxohydropyrimidinyl)-2-((methylbenzylamino)carbonylamino)propanoic acid (yield: 61%).
LR-MS(m/z):517(M⁺)
IR(KBr):3423,1718,1628,1561,1493,1432,1366,1305,1247,790cm⁻¹
NMR(300MHz,CD₃OD, δ ppm):2.85(3H,s),4.07-4.16(1H,m),4.37-4.60(2H,m),4.63-4.70(1H,m),4.73-4.80(1H,m),7.15-7.25(3H,m),7.27-7.38(2H,m),7.40-7.54(4H,m), 7.90-7.94(1H,m)

### Example 47

### Methyl 3-(4-((2,6-dichlorophenyl)carbonylamino)-2-oxohydropyrimidinyl)-2-((4-cyano-4-phenylpiperidyl)carbonylamino) propionate (47)

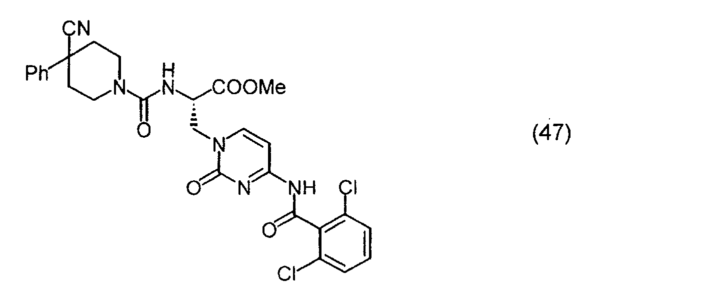

Under argon atmosphere, 69.3 mg of methyl 2-amino-3-(4-((2,6-dichlorophenyl)carbonylamino)-2-oxohydropyrimidinyl) propionate was dissolved in 0.5 ml of acetonitrile and 1 ml of dichloromethane, and 27.2 mg of sodium hydrogen carbonate and 43.5 mg of chloroformic acid p-nitrophenyl ester were added thereto while cooling the mixture in ice, followed by stirring the resulting mixture at room temperature for 1 hour. To the reaction mixture, 52.1 mg of 4-phenylpiperidine-4-carbonitrile and 100 ml of triethylamine were added, and the resulting mixture was stirred overnight. Aqueous potassium carbonate solution was added to the reaction solution, and the resulting mixture was extracted with ethyl acetate. Organic phases were combined, washed with 1N hydrochloric acid and with saturated saline, dried over anhydrous sodium sulfate and concentrated. The residue was purified by silica gel column chromatography (ethyl acetate/methanol = 20:1) to obtain 54.5 mg of methyl 3-(4-((2,6-dichlorophenyl)carbonylamino)-2-oxohydropyrimidinyl)-2-((4-cyano-4-phenylpiperidyl)carbonylamino) propionate (yield: 51%).
LR-MS(m/z):596(M⁺-H)
NMR(300MHz,CDCl₃, δ ppm):1.90-2.16(4H,m),3.21-3.33(2H,m),3.79(3H,m),4.08-4.18(2H,m),4.37-4.40(2H,m),4.73-4.81(1H,m),6.58(1H,brs),7.30-7.60(9H,m), 7.73-7.78(1H,m),8.40(1H,brs)

### Example 48

### 3-(4-((2,6-dichlorophenyl)carbonylamino)-2-oxohydropyrimidinyl)-2-((4-cyano-4-phenylpiperidyl)carbonylamino)propanoic acid (48)

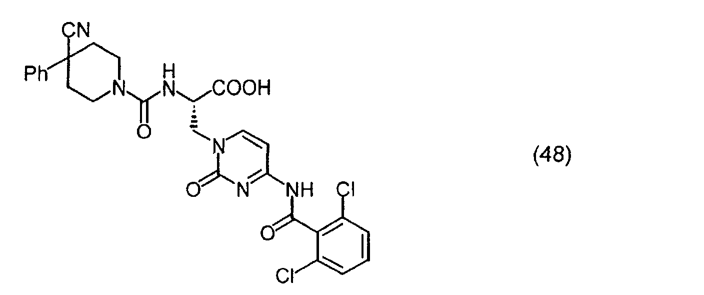

Under argon atmosphere, 46.5 mg of methyl 3-(4-((2,6-dichlorophenyl)carbonylamino)-2-oxohydropyrimidinyl)-2-((4-cyano-4-phenylpiperidyl)carbonylamino) propionate was dissolved in 1.0 ml of methanol, and 0.24 ml of 1N aqueous sodium hydroxide solution was added thereto, followed by stirring the resulting mixture overnight. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/ether to obtain 35.5 mg of 3-(4-((2,6-dichlorophenyl)carbonylamino)-2-oxohydropyrimidinyl)-2-((4-cyano-4-phenylpiperidyl)carbonylamino)propanoic acid (yield: 78%).
LR-MS(m/z):581(M⁺)
IR(KBr):3423,2927,1724,1655,1624,1558,1493,1432,1366,1242cm⁻¹
NMR(300MHz,CD₃OD, δ ppm):1.99-2.15(4H,m).3.15-3.30(2H,m),4.08-4.21 (3H,m),4.61-4.69(1H,m),4.72-4.78(1H,m),7.29-7.58(9H,m),7.98-8.02(1H,m)

### Example 49

### Methyl 3-(4-((2,6-dichlorophenyl)carbonylamino)-2-oxohydropyrimidinyl)-2-((2,6-dimethylpiperidyl)carbonylamino) propionate (49)

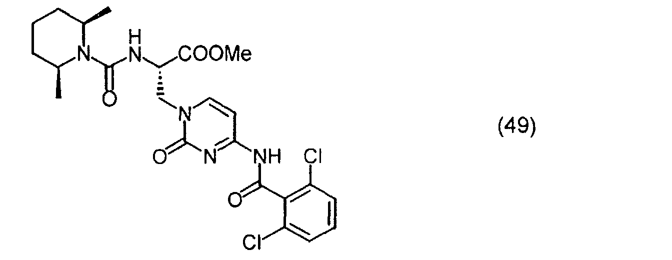

Under argon atmosphere, 75.0 mg of methyl 2-amino-3-(4-((2,6-dichlorophenyl)carbonylamino)-2-oxohydropyrimidinyl) propionate was dissolved in 1.0 ml of acetonitrile and 2 ml of dichloromethane, and 29.5 mg of sodium hydrogen carbonate and 47.2 mg of chloroformic acid p-nitrophenyl ester were added while cooling the mixture in ice, followed by stirring the resulting mixture at room temperature for 1 hour. To the reaction mixture, 34.2 ml of cis-2,6-dimethylpiperidine and 68 ml of triethylamine were added, and the resulting mixture was stirred overnight. To the reaction solution, aqueous potassium carbonate solution was added and the resulting mixture was extracted with ethyl acetate. Organic phases were combined, washed with saturated saline, dried over anhydrous sodium sulfate and concentrated. The residue was purified by silica gel column chromatography (ethyl acetate/methanol = 20:1) to obtain 47.4 mg of methyl 3-(4-((2,6-dichlorophenyl)carbonylamino)-2-oxohydropyrimidinyl)-2-((2,6-dimethylpiperidyl)carbonylamino) propionate (yield: 46%).
LR-MS(m/z):523(M⁺)
NMR(300MHz,CDCl₃, δ ppm): 1.19(6H,d,J=6.8),1.42-1.80(6H,m),3.80(3H,s),4.09-4.20(2H,m),4.36-4.46(2H,m),4.70-4.77(1H,m),5.83-5.90(1H,m),7.37-7.50(4H,m),7.68-7.75(1H,m),8.30(1H,m)

### Example 50

### 3-(4-((2,6-dichlorophenyl)carbonylamino)-2-oxohydropyrimidinyl)-2-((2,6-dimethylpiperidyl)carbonylamino)propanoic acid (50)

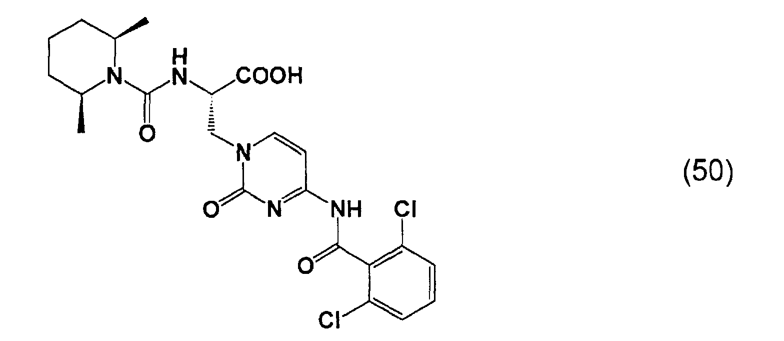

In 1.0 ml of methanol, 38.3 mg of methyl 3-(4-((2,6-dichlorophenyl)carbonylamino)-2-oxohydropyrimidinyl)-2-((2,6-dimethylpiperidyl)carbonylamino) propionate was dissolved, and 0.22 ml of 1N aqueous sodium hydroxide solution was added thereto, followed by stirring the resulting mixture overnight. To the reaction solution, 1N hydrochloric acid was added and the resulting mixture was extracted with ethyl acetate. Organic phases were combined, washed with saturated saline, dried over anhydrous sodium sulfate and concentrated. The residue was reprecipitated from chloroform/ether to obtain 33.3 mg of 3-(4-((2,6-dichlorophenyl)carbonylamino)-2-oxohydropyrimidinyl)-2-((2,6-dimethylpiperidyl)carbonylamino)propanoic acid (yield: 89%).
LR-MS(m/z):508(M⁺-H)
IR(KBr):3424,2942,1718,1658,1626,1563,1493,1432,1366,1305,1247,1192,790cm⁻¹
NMR(300MHz,CD₃OD,δppm):1.16(3H,d,J=6.8),1.20(3H,d,J=6.8),1.26-1.32(1H,m),1.40-1.53(1H,m),1.54-1.97(4H,m),4.08-4.20(3H,m),4.59-4.67(1H,m),4.70-4.76(1H,m),7.40-7.51(4H,m),7.98(1H,m)

### Example 51

### Inhibitory Effect by Compounds Against Binding between CS-1 Peptide and VLA-4-IgG Chimera Protein

In accordance with the teaching of a report (Humphrise,M.J. et al. J.Bio.Chem.,262,6886-6892(1987)), a conjugate between a peptide (Gys Leu His Gly Pro Glu Glu Ile Leu Asp Val Pro Ser Thr) containing CS-1 sequence and rabbit IgG (Sigma) was prepared. This was diluted with phosphate buffer (hereinafter referred to as "PBS(-)" for short), and the obtained solution was placed in the wells of a 96-well immunoplate (NUNC) in an amount of 100 µl/well, followed by leaving to stand the immunoplate at 4°C for 16 hours to immobilize the conjugate.

The wells were then washed twice with PBS(-), and 1% BSA solution in PBS, which BSA was heated at 80°C for 10 minutes, was placed in each well in an amount of 300 µl/well. The immunoplate was left to stand at 4°C for 3 hours, and then the solution in each well was removed by suction.

Each compound and VLA-4-IgG chimera protein (100 µl) were preliminarily reacted at room temperature for 20 minutes, and then the resulting mixture was allowed to react with the CS-1 peptide in each well at 30°C for 3 hours. Thereafter, non-bound VLA-4-IgG chimera protein was removed by suction, and each well was washed twice with 0.1% BSA-containing TBS buffer (150mM NaCI, 25mM Tris-HCl, 1 mM MnCl₂, PH7.4). To the bound VLA-4-IgG chimera protein, biotin-labelled anti-human IgG antibody (Vector) as a primary antibody was added, and then avidin-labelled horse radish peroxidase (Sigma) as a secondary antibody was added, thereby allowing the reactions. Then o-phenylenediamine as a substrate was added to color the reaction solution, and the absorbance at 490 nm was measured. From this absorbance, the binding inhibitory activity of each compound was determined. The inhibitory activities of the representative compounds are shown in Table 1.

**Table 1**

| Compound No. | Inhibitory Activity (IC₅₀:nM) |
|---|---|
| 28 | 3.8 |
| 32 | 0.97 |
| 35 | 2.4 |
| 37 | 3.5 |
| 50 | 4.5 |

### Industrial Field

The novel urea derivatives according to the present invention have activities to inhibit cell adhesion via adhesion molecules, especially adhesion molecule VLA-4. Since the urea derivatives according to the present invention are excellent in the effect of inhibiting cell adhesion via adhesion molecules, they are useful as therapeutic drugs against various inflammatory diseases.

## Claims

1. A urea acid derivative of the Formula I: 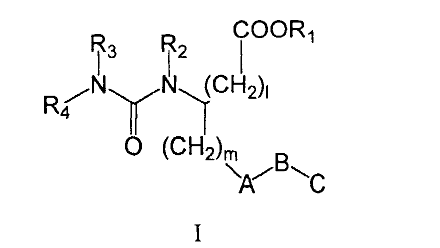 [wherein l represents an integer of 0 to 2; m represents an integer of 1 to 3; R₁ and R₂ independently represent hydrogen or C₁-C₆ linear alkyl; R₃ and R₄ independently represent hydrogen, C₁-C₆ linear alkyl, C₃-C₈ branched alkyl, or phenyl or benzyl, this phenyl or benzyl being substituted with 0 to 2 substituents selected from the group consisting of halogen, methyl, methoxy, cyano, nitro, hydroxyl, amino and tetrazole (excluding cases wherein C is represented by the Formula XIII: 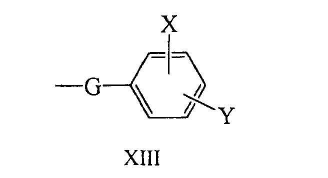 (wherein X and Y independently represent hydrogen, halogen, methyl, methoxy, cyano, nitro, hydroxyl, amino or tetrazole; G may or may not exist, and when G exists, G is a nitrogen atom))
or represent Formula II: 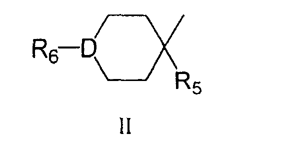 (wherein D represents a carbon atom or nitrogen atom; R₅ represents hydrogen, C₁-C₆ linear alkyl C₃-C₈ branched alkyl, C₁-C₆ linear N-alkylcarboxamide, C₃-C₈ branched N-alkylcarboxamide, or phenyl or N-phenylcarboxamide, this phenyl or N-phenylcarboxamide being substituted with 0 to 2 substituents selected from the group consisting of halogen, methyl, methoxy, cyano, nitro, hydroxyl, amino and tetrazole (with the proviso that when C is represented by said Formula XIII (wherein symbols therein represent the same meanings as described above), R₅ is C₁-C₆ linear N-alkylcarboxamide, C₃-C₈ branched N-alkylcarboxamide or N-phenylcarboxamide substituted with 0 to 2 substituents selected from the group consisting of halogen, methyl, methoxy, cyano, nitro, hydroxyl, amino and tetrazole); R₆ represents hydrogen, C₁-C₆ linear alkyl, C₃-C₈ branched alkyl, C₁-C₆ linear alkylacyl, C₃-C₈ branched alkylacyl, or phenylsulfonyl, benzoyl or benzyl, this phenylsulfonyl, benzoyl or benzyl being substituted with 0 to 2 substituents selected from the group consisting of halogen, methyl, methoxy, cyano, nitro, hydroxyl, amino and tetrazole) (with the proviso that when C is represented by said Formula XIII (wherein symbols therein represent the same meanings as described above), R₆ is phenylsulfonyl, benzoyl or benzyl, this phenylsulfonyl, benzoyl or benzyl being substituted with 0 to 2 substituents selected from the group consisting of methyl, cyano, nitro, amino and tetrazole);
R₂ and R₃ may cooperatively represent Formula III: 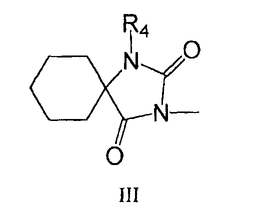 (wherein R₄ represents the same meanings as described above);
R₃ and R₄ may cooperatively represent (i) Formula IV: 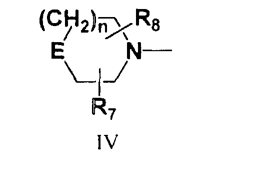 (wherein n represents an integer of 0 to 4; E represents a carbon atom or nitrogen atom; R₇ and R₈ independently represent hydrogen, C₁-C₆ linear alkyl, C₃-C₈ branched alkyl, C₁-C₆ linear alkylacyl, C₃-C₈ branched alkylacyl, pyrrolidine carbonyl, piperidine carbonyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of halogen, methyl, methoxy, cyano, nitro, hydroxyl, amino and tetrazole (with the proviso that when C is represented by said Formula XIII (wherein symbols therein represent the same meanings as described above), R₇ and R₈ independently represent pyrrolidine carbonyl, piperidine carbonyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of methyl, cyano, nitro, amino and tetrazole)
or Formula V: 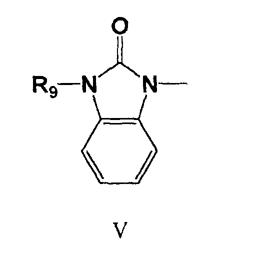 (wherein R₉ represents hydrogen, C₁-C₆ linear alkyl, C₃-C₈ branched alkyl, or phenyl or benzyl, this phenyl or benzyl being substituted with 0 to 2 substituents selected from the group consisting of halogen, methyl, methoxy, cyano, nitro, hydroxyl, amino and tetrazole),
(ii) Formula VI: 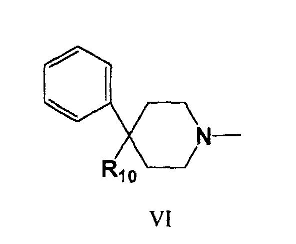 (wherein R₁₀ represents cyano, C₁-C₆ linear alkyl, C₃-C₈ branched alkyl, C₁-C₆ linear alkylamide, C₃-C₈ branched alkylamide, C₅-C₇ cycloalkylamide, C₁-C₆ linear alkylsulfonylamine, C₃-C₈ branched alkylsulfonylamine, or benzamide, phenylsulfonylamine or benzylamino, this benzamide, phenylsulfonylamine or benzylamino being substituted with 0 to 2 substituents selected from the group consisting of halogen, methyl, methoxy, cyano, nitro, hydroxyl, amino and tetrazole) (with the proviso that when C is represented by said Formula XIII (wherein symbols therein represent the same meanings as described above), R₁₀ is C₁-C₆ linear alkylsulfonylamine, C₃-C₈ branched alkylsulfonylamine, or phenylsulfonylamine substituted with 0 to 2 substituents selected from the group consisting of halogen, methyl, methoxy, cyano, nitro, hydroxyl, amino and tetrazole),
(iii) Formula VII: 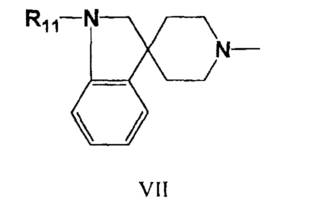 (wherein R₁₁ represents hydrogen, C₁-C₆ linear alkyl, C₃-C₈ branched alkyl, C₁-C₆ linear alkylacyl, C₃-C₈ branched alkylacyl, C₁-C₆ linear alkylsulfonyl, C₃-C₈ branched alkylsulfonyl, or phenylsulfonyl, benzyl or benzoyl, this phenylsulfonyl, benzyl or benzoyl being substituted with 0 to 2 substituents selected from the group consisting of halogen, methyl, methoxy, cyano, nitro, hydroxyl, amino and tetrazolc) (excluding cases where C is represented by said Formula XIII (wherein symbols therein represent the same meanings as described above),
(iv) Formula VIII: 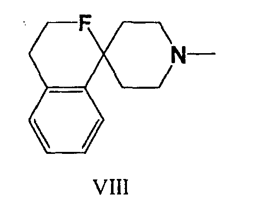 (wherein F represents a carbon atom, oxygen atom, sulfur atom or nitrogen atom; when F is a nitrogen atom, the substituent on said nitrogen atom is hydrogen, C₁-C₆ linear alkyl, C₃-C₈ branched alkyl, C₁-C₆ linear alkylacyl, C₃-C₈ branched alkylacyl, C₁-C₆ linear alkylsulfonyl, C₃-C₈ branched alkylsulfonyl, or phenylsulfonyl, benzyl or benzoyl, this phenylsulfonyl, benzyl or benzoyl being substituted with 0 to 2 substituents selected from the group consisting of halogen, methyl, methoxy, cyano, nitro, hydroxyl, amino and tetrazole) (excluding cases where C is represented by said Formula XIII (wherein symbols therein represent the same meanings as described above), or
(v) Formula IX: 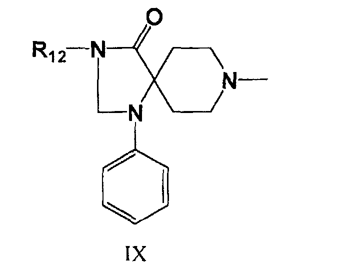 (wherein R₁₂ represents hydrogen, C₁-C₆ linear alkyl, C₃-C₈ branched alkyl, C₆-C₁₀ alkylcycloalkyl, or phenyl or benzyl, this phenyl or benzyl being substituted with 0 to 2 substituents selected from the group consisting of halogen, methyl, methoxy, cyano, nitro, hydroxyl, amino and tetrazole;
A is represented by Formula XI or XII: 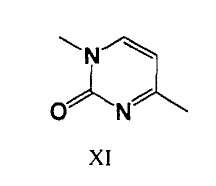 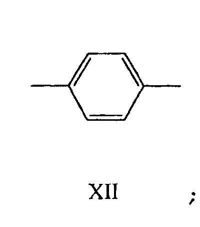
B may or may not exist, when B exists, B represents amide or C₁-C₃ methylene chain;
C is represented by said Formula IV, VI, VII, VIII, IX or XIII (wherein symbols therein represent the same meanings as described above),
or a pharmaceutically acceptable salt thereof.

2. The urea derivative or a pharmaceutically acceptable salt thereof according to claim 1, wherein in Formula I, R₃ and R₄ independently represent said Formula II (excluding cases where R₃ and R₄ simultaneously represent said Formula II, and excluding cases where, when C is represented by said Formula XIII, R₅ is hydrogen, C₁-C₆ linear alkyl, C₃-C₈ branched alkyl, or phenyl substituted with 0 to 2 substituents selected from the group consisting of halogen, methyl, methoxy, cyano, nitro, hydroxyl, amino and tetrazole, and simultaneously R₆ is hydrogen, C₁-C₆ linear alkyl, C₃-C₈ branched alkyl, C₁-C₆ linear alkylacyl, C₃-C₈ branched alkylacyl, or phenylsulfonyl, benzoyl or benzyl, this phenylsulfonyl, benzoyl or benzyl being substituted with 0 to 2 substituents selected from the group consisting of halogen, methoxy and hydroxyl); R₂ and R₃ cooperatively represent said Formula III; or R₃ and R₄ cooperatively represent (i) said Fromula IV (excluding cases where, when C is represented by said Formula XIII, R₇ and R₈ independently represent hydrogen, C₁-C₆ linear alkyl, C₃-C₈ branched alkyl, C₁-C₆ linear alkylacyl, C₃-C₈ branched alkylacyl, or phenyl, phenylsulfonyl, benzoyl, benzyl, indole, benzamide or N-phenylcarboxamide, this phenyl, phenylsulfonyl, benzoyl, benzyl, indole, benzamide or N-phenylcarboxamide being substituted with 0 to 2 substituents selected from the group consisting of halogen, methoxy and hydroxyl), (ii) said Formula VI (excluding cases where, when C is represented by said Formula XIII, R₁₀ is cyano, C₁-C₆ linear alkyl, C₃-C₈ branched alkyl, C₁-C₆ linear alkylamide, C₃-C₈ branched alkylamide, C₅-C₇ cycloalkylamide, or benzamide or benzylamide, this benzamide or benzylamide being substituted with 0 to 2 substituents selected from the group consisting of halogen, methyl, methoxy, cyano, nitro, hydroxyl and amino), (iii) said Formula VII (excluding cases where C is represented by said Formula XIII), or (iv) said Formula IX, the definitions of symbols other than stated above being the same as in claim 1.

3. A pharmaceutical comprising said urea derivative or a pharmaceutically acceptable salt thereof according to claim 1 or 2 as an effective ingredient.

4. An adhesion molecule inhibitor comprising said urea derivative or a pharmaceutically acceptable salt thereof according to claim 1 or 2 as an effective ingredient.

5. The adhesion molecule inhibitor according to claim 4, wherein said adhesion molecule belongs to integrin family.

6. The adhesion molecule inhibitor according to claim 5, wherein said integrin family is VLA-4.

7. The adhesion molecule inhibitor according to any one of claims 4 to 6, which is for against inflammatory disease.

8. The adhesion molecule inhibitor according to claim 7, wherein said inflammatory disease is an allergic disease.

9. A method for inhibiting an adhesion molecule, comprising administering an effective amount of said urea derivative or a pharmaceutically acceptable salt thereof according to claim 1 or 2.

10. The method according to claim 9, wherein said adhesion molecule belongs to integrin family.

11. The method according to claim 10, wherein said integrin family is VLA-4.

12. The method according to any one of claims 9 to 11, which is for against inflammatory disease.

13. The method according to claim 12, wherein said inflammatory disease is an allergic disease.

14. Use of said urea derivative or a pharmaceutically acceptable salt thereof according to claim 1 or 2 for the production of a pharmaceutical.

15. Use of said urea derivative or a pharmaceutically acceptable salt thereof according to claim 1 or 2 for the production of an adhesion molecule inhibitor.

16. The use according to claim 15, wherein said adhesion molecule belongs to integrin family.

17. The use according to claim 16, wherein said integrin family is VLA-4.

18. The use according to any one of claims 14 to 17, wherein said pharmaceutical or adhesion molecule inhibitor is for against inflammatory disease.

19. The adhesion molecule inhibitor according to claim 18, wherein said inflammatory disease is an allergic disease.
